# EUROPEAN PATENT SPECIFICATION

(11) **EP 1 718 340 B1**
(45) Date of publication and mention of the grant of the patent: **21.11.2007**
(21) Application number: 05707090.6
(22) Date of filing: 31.01.2005
(51) Int. Cl.: A61K 31/427, A61P 25/00, A61P 35/00

(54) **NEW EFFECTOR CONJUGATES, PROCESS FOR THEIR PRODUCTION AND THEIR PHARMACEUTICAL USE**
NEUE EFFEKTOR-KONJUGATE, VERFAHREN ZU IHRER HERSTELLUNG UND IHRE PHARMAZEUTISCHE VERWENDUNG
NOUVEAUX CONJUGUES D'EFFECTEURS, LEURS PROCEDES DE PRODUCTION ET LEUR UTILISATION PHARMACEUTIQUE

(30) Priority: 30.01.2004 DE 102004004787; 30.01.2004 US 539977 P
(43) Date of publication of application: 08.11.2006
(73) Proprietor: Bayer Schering Pharma Aktiengesellschaft, 13353 Berlin (DE)
(72) Inventor: KLAR, Ulrich, 13503 Berlin (DE); WILLUDA, Jörg, 13156 Berlin (DE); MENRAD, Andreas, 16515 Oranienburg (DE); BOSSLET, Klaus, 13465 Berlin (DE)
(74) Representative: Krüger, Anita
(86) International application number: PCT/EP2005/000917
(87) International publication number: WO 2005/074901

(56) References cited:
- WO-A-20/04012735
- WO-A-20/04050089
- DE-A1- 10 234 975
- DE-A1- 10 305 098
- US-A1- 2004 126 379
- US-A1- 2004 167 083
- NICOLAOU K C ET AL: "Chemical Biology of Epothilones" ANGEWANDTE CHEMIE. INTERNATIONAL EDITION, VERLAG CHEMIE. WEINHEIM, DE, vol. 37, no. 15, August 1998 (1998-08), pages 2014-2045, XP002131418 ISSN: 0570-0833
- HARRIS ET AL: "New Chemical Synthesis of the Promising Cancer Chemotherapeutic Agent 12,13-Desoxyepothilone B: Discovery of a Surprising Long-Range Effect on the Diastereoselectivity of an Aldol Condensation" JOURNAL OF THE AMERICAN CHEMICAL SOCIETY, AMERICAN CHEMICAL SOCIETY, WASHINGTON, DC, US, vol. 121, 1999, pages 7050-7062, XP002150959 ISSN: 0002-7863

## Description

The development of the understanding relative to the recognition of binding regions, especially in the field of monoclonal antibodies or their fragments from specific tumor antigens, makes it possible to conceive a selective tumor therapy by specific release of an anti-tumor active ingredient at the target site.

A requirement for such an approach, in which a highly active (toxic) active ingredient (effector) is coupled to a high-molecular tumor-specific recognition unit, such as, for example, to an antibody, is a substantial inactivity of the conjugate, whose least components represent a recognition unit and an effector, until the latter has reached the target site (tumor). When the target site is reached, the conjugate binds to the cell surface, and the active ingredient can be released optionally after the entire complex is first internalized.

The successful therapy of solid tumors, especially with monoclonal antibodies, can be limited, however, by an inadequate penetration of the antibody in the tumor as well as the heterogeneous distribution of the corresponding tumor-associated antigen in the tumor tissue.

These limitations thus could be avoided by having the tumor-vascular system be attacked in a specific way. The growth of tumors below a volume of about 2 mm³ is based on a neoangiogenesis. The additional tumor growth is based on an intact vascular system, which ensures the supply with nutrients or disposal of waste products. The selective destruction of this system should therefore result in a necrosis of the tumor. Attacking the vascular system of the tumor offers a number of advantages compared to the direct attack on the tumor itself. In comparison to tumor cells, endothelial cells are easier to access, since no tumor tissue has to be penetrated. The damage of an individual tumor vessel should result in the necrosis of a thousand tumor cells. To damage a tumor vessel, it is not necessary to kill off all endothelial cells. The specific attack of endothelial cells in or near tumors minimizes systemic side effects. Endothelial cells are genetically very stable, such that the probability of a development of resistance against the tumor therapeutic agent is low.

Within the scope of this invention, surprisingly enough, a possibility has now been found to link the chemically very sensitive, highly-functionalized active ingredient class of the epothilones and analogs thereof with a high-molecular recognition unit via different linkers that are distinguished in that they can be cleaved selectively by intracellular at various positions of the active ingredient.

The object of this invention is thus based on, i.a.,
1. Finding a method to link highly active active ingredients from the structural class of the epothilones and epothilone derivatives with suitable linkers,
2. Synthesizing suitable, intracellularly cleavable linkers;
3. Developing a method to link these epothilone-linker conjugates to recognition units, such as, for example, monoclonal antibodies or their fragments, to immune conjugates, which are sufficiently stable both chemically and metabolically for a pharmaceutical agent development and which are superior to the epothilone or epothilone derivatives that are the underlying compounds with respect to their therapeutic width, their selectivity of action and/or undesirable toxic side effects and/or their active strength.

This invention correspondingly comprises effector conjugates of general formula I 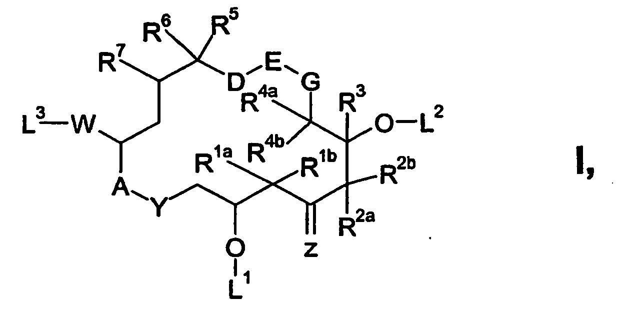 in which
- R^{1a}, R^{1b},: independently of one another, are hydrogen, C₁-C₁₀ alkyl, aryl, aralkyl, or together a -(CH₂)ₘ group, in which m is 2 to 5,
- R^{2a}, R^{2b},: independently of one another, are hydrogen, C₁-C₁₀ alkyl, aryl, aralkyl, or together a -(CH₂)ₙ group, in which n is 2 to 5, or C₂-C₁₀ alkenyl, or C₂-C₁₀ alkinyl,
- R³: is hydrogen, C₁-C₁₀ alkyl, aryl or aralkyl, and
- R^{4a}, R^{4b},: independently of one another, are hydrogen, C₁-C₁₀ alkyl, aryl, aralkyl, or together a -(CH₂)ₚ group, in which p is 2 to 5,
- R⁵: is hydrogen, C₁-C₁₀ alkyl, aryl, aralkyl, CO₂H, CO₂alkyl, CH₂OH, CH₂-alkyl, CH₂Oacyl, CN, CH₂NH₂, CH₂N(alkyl, acyl)_{1,2}, or CH₂Hal,
- Hal: is a halogen atom,
- R⁶, R⁷,: in each case, are hydrogen, or together an additional bond or together an oxygen atom, or together an NH group, or together an N-alkyl group, or together a CH₂ group, and
- G: is an oxygen atom or CH₂,
- D-E: is a group H₂C-CH₂, HC=CH, C≡C, CH(OH)-CH(OH), CH(OH)-CH₂, CH₂-CH(OH), 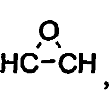 O-CH₂, or, if G represents a CH₂ group, D-E is also CH₂-O,
- W: is a group C(=X)R⁸, or a bi- or tricyclic aromatic or heteroaromatic radical,
- L³: is hydrogen, or, if a radical in W contains a hydroxyl group, forms a group O-L⁴ with the latter, or, if a radical in W contains an amino group, forms a group NR²⁵-L⁴ with the latter,
- R²⁵: is hydrogen or C₁-C₁₀alkyl,
- X: is an oxygen atom, or two OR²⁰ groups, or a C₂-C₁₀ alkylenedioxy group, which should be straight-chain or branched, or H/OR⁹, or a CR¹⁰R¹¹ group,
- R⁸: is hydrogen, C₁-C₁₀ alkyl, aryl, aralkyl, halogen or CN, and
- R⁹: is hydrogen or a protective group PG^{X},
- R¹⁰, R¹¹,: in each case independently of one another, are hydrogen, C₁-C₂₀ alkyl, aryl, or aralkyl, or together with a methylene carbon atom form a 5- to 7-membered carbocyclic ring,
- Z: can represent oxygen or H/OR¹²,
- R¹²: can represent hydrogen or a protective group PG^{Z},
- A-Y: can represent a group O-C(=O), O-CH₂, CH₂-C(=O), NR²¹-C(=O) or NR²¹-SO₂,
- R²⁰: can represent C₁-C₂₀ alkyl,
- R²¹: can represent a hydrogen atom or C₁-C₁₀ alkyl,
- PG^{X}, PG^{Y}, and PG^{Z}: can represent a protective group PG, and
- L¹, L², L⁴,: independently of one another, can represent hydrogen, a group C(=O)Cl, a group PG^{Y} or a linker of general formula III; with the condition that at least one substituent L¹, L² or L⁴ represents a linker of general formula (III);
the linker of general formula (III) has the following structure, 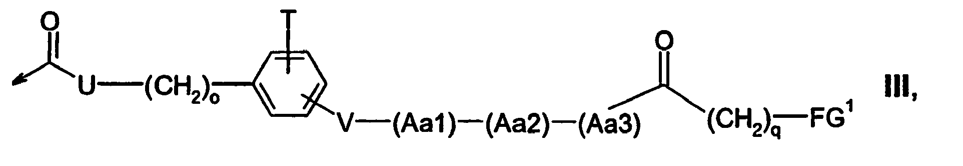 in which
- T: represents halogen, OH, O-alkyl, CO₂H, CO₂-alkyl, -NHR²³, -NO₂, -N₃, -CN, C₁-C₂₀-alkyl, C₁-C₂₀-acyl or C₁-C₂₀-acyloxy groups,
- U: represents a bond, oxygen, or NR^{24a},
- o: represents 0 to 5,
- V: represents oxygen or NR^{24b},
- Aa1: represents a bond or a group of general formula IV and
- Aa2, Aa3,: independently of one another, represent a group of general formula IV 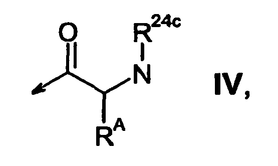 which is derived from a natural or unnatural amino acid HO-Aa1-H, HO- Aa2-H, or HO-Aa3-H of general formula IV' 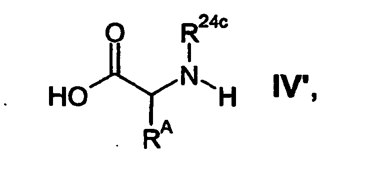
in which R^{A} can be the same or different in HO-Aa1-H, HO-Aa2-H, or HO-Aa3-H, and the a-substituent represents a natural or unnatural amino acid,
- R²²: can represent hydrogen, C₁-C₁₀ alkyl, aryl or aralkyl,
- R²³: can represent hydrogen, C₁-C₁₀ alkyl, or C₁-C₁₀ acyl,
- R^{24a}, R^{24b}, R^{24c},: independently of one another, can represent hydrogen or C₁-C₁₀ alkyl,
- q: can represent 1 to 20,
- FG¹: can represent C₁-C₁₀ alkyl-S₃, 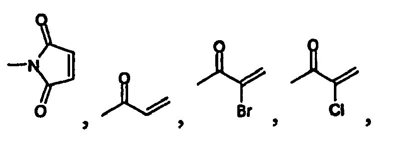 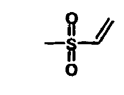 or CO₂H;
as a uniform isomer or a mixture of different isomers and/or as a pharmaceutically acceptable salt thereof.

In addition, the invention describes the production of effect-recognition unit conjugates of general formula (I), whereby the substituents here have the above-mentioned meanings, but at least one group FG¹ is replaced by a group FG^{2a} or FG^{2b}, whereby FG^{2a} or FG^{2b} can have the following meanings:
FG^{2a} is -S-S-, 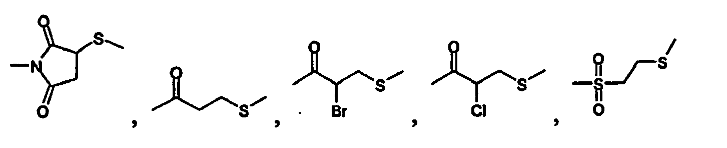 FG^{2b} is -CONH-
and whereby a recognition unit is conjugated via a sulfur atom with the group FG^{2a}, whereby the sulfur atom that is shown is a component of the recognition unit, or via an amide group with group FG^{2b}, whereby the nitrogen atom that is shown is a component of the recognition unit;
whereby the recognition unit can be, for example, a peptide, a soluble receptor, a cytokine, a lymphokine, an aptamer, a spiegelmer, a recombinant protein, a framework structure, a monoclonal antibody or a fragment of a monoclonal antibody.

The thiol groups that are required for a conjugation, at least one thiol group is required, can already be included in the recognition unit or can be produced, for example, by reductive cleavage of disulfide bridges or by functionalization of suitable amino acid units according to the methods that are known to one skilled in the art. For example, the reaction of proteins that contain amino groups with Traut's reagent can be mentioned.

According to this invention, the above-mentioned effector-recognition unit conjugates can comprise one or more recognition units; in this case, the recognition units that correspond to a conjugate can be identical or different. It is preferred that the recognition units of a conjugate be identical.

The effector-recognition unit conjugates according to the invention can be used in the form of their α-, β- or γ-cyclodextrin clathrates or in the form of liposomal or pegylated compositions.

The conjugates according to the invention are preferably used for the treatment of diseases that are linked with proliferative processes. For example, the therapy of widely varying tumors, the therapy of inflammatory and/or neurodegenerative diseases, such as multiple sclerosis or Alzheimer's disease, the therapy of angiogenesis-associated diseases, such as the growth of solid tumors, rheumatoid arthritis or diseases of the ocular fundus can be mentioned.

The production of epothilones, their precursors and derivatives of general formula I is carried out according to the methods that are known to one skilled in the art, as they are described in, for example, DE 19907588, WO 98/25929, WO 99/58534, WO 99/2514, WO 99/67252, WO 99/67253, WO 99/7692, EP 99/4915, WO 00/485, WO 00/1333, WO 00/66589, WO 00/49019, WO 00/49020, WO 00/49021, WO 00/71521, WO 00/37473, WO 00/57874, WO 01/92255, WO 01/81342, WO 01/73103, WO 01/64650, WO 01/70716, US 6204388, US 6387927, US 6380394, US 02/52028, US 02/58286, US 02/62030, WO 02/32844, WO 02/30356, WO 02/32844, WO 02/14323, and WO 02/8440.

As alkyl groups R^{1a}, R^{1b}, R^{2a}, R^{2b}, R³, R^{4a}, R^{4b}, R⁵, R⁸, R¹⁰, R¹¹, R²⁰, R²¹, R²², R²³, R^{24a}, R^{24b}, R^{24c}, R²⁵, and R²⁶, straight-chain or branched-chain alkyl groups with 1-20 carbon atoms can be considered, such as, for example, methyl, ethyl, propyl, isopropyl, butyl, isobutyl, tert.-butyl, pentyl, isopentyl, neopentyl, heptyl, hexyl, and decyl.

Alkyl groups R^{1a}, R^{1b}, R^{2a}, R^{2b}, R³, R^{4a}, R^{4b}, R⁵, R⁸, R¹⁰, R¹¹, R²⁰, R²¹, R²², R²³, R^{24a}, R^{24b}, R^{24c}, R²⁵ and R²⁶ can also be perfluorinated or substituted by 1-5 halogen atoms, hydroxy groups, C₁-C₄-alkoxy groups, or C₆-C₁₂-aryl groups (which can be substituted by 1-3 halogen atoms).

As aryl radicals R^{1a}, R^{1b}, R^{2a}, R^{2b}, R³, R^{4a}, R^{4b}, R⁵, R⁸, R¹⁰, R¹¹, R²², and R²⁶, substituted and unsubstituted carbocyclic or heterocyclic radicals with one or more heteroatoms, such as phenyl, naphthyl, furyl, thienyl, pyridyl, pyrazolyl, pyrimidinyl, oxazolyl, pyridazinyl, pyrazinyl, quinolyl, thiazolyl, benzothiazolyl, or benzoxazolyl, which can be substituted in one or more places by halogen, OH, O-alkyl, CO₂H CO₂-alkyl, -NH₂, -NO₂, -N₃, -CN, C₁-C₂₀-alkyl, C₁-C₂₀-acyl, or C₁-C₂₀-acyloxy groups, are suitable. The heteroatoms can be oxidized if as a result the aromatic character is not lost, such as, for example, the oxidation of a pyridyl to a pyridyl-N-oxide.

As bi- and tricyclic aryl radicals W, substituted and unsubstituted carbocyclic or heterocyclic radicals with one or more heteroatoms, such as naphthyl, anthryl, benzothiazolyl, benzoxazolyl, benzimidazolyl, quinolyl, isoquinolyl, benzoxazinyl, benzofuranyl, indolyl, indazolyl, quinoxalinyl, tetrahydroisoquinolinyl, tetrahydroquinolinyl, thienopyridinyl, pyridopyridinyl, benzopyrazolyl, benzotriazolyl, or dihydroindolyl, which can be substituted in one or more places by halogen, OH, O-alkyl, CO₂H, CO₂-alkyl, -NH₂, -NO₂, -N₃, -CN, C₁-C₂₀-alkyl, C₁-C₂₀-acyl, or C₁-C₂₀-acyloxy groups, are suitable. The heteroatoms can be oxidized if as a result the aromatic character is not lost, such as, for example, the oxidation of a quinolyl to a quinolyl-N-oxide.

The aralkyl groups in R^{1a}, R^{1b}, R^{2a}, R^{2b}, R³, R^{4a}, R^{4b}, R⁵, R⁸, R¹⁰, R¹¹, R²²_{,} and R²⁶ can contain in the ring up to 14 C atoms, preferably 6 to 10 C atoms, and in the alkyl chain 1 to 8 atoms, preferably 1 to 4 atoms. As aralkyl radicals, for example, benzyl, phenylethyl, naphthylmethyl, naphthylethyl, furylmethyl, thienylethyl, or pyridylpropyl are considered. The rings can be substituted in one or more places by halogen, OH, O-alkyl, CO₂H, CO₂-alkyl, -NO₂, -N₃, -CN, C₁-C₂₀-alkyl, C₁-C₂₀-acyl, or C₁-C₂₀-acyloxy groups.

As representatives of protective groups PG, tris(C₁-C₂₀ alkyl)silyl, bis(C₁-C₂₀ alkyl)-arylsilyl, (C₁-C₂₀ alkyl)-diarylsilyl, tris(aralkyl)-silyl, C₁-C₂₀-alkyl, C₂-C₂₀-alkenyl, C₄-C₇-cycloalkyl, which in addition can contain an oxygen atom in the ring, aryl, C₇-C₂₀-aralkyl, C₁-C₂₀-acyl, aroyl, C₁-C₂₀-alkylsulfonyl as well as arylsulfonyl can be mentioned.

As alkyl, silyl and acyl radicals for protective groups PG, in particular the radicals that are known to one skilled in the art are considered. Preferred are the alkyl or silyl radicals that are easily cleavable from the corresponding alkyl ethers and silyl ethers, such as, for example, the methoxymethyl, methoxyethyl, ethoxyethyl, tetrahydropyranyl, tetrahydrofuranyl, trimethylsilyl, triethylsilyl, tert.-butyldimethylsilyl, tert.-butyldiphenylsilyl, tribenzylsilyl, triisopropylsilyl, benzyl, para-nitrobenzyl, or para-methoxybenzyl radical as well as alkylsulfonyl and arylsulfonyl radicals. As acyl radicals, e.g., formyl, acetyl, propionyl, isopropionyl, trichloromethylcarbonyl, pivalyl, butyryl or benzoyl, which can be substituted with amino groups and/or hydroxy groups, are suitable.

As amino protective groups PG, the radicals that are known to one skilled in the art are considered. For example, the Alloc, Boc, trityl, monomethoxy-trityl, Z, benzyl, f-Moc, Troc, Stabase or benzostabase group can be mentioned.

As halogen atoms, fluorine, chlorine, bromine or iodine is considered.

The acyl groups can contain 1 to 20 carbon atoms, whereby formyl, acetyl, propionyl, isopropionyl and pivalyl groups are preferred.

The C₂-C₁₀-alkylene-α,ω-dioxy group that is possible for X is preferably an ethyleneketal or neopentylketal group.

As natural or unnatural amino acids HO-Aa1-H, HO-Aa2-H, and HO-Aa3-H, there can be mentioned, for example: glycine, alanine, serine, proline, valine, threonine, cysteine, isoleucine, leucine, asparagine, asparaginic acid, glutamine, lysine, ornithine, glutamic acid, methionine, histidine, phenylalanine, arginine, tyrosine, tryptophan, citrulline, hydroxyproline, tetrahydroisoquinoline-3-carboxylic acid, α-pyridylalanine, α-naphthylalanine, p-nitrophenylalanine or homophenylalanine in each case in the D- or L- or D/L form or their salts. Free hydroxyl groups or free amino groups in which α-substituents of the natural or unnatural amino acids can optionally carry a protective group PG.

Preferred compounds of general formula I are those in which A-Y represents O-C(=O) or NR²¹-C(=O); D-E represents an H₂C-CH₂ group or an H₂C=CH₂ group; G represents a CH₂ group; Z represents an oxygen atom; R^{1a}, R^{1b} in each case represent C₁-C₁₀ alkyl or together a-(CH₂)ₚ group with p equal to 2 or 3 or 4; R^{2a}, R^{2b}, independently of one another, represent hydrogen, C₁-C₁₀ alkyl, C₂-C₁₀ alkenyl, or C₂-C₁₀ alkinyl; R³ represents hydrogen; R^{4a}, R^{4b}, independently of one another, represent hydrogen or C₁-C₁₀ alkyl; R⁵ represents hydrogen or C₁-C₄ alkyl or CH₂OH or CH₂NH₂ or CH₂N(alkyl, acyl) 1,2 or CH₂Hal; R⁶ and R⁷ together represent an additional bond or together an NH group or together an N-alkyl group or together a CH₂ group or together an oxygen atom; W represents a group C(=X)R⁸ or a 2-methylbenzothiazol-5-yl radical or a 2-methylbenzoxazol-5-yl radical or a quinolin-7-yl radical or a 2-aminomethylbenzothiazol-5-yl radical or a 2-hydroxymethylbenzothiazol-5-yl radical or a 2-aminomethylbenzoxazol-5-yl radical or a 2-hydroxymethyl-benzoxazol-5-yl radical; X represents a CR¹⁰R¹¹ group; R⁸ represents hydrogen or C₁-C₄ alkyl or a fluorine atom or a chlorine atom or a bromine atom; R¹⁰/R¹¹ represent hydrogen/2-methylthiazol-4-yl or hydrogen/2-pyridyl or hydrogen/2-methyloxazol-4-yl or hydrogen/2-aminomethylthiazol-4-yl or hydrogen/2-aminomethyloxazol-4-yl or hydrogen/2-hydroxymethylthiazol-4-yl or hydrogen/2-hydroxymethyloxazol-4-yl.

As linkers of general formula (III), compounds are preferred in which Aa1 represents a bond.

As linkers of general formula (III), in addition compounds are preferred in which FG¹ is C₁-C₁₀ alkyl-S₃ or

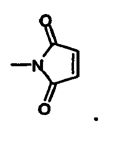

As recombinant proteins for use as recognition units, for example, binding regions that are derived from antibodies, so-called CDRs, are suitable.

As framework structures for use as recognition units, for example, high-molecular structures, which are not derived from antibodies, are suitable. For example, structures such as fibronectin-type 3 and crystallines can be mentioned.

As fragments of monoclonal antibodies for use as recognition units, for example, single-chain Fv, Fab, F(ab)₂ as well as recombinant multimers can be mentioned.

As preferred recognition units, those are considered that are suitable for, for example, the recognition and/or diagnosis and/or therapy of solid tumors and malignant diseases of the hematopoietic system.

As recognition units that are preferred in addition, those are considered that make possible a selective recognition of the disease-specific vascular system, preferably angiogenesis.

Table 1 cites examples of especially preferred recognition units for treating solid tumors.

**TABLE 1**

| **Tumor** | **Antigen Identity/ Characteristics** | **Monoclonal Antibodies** | **References** |
|---|---|---|---|
| **Gynecol. (GY)** | CA 125' > 200 kD mucin GP | OC 125 | Kabawat et al., 1983; Szymendera, 1986 |
| Ovarian | 80 Kd GP | OC 133 | Masuko et al., Cancer Res, 1984 |
| Ovarian | 'SGA' 360 Kd GP | OMI | de Krester et al., 1986 |
| Ovarian | High Mᵣ mucin | Mo v1 | Miotti et al., Cancer Res, 1985 |
| Ovarian | High Mᵣ mucin/ glycolipid | Mo v2 | Miotti et al., Cancer Res, 1985 |
| Ovarian | NS | 3C2 | Tsuji et al., Cancer Res, 1985 |
| Ovarian | NS | 4C7 | Tsuji et al., Cancer Res, 1985 |
| Ovarian | High Mᵣ mucin | ID3 | Gangopadhyay et al., 1985 |
| Ovarian | High Mᵣ mucin | DU-PAN-2 | Lan et al., 1985 |
| GY | 7700 Kd GP | F 36/22 | Croghan et al., 1984 |
| Ovarian | 'gp 68'48 Kd GP | 4F₇/7A₁₀ | Bhattacharya et al., 1984 |
| GY | 40, 42kD GP | OV-TL3 | Poels et al., 1986 |
| GY | 'TAG-72' High Mᵣ mucin | B72.3 | Thor et al., 1986 |
| Ovarian | 300-400 Kd GP | DF₃ | Kufe et al., 1984 |
| Ovarian | 60 Kd GP | 2C₈/2F₇ | Bhattacharya et al., 1985 |
| GY | 105 Kd GP | MF 116 | Mattes et al., 1984 |
| Ovarian | 38-40 kD GP | Mov18 | Miotti et al., 1987 |
| GY | 'CEA' 180 Kd GP | CEA 11-H5 | Wagener et al., 1984 |
| Ovarian | CA 19-9 or GICA | CA 19-9 (1116NS 19-9) | Atkinson et al., 1982 |
| Ovarian | 'FLAP' 67 Kd GP | H17-E2 | McDicken et al., 1985 |
| Ovarian | 72 Kd | 791T/36 | Perkins et al., 1985 |
| Ovarian | 69 Kd PLAP | NDOG₂ | Sunderland et al., 1984 |
| Ovarian | unknown Mᵣ PLAP | H317 | Johnson et al., 1981 |
| Ovarian | p₁₈₅HER2 | 4D5, 3H4, 7C2, 6E9, 2C4, 7F3, 2H11, 3E8, 5B8, 7D3, SB8 | Shepard et al., 1991 |
| Uterus, Ovary | HMFG-2 | HMFG2 | Epenetos et al., 1982 |
| GY | HMFG-2 | 3.14.A3 | Burchell et al., 1983 |
| **Breast** | 330-450 Kd GP | DF3 | Hayes et al., 1985 |
| Breast | NS | NCRC-11 | Ellis et al., 1984 |
| Breast | 37kD | 3C6F9 | Mandeville et al., 1987 |
| Breast | NS | MBE6 | Teramoto et al., 1982 |
| Breast | NS | CLNH5 | Glassy et al., 1983 |
| Breast | 47 Kd GP | MAC 40/43 | Kjeldsen et al., 1986 |
| Breast | High Mᵣ GP | EMA | Sloane et al., 1981 |
| Breast | High Mᵣ GP | HMFG1 HFMG2 | Arklie et al., 1981 |
| Breast | NS | 3.15.C3 | Arklie et al., 1981 |
| Breast | NS | M3, M8, M24 | Foster et al., 1982 |
| Breast | 1 (Ma) Blood Group Ags | M18 | Foster et al., 1984 |
| Breast | NS | 67-D-11 | Rasmussen et al., 1982 |
| Breast | Estrogen Receptor | D547Sp, D75P3, H222 | Kinsel et al., 1989 |
| Breast | EGF Receptor | Anti EGF | Sainsbury et al., 1985 |
| Breast | Laminine Receptor | LR-3 | Horan Hand et al., 1985 |
| Breast | *erb* B-2 p185 | TA1 | Gusterson et al., 1988 |
| Breast | NS | H59 | Hendler et al., 1981 |
| Breast | 126 Kd GP | 10-3D-2 | Soule et al., 1983 |
| Breast | NS | HmAB1,2 | Imam et al., 1984; Schlom et al., 1985 |
| Breast | NS | MBR 1,2,3 | Menard et al., 1983 |
| Breast | 95 Kd | 24-17-1 | Thompson et al., 1983 |
| Breast | 100 Kd | 24-17-2 (3E1-2) | Croghan et al., 1983 |
| Breast | NS | F36/22.M7/105 | Croghan et al., 1984 |
| Breast | 24 Kd | C11, G3, H7 | Adams et al., 1983 |
| Breast | 90 Kd GP | B6-2 | Colcher et al., 1981 |
| Breast | CEA & 180 Kd GP | B1-1 | Colcher et al., 1983 |
| Breast | Colon & pancreas, mucin-like Ca 19-9 | Cam 17-1 | Imperial Cancer Research Technology MAb listing |
| Breast | Milk mucin, nuclear protein | SM3 | Imperial Cancer Research Technology Mab listing |
| Breast | Milk mucin, nuclear protein | SM4 | Imperial Cancer Research Technology Mab listing |
| Breast | Affinity-purified milk mucin | C-Mul (566) | Imperial Cancer Research Technology Mab listing |
| Breast | _{P185}HER2 | 4D5 3H4, 7C2, 6E9, 2C4, 7F3, 2H11, 3E8, 5B8, 7D3, 5B8 | Shepard et al., 1991 |
| Breast | CA 125 > 200 Kd GP | OC 125 | Kabawat et al., 1985 |
| Breast | High Mᵣ mucin/ glycolipid | MO v2 | Miotti et al., 1985 |
| Breast | High Mᵣ mucin | DU-PAN-2 | Lan et al., 1984 |
| Breast | 'gp48' 48 Kd GP | 4F₇/7A₁₀ | Bhattacharya et al., 1984 |
| Breast | 300-400 Kd GP | DF₃ | Kufe et al., 1984 |
| Breast | 'TAG-72' high Mᵣ mucin | B72-3 | Thor et al., 1986 |
| Breast | 'CEA' 180 Kd GP | cccccCEA 11 | Wagener et al., 1984 |
| Breast | 'PLAP' 67 Kd GP | H17-E2 | McDicken et al., 1985 |
| Breast | HMFG-2 > 400 Kd GP | 3-14-A3 | Burchell et al., 1983 |
| Breast | NS | F023C5 | Riva et al., 1988 |
| **Colorectal** | TAG-72 High Mᵣ mucin | B72-3 | Colcher et al., 1987 |
| Colorectal | GP37 | (17-1A) 1038-17-1A | Paul et al., 1986 |
| Colorectal | Surface GP | CO17-1A | LoBuglio et al., 1988 |
| Colorectal | CEA | ZCE-025 | Patt et al., 1988 |
| Colorectal | CEA | AB2 | Griffin et al., 1988a |
| Colorectal | Cell surface AG | HT-29-15 | Cohn et al., 1987 |
| Colorectal | Secretory epithelium | 250-30.6 | Leydem et al., 1986 |
| Colorectal | Surface glycoprotein | 44X14 | Gallagher et al., 1986 |
| Colorectal | NS | A7 | Takahashi et al., 1988 |
| Colorectal | NS | GA73-3 | Munz et al., 1986 |
| Colorectal | NS | 791T/36 | Farrans et al., 1982 |
| Colorectal | Cell Membrane & Cytoplasmatic Ag | 28A32 | Smith et al., 1987 |
| Colorectal | CEA & Vindesin | 28.19.8 | Corvalen, 1987 |
| Colorectal | gp72 | X MMCO-791 | Byers et al., 1987 |
| Colorectal | high Mᵣ mucin | DU-PAN-2 | Lan et al., 1985 |
| Colorectal | high Mᵣ mucin | ID₃ | Gangopadhyay et al., 1985 |
| Colorectal | CEA 180 Kd GP | CEA 11-H5 | Wagener et al., 1984 |
| Colorectal | 60 Kd GP | 2C₈/2F₇ | Bhattacharya et al., 1985 |
| Colorectal | CA-19-9 (or GICA) | CA-19-9 (1116NS 19-9) | Atkinson et al., 1982 |
| Colorectal | Lewis a | PR5C5 | Imperial Cancer Research Technology Mab Listing |
| Colorectal | Lewis a | PR4D2 | Imperial Cancer Research Technology Mab Listing |
| Colorectal | Colon mucus | PR4D1 | Imperial Cancer Research Technology Mab Listing |
| **Melanoma** | P97^{a} | 4-1 | Woodbury et al., 1980 |
| Melanoma | P97^{a} | 8-2 M₁₇ | Brown, et al., 1981a |
| Melanoma | P97^{b} | 96-5 | Brown, et al., 1981 a |
| Melanoma | P97^{c} | 118-1,133-2, (113-2) | Brown, et al., 1981 a |
| Melanoma | P97^{c} | L₁, L₁₀, R₁₀ (R₁₉) | Brown et al., 1981b |
| Melanoma | P97^{d} | I₁₂ | Brown et al., 1981b |
| Melanoma | P97^{e} | K₅ | Brown et al., 1981b |
| Melanoma | P155 | 6-1 | Loop et al., 1981 |
| Melanoma | G_{D3} disialogan-gliosides | R24 | Dippold et al., 1980 |
| Melanoma | P210, p60, p250 | 5-1 | Loop et al., 1981 |
| Melanoma | P280 p440 | 225.28S | Wilson et al., 1981 |
| Melanoma | GP 94, 75, 70 & 25 | 465.12S | Wilson et al., 1981 |
| Melanoma | P240-P250, P450 | 9-2-27 | Reisfeld et al., 1982 |
| Melanoma | 100, 77, 75 Kd | F11 | Chee et al., 1982 |
| Melanoma | 94 Kd | 376.96S | Imai et al., 1982 |
| Melanoma | 4 GP Chains | 465.12S | Imai et al., 1982; Wilson et al., 1981 |
| Melanoma | GP 74 | 15-75 | Johnson & Reithmuller, 1982 |
| Melanoma | GP 49 | 15-95 | Johnson & Reithmuller, 1982 |
| Melanoma | 230 Kd | Mel-14 | Carrel et al., 1982 |
| Melanoma | 92 Kd | Mel-12 | Carrel et al., 1982 |
| Melanoma | 70 Kd | Me3-TB7 | Carrel et al., 1:387, 1982 |
| Melanoma | HMW MAA similar to 9-2-27 AG | 225.28SD | Kantor et al., 1982 |
| Melanoma | HMW MAA similar to 9-2-27 AG | 763.24TS | Kantor et al., 1982 |
| Melanoma | GP95 similar to 376-96S 465-12S | 705F6 | Stuhlmiller et al., 1982 |
| Melanoma | GP125 | 436910 | Saxton et al., 1982 |
| Melanoma | CD41 | M148 | Imperial Cancer Research Technology Mab listing |
| Gastrointestinal (GI) | high Mᵣ mucin | ID3 | Gangopadhyay et al., 1985 |
| Gallbladder, Pancreas, Stomach | high Mᵣ mucin | DU-PAN-2 | Lan et al., 1985 |
| Pancreas | NS | OV-TL3 | Poels et al., 1984 |
| Pancreas, Stomach, Esophagus | 'TAG-72' high Mᵣ mucin | B72-3 | Thor et al., 1986 |
| Stomach | 'CEA' 180 Kd GP | CEA 11-H5 | Wagener et al.,1984 |
| Pancreas | HMFG-2 > 400 Kd GP | 3-14-A3 | Burchell et al., 1983 |
| GI | NS | C COLI | Lemkin et al., 1984 |
| Pancreas, Stomach | CA 19-9 (or GICA) | CA-19-9 (1116NS 19-9) and CA50 | Szymendera, 1986 |
| Pancreas | CA125 GP | OC125 | Szymendera, 1986 |
| **Lung** | ₚ₁₈₅HER2 | 4D5, 3H4, 7C2, | Shepard et al., 1991 |
| Non-small-cell lung cancer (NSCLC) | | 6E9, 2C4, 7F3, 2H11, 3E8, 5B8, 7D3, SB8 | |
| NSCLC | high Mᵣ mucin/glycolipid | MO v2 | Miotti et al., 1985 |
| NSCLC | 'TAG -72' high Mᵣ mucin | B72-3 | Thor et al., 1986 |
| NSCLC | High Mᵣ mucin | DU-PAN-2 | Lan et al., 1985 |
| NSCLC | 'CEA' 180 kD GP | CEA 11-H5 | Wagener et al., 1984 |
| ***Malignant Glioma*** | Cytoplastic antigen that consists of 85HG-22 cells | MUG 8-22 | Stavrou, 1990 |
| Malignant Glioma | Cell surface Ag that consists of 85HG-\63 cells | MUC 2-63 | Stavrou, 1990 |
| Malignant Glioma | Cell surface Ag that consists of 86HG-39 cells | MUC 2-39 | Stavrou, 1990 |
| Malignant Glioma | Cell surface Ag that consists of 86HG-39 cells | MUG 7-39 | Stavrou, 1990 |
| ***GI**, **Other*** | P53 | PAb 240, PAb 246, PAb 1801 | Imperial Cancer Research Technology MaB Listing |
| Small, Round-Cell Tumors | Neural cell adhesion molecules | ERIC-1 | Imperial Cancer Research Technology MaB Listing |
| Medulloblastomas, Neuro-blastomas, Rhabdomyosarcomas | | M148 | Imperial Cancer Research Technology MaB Listing |
| Neuro-blastomas | | FMH25 | Imperial Cancer Research Technology MaB Listing |
| Kidneys & Glioblastomas | P155 | 6-1 | Loop et al., 1981 |
| Bladders & Laryngeal Tumors | "Ca Antigen" 350-390 kD | CA1 | Ashall et al., 1982 |
| Neuroblastoma | GD2 | 3F8 | Cheung et al., 1986 |
| Prostate | Gp48 48 kD GP | 4F₇/7A₁₀ | Bhattacharya et al., 1984 |
| Prostate | 60 kD GP | 2C₈/2F₇ | Bhattacharya et al., 1985 |
| Prostate | TMEFF2, TENB2, NC-2 | 2H8,48G2 | WO 2003075855; Glynne-Jones et al., 2001 |
| Thyroid | 'CEA' 180 kD GP | CEA 11-H5 | Wagener et al., 1984 |

As especially preferred recognition units for treating hematological tumors, antibodies or antibody fragments, such as CD19, CD20, CD40, CD22, CD25, CD5, CD52, CD10, CD2, CD7, CD33, CD38, CD40, CD72, CD4, CD21, CD5, CD37 and CD30, can also be mentioned.

As especially preferred recognition units for anti-angiogenic therapy, antibodies or fragments thereof, such as VCAM, CD31, ELAM, endoglin, VEGFRI/II, VEGFRvIII, scFv(14E1), αᵥβ₃,Tie1/2, TES23 (CD44ex6), phosphatidylserine, PSMA, VEGFR/VEGF complex or ED-B-fibronectin, can also be mentioned.

The compounds that are mentioned below are especially preferred according to the invention as effector elements:
(4S,7R,8S,9S,13Z,16S(E))-4,8-Dihydroxy-5,5,7,9,13-pentamethyl-16-[1-methyl-2-(2-methyl-thiazol-4-yl)-vinyl]-oxacyclohexadec-13-ene-2,6-dione,
(4S,7R,8S,9S,13Z,16S(E))-4,8-Dihydroxy-16-[2-(2-hydroxymethyl-thiazol-4-yl)-1-methyl-vinyl]-5,5,7,9,13-pentamethyl-oxacyclohexadec-13-ene-2,6-dione,
(4S,7R,8S,9S,13Z,16S(E))-16-[2-(2-Aminomethyl-thiazol-4-yl)-1-methyl-vinyl]-4,8-dihydroxy-5,5,7,9,13-pentamethyl-oxacyclohexadec-13-ene-2,6-dione,
(1S,3S(E),7S,10R,11S,12S,16R)-7,11-Dihydroxy-8,8,10,12,16-pentamethyl-3-[1-methyl-2-(2-methyl-thiazol-4-yl)-vinyl]-4,17-dioxa-bicyclo[14.1.0]heptadecane-5,9-dione,
(1S,3S(E),7S,10R,11S,12S,16R)-7,11-Dihydroxy-3-[2-(2-hydroxymethyl-thiazol-4-yl)-1-methyl-vinyl]-8,8,10,12,16-pentamethyl-4,17-dioxa bicyclo[14.1.0]-heptadecane-5,9-dione,
(1S,3S(E),7S,10R,11S,12S,16R)-3-[2-(2-Aminomethyl-thiazol-4-yl)-1-methylvinyl]-7,11-dihydroxy-8,8,10,12,16-pentamethyl-4,17-dioxa-bicyclo[14.1.0]-heptadecane-5,9-dione,
(4S,7R,8S,9S,13Z,16S(E))-4,8-Dihydroxy-7-ethyl-5,5,9,13-tetramethyl-16-[1-methyl-2-(2-methyl-thiazol-4-yl)-vinyl]-oxacyclohexadec-13-ene-2,6-dione,
(4S,7R,8S,9S,13Z,16S(E))-4,8-Dihydroxy-16-[2-(2-hydroxymethyl-thiazol-4-yl)-1-methyl-vinyl]-7-ethyl-5,5,9,13-tetramethyl-oxacyclohexadec-13-ene-2,6-dione,
(4S,7R,8S,9S,13Z,16S(E))-16-[2-(2-Aminomethyl-thiazol-4-yl)-1-methyl-vinyl]-4,8-dihydroxy-7-ethyl-5,5,9,13-tetramethyl-oxacyclohexadec-13-ene-2,6-dione,
(1S,3S(E),7S,10R,11S,12S,16R)-7,11-Dihydroxy-10-ethyl-8,8,12,16-tetramethyl-3-[1-methyl-2-(2-methyl-thiazol-4-yl)-vinyl]-4,17-dioxa-bicyclo[14.1.0]-heptadecane-5,9-dione;
(1S,3S(E),7S,10R,11S,12S,16R)-7,11-Dihydroxy-3-[2-(2-hydroxymethyl-thiazol-4-yl)-1-methyl-vinyl]-10-ethyl-8,8,12,16-tetramethyl-4,17-dioxa-bicyclo[14.1.0]heptadecane-5,9-dione;
(1S,3S(E),7S,10R,11S,12S,16R)-3-[2-(2-Aminomethyl-thiazol-4-yl)-1-methylvinyl]-7,11-dihydroxy-10-ethyl-8,8,12,16-tetramethyl-4,17-dioxa-bicyclo[14.1.0]-heptadecane-5,9-dione;
(4S,7R,8S,9S,13Z,16S(2))-4,8-Dihydroxy-5,5,7,9,13-pentamethyl-16-[1-fluoro-2-(2-methyl-thiazol-4-yl)-vinyl]-oxacyclohexadeo-l 3-ene-2,6-dione;
(4S,7R,8S,9S,13Z,16S(Z))-4,8-Dihydroxy-16-[2-(2-hydroxymethyl-thiazol-4-yl)-1-fluoro-vinyl]-5,5,7,9,13-pentamethyl-oxacyclohexadec-13-ene-2,6-dione;
(4S,7R,8S,9S,13Z,16S(Z))-16-[2-(2-Aminomethyl-thiazol-4-yl)-1-fluoro-vinyl]-4,8-dihydroxy 5,5,7,9,13-pentamethyl-oxacyclohexadec-13-ene-2,6-dione;
(1S,3S(Z),7S,10R,11S,12S,16R)-7,11-Dihydroxy-8,8,10,12,16-pentamethyl-3-[1-fluoro-2-(2-methyl-thiazol-4-yl)-vinyl]-4,17-dioxa-bicyclo[14.1.0]heptadecane-5,9-dione;
(1S,3S(Z),75,10R,11S,12S,16R)-7,11-Dihydroxy-3-[2-(2-hydroxymethyl-thiazol-4-yl)-1-fluoro-vinyl]-8,8,10,12,16-pentamethyl-4,17-dioxa-bicyclo[14.1.0]-heptadecane-5,9-dione;
(1S,3S(Z),75,10R,11S,12S,16R)-3-[2-(2-Aminomethyl-thiazol-4-yl)-1-fluorovinyl]-7,11-dihydroxy 8,8,10,12,16-pentamethyl-4,17-dioxa bicyclo[14.1.0]-heptadecane-5,9-dione;
(4S,7R,8S,9S,13Z,16S(Z))-4,8-Dihydroxy 5,5,7,9,13-pentamethyl-16-[1-chloro-2-(2-methyl-thiazol-4-yl)-vinyl]-oxacyclohexadec-13-ene-2,6-dione;
(4S,7R,8S,9S,13Z,16S(Z))-4,8-Dihydroxy-16-[2-(2-hydroxymethyl-thiazol-4-yl)-1-chloro-vinyl]-5,5,7,9,13-pentamethyl-oxacyclohexadec-13-ene-2,6-dione;
(4S,7R,8S,9S,13Z,16S(Z))-16-[2-(2-Aminomethyl-thiazol-4-yl)-1-chloro-vinyl]-4,8-dihydroxy-5,5,7,9,13-pentamethyl-oxacyclohexadec-13-ene-2,6-dione;
(1S,3S(Z),7S,10R,11S,12S,16R)-7,11-Dihydroxy-8,8,10,12,16-pentamethyl-3-[1-chloro-2-(2-methyl-thiazol-4-yl)-vinyl]-4,17-dioxa-bicyclo[14.1.0]heptadecane-5,9-dione;
(1S,3S(Z),7S,10R,11S,12S,16R)-7,11-Dihydroxy-3-[2-(2-hydroxymethyl-thiazol-4-yl)-1-chloro-vinyl]-8,8,10,12,16-pentamethyl-4,17-dioxa-bicyclo[14.1.0]-heptadecane-5,9-dione;
(1S,3S(Z),7S,10R,11S,12S,16R)-3-[2-(2-Aminomethyl-thiazol-4-yl)-1-chloro-vinyl]-7,11-dihydroxy-8,8,10,12,16-pentamethyl-4,17-dioxa-bicyclo[14.1.0]-heptadecane-5,9-dione;
(4S,7R,8S,9S,13Z,16S(Z))-4,8-Dihydroxy-7-ethyl-5,5,9,13-tetramethyl-16-[1-fluoro-2-(2-methyl-thiazol-4-yl)-vinyl]-oxacyclohexadec-13-ene-2,6-dione;
(4S,7R,8S,9S,13Z,16S(Z))-4,8-Dihydroxy-16-[2-(2-hydroxymethyl-thiazol-4-yl)-1-fluoro-vinyl]-7-ethyl-5,5,9,13-tetramethyl-oxacyclohexadec-13-ene-2,6-dione;
(4S,7R,8S,9S,13Z,16S(Z))-16-[2-(2-Aminomethyl-thiazol-4-yl)-1-fluoro-vinyl]-4,8-dihydroxy-7-ethyl-5,5,9,13-tetramethyl-oxacyclohexadec-13-ene-2,6-dione;
(1S,3S(Z),7S,10R,11S,12S,16R)-7,11-Dihydroxy-10-ethyl-8,8,12,16-tetramethyl-3-[1-fluoro-2-(2-methyl-thiazol-4-yl)-vinyl]-4,17-dioxa-bicyclo[14.1.0]-heptadecane-5,9-dione;
(1S,3S(2),7S,10R,11S,12S,16R)-7,11-Dihydroxy-3-[2-(2-hydroxymethyl-thiazol-4-yl)-1-fluoro-vinyl]-10-ethyl-8,8,12,16-tetramethyl-4,17-dioxa-bicyclo[14.1.0]-heptadecane-5,9-dione;
(1S,3S(Z),7S,10R,11S,12S,16R)-3-[2-(2-Aminomethyl-thiazol-4-yl)-1-fluorovinyl]-7,11-dihydroxy-10-ethyl-8,8,12,16-tetramethyl-4,17-dioxa-bicyclo[14.1.0]-heptadecane-5,9-dione;
(4S,7R,8S,9S,13Z,16S(Z))-4,8-Dihydroxy-7-ethyl-5,5,9,13-tetramethyl-16-[1-chloro-2-(2-methyl-thiazol-4-yl)-vinyl]-oxacyclohexadec-13-ene-2,6-dione;
(4S,7R,8S,9S,13Z,16S(Z))-4,8-Dihydroxy-16-[2-(2-hydroxymethyl-thiazol-4-yl)-1-chloro-vinyl]-7-ethyl-5,5,9,13-tetramethyl-oxacyclohexadec-13-ene-2,6-dione;
(4S,7R,8S,9S,13Z,16S(Z))-16-[2-(2-Aminomethyl-thiazol-4-yl)-l-chloro-vinyl]-4,8-dihydroxy-7-ethyl-5,5,9,13-tetramethyl-oxacyclohexadec-13-ene-2,6-dione;
(1S,3S(Z),7S,10R, 11S,12S,16R)-7,11-Dihydroxy-10-ethyl-8,8,12,16-tetramethyl-3-[1-chloro-2-(2-methyl-thiazol-4-yl)-vinyl]-4,17-dioxa-bicyclo[14.1.0]-heptadecane-5,9-dione;
(1S,3S(Z),7S,10R,11S,12S,16R)-7,11-Dihydroxy-3-[2-(2-hydroxymethyl-thiazol-4-yl)-1-chloro-vinyl]-10-ethyl-8,8,12,16-tetramethyl-4,17-dioxa-bicyclo[14.1.0]heptadecane-5,9-dione;
(1S,3S(Z),7S,10R,11S,12S,16R)-3-[2-(2-Aminomethyl-thiazol-4-yl)-1-chloro-vinyl]-7,11-dihydroxy-10-ethyl-8,8,12,16-tetramethyl-4,17-dioxa-bicyclo[14.1.0]-heptadecane-5,9-dione;
(4S,7R,8S,9S,13Z,16S(E))-4,8-Dihydroxy-5,5,7,9,13-pentamethyl-16-[1-methyl-2-(2-pyridyl)-vinyl]-oxacyclohexadee-13-ene-2,6-dione;
(1S,3S(E),7S,10R,11S,12S,16R)-7,11-Dihydroxy-8, 8,10,12,16-pentamethyl-3-[1-methyl-2-(2-pyridyl)-vinyl]-4,17-dioxa-bicyclo[14.1.0]heptadecane-5,9-dione;
(4S,7R,8S,9S,13Z,16S(E))-4,8-Dihydroxy-7-ethyl-5,5,9,13-tetramethyl-16-[1-methyl-2-(2-pyridyl)-vinyl]-oxacyclohexadec-13-ene-2,6-dione;
(1S,3S(E),7S,10R,11S,12S,16R)-7,11-Dihydroxy-10-ethyl-8,8,12,16-tetramethyl-3-[1-methyl-2-(2-pyridyl)-vinyl]-4,17-dioxa-bicyclo[14.1.0]heptadecane-5,9-dione;
(4S,7R,8S,9S,13Z,16S(Z))-4,8-Dihydroxy-5,5,7,9,13-pentamethyl-16-[1-fluoro-2-(2-pyridyl)-vinyl]-oxacyclohexadec-13-ene-2,6-dione;
(1S,3S(Z),7S,10R,11S,12S,16R)-7,11-Dihydroxy-8,8,10,12,16-pentamethyl-3-[1-fluoro-2-(2-pyridyl)-vinyl]-4,17-dioxa-bicyclo[14.1.0]heptadecane-5,9-dione;
(4S,7R,8S,9S,13Z,16S(Z))-4,8-Dihydroxy-5,5,7,9,13-pentamethyl-16-[1-chloro-2-(2-pyridyl)-vinyl]-oxacyclohexadec-13-ene-2,6-dione;
(1S,3S(Z),7S,10R,11S,12S,16R)-7,11-Dihydroxy-8,8,10,12,16-pentamethyl-3-[1-chloro-2-(2-pyridyl)-vinyl]-4,17-dioxa-bicyclo[14.1.0]heptadecane-5,9-dione;
(4S,7R,8S,9S,13Z,16S(Z))-4,8-Dihydroxy-7-ethyl-5,5,9,13-tetramethyl-16-[1-fluoro-2-(2-pyridyl)-vinyl]-oxacyclohexadeo-13-ene-2,6-dione;
(1S,3S(Z),7S,10R,1 S,12S,16R)-7,11-Dihydroxy-10-ethyl-8,8,12,16-tetramethyl-3-[1-fluoro-2-(2-pyridyl)-vinyl]-4,17-dioxa-bicyclo[14.1.0]heptadecane-5,9-dione;
(4S,7R,8S,9S,13Z,16S(Z))-4,8-Dihydroxy-7-ethyl-5,5,9,13-tetramethyl-16-[1-chloro-2-(2-pyridyl)-vinyl]-oxacyclohexadec-13-ene-2,6-dione;
(1S,3S(Z),7S,10R,11S,12S,16R)-7,1 1-Dihydroxy-10-ethyl-8,8,12,16-tetramethyl-3-[1-chloro-2-(2-pyridyl)-vinyl]-4,17-dioxa-bicyclo[14.1.0]heptadecane-5,9-dione;
(4S,7R,8S,9S,13Z,16S(E))-4,8-Dihydroxy-5,5,7,9,13-pentamethyl-16-[1-methyl-2-(2-methyl-oxazol-4-yl)vinyl]-oxacyclohexadeo-13-ene-2,6-dione;
(4S,7R,8S,9S,13Z,16S(E))-4,8-Dihydroxy-16-[2-(2-hydroxymethyl-oxazol-4-yl)-1-methyl-vinyl]-5,5,7,9,13-pentamethyl-oxacyclohexadec-13-ene-2,6-dione;
(4S,7R,8S,9S,13Z,16S(E))-16-[2-(2-Aminomethyl-oxazol-4-yl)-1-methyl-vinyl]-4,8-dihydroxy-5,5,7,9,13-pentamethyl-oxacyclohexadec-13-ene-2,6-dione;
(1S,3S(E),75,10R,11S,12S,16R)-7,11-Dihydroxy-8,8,10,12,16-pentamethyl-3-[1-methyl-2-(2-methyl-oxazol-4-yl)-vinyl]-4,17-dioxa-bicyclo[14.1.0]heptadecane-5,9-dione;
(1S,3S(E),7S,10R,11S,12S,16R)-7,11-Dihydroxy-3-[2-(2-hydroxymethyl-oxazol-4-yl)-1-methyl-vinyl]-8,8,10,12,16-pentamethyl-4,17-dioxa-bicyclo[14.1.0]-heptadecane-5,9-dione ;
(1S,3S(E),7S,10R,HS,12S,16R)-3-[2-(2-Aminomethyl-oxazol-4-yl)-1-methylvinyl]-7,11-dihydroxy-8,8,10,12,16-pentamethyl-4,17-dioxa-bicyclo[14.1.0]-heptadecane-5,9-dione;
(4S,7R,8S,9S,13Z,16S(E))-4,8-Dihydroxy-7-ethyl-5,5,9,13-tetramethyl-16-[1-methyl-2-(2-methyl-oxazol-4-yl)-vinyl]-oxacyclohexadec-13-ene-2,6-dione;
(4S,7R,8S,9S,13Z,16S(E))-4,8-Dihydroxy-16-[2-(2-hydroxymethyl-oxazol-4-yl)-1-methyl-vinyl]-7-ethyl-5,5,9,13-tetramethyl-oxacyclohexadec-13-ene-2,6-dione;
(4S,7R,8S,9S,13Z,16S(E))-16-[2-(2-Aminomethyl-oxazol-4-yl)-methyl-vinyl]-4,8-dihydroxy-7-ethyl-5,5,9,13-tetramethyl-oxacyclohexadec-13-ene-2,6-dione;
(1S,3S(E),7S,10R,11S,12S,16R)-7,11-Dihydroxy-10-ethyl-8,8,12,16-tetramethyl-3-[1-methyl-2-(2-methyl-oxazol-4-yl)-vinyl]-4,17-dioxa-bicyclo[14.1.0]-heptadecane-5,9-dione;
(1S,3S(E),75,10R,11S,12S,16R)-7,11-Dihydroxy-3-[2-(2-hydroxymethyl-oxazol-4-yl)-1-methyl-vinyl]-10-ethyl-8,8,12,16-tetramethyl-4,17-dioxa-bicyclo[14.1.0]heptadecane-5,9-dione;
(1 S,3S(E),7S,10R,11S,12S,16R)-3-[2-(2-Aminomethyl-oxazol-4-yl)-1-methyl-vinyl]-7,11-dihydroxy-10-ethyl-8,8,12,16-tetramethyl-4,17-dioxa-bicyclo[14.1.0]-heptadecane-5,9-dione;
(4S,7R,8S,9S,13Z,16S(Z))-4,8-Dihydroxy-5,5,7,9,13-pentamethyl-16-[1-fluoro-2-(2-methyl-oxazol-4-yl)-vinyl]-oxacyclohexadec-13-ene-2,6-dione;
(4S,7R,8S,9S,13Z,16S(Z))-4,8-Dihydroxy-16-[2-(2-hydroxymethyl-oxazol-4-yl)-1-fluoro-vinyl]-5,5,7,9,13-pentamethyl-oxacyclohexadec-13-ene-2,6-dione;
(4S,7R,8S,9S,13Z,16S(Z))-16-[2-(2-Aminomethyl-oxazol-4-yl)-1-fluoro-vinyl]-4,8-dihydroxy-5,5,7,9,13-pentamethyl-oxacyclohexadec-13-ene-2,6-dione;
(1S,3S(Z),7S,10R,11S,12S,16R)-7,11-Dihydroxy-8,8,10,12,16-pentamethyl-3-[1-fluoro-2-(2-methyl-oxazol-4-yl)-vinyl]-4,17-dioxa-bicyclo[14.1.0]heptadecane-5,9-dione;
(1S,3S(Z),7S,10R,11S,12S,16R)-7,11-Dihydroxy-3-[2-(2-hydroxymethyl-oxazol-4-yl)-1-fluoro-vinyl]-8,8,10,12,16-pentamethyl-4,17-dioxa-bicyclo[14.1.0]-heptadecane-5,9-dione;
(1S,3S(Z),7S,10R,11S,12S,16R)-3-[2-(2-Aminomethyl-oxazol-4-yl)-1-fluorovinyl]-7,11-dihydroxy-8,8,10,12,16-pentamethyl-4,17-dioxa-bicyclo[14.1.0]-heptadecane-5,9-dione;
(4S,7R,8S,9S,13Z,16S(Z))-4,8-Dihydroxy-5,5,7,9,13-pentamethyl-16-[1-chloro-2-(2-methyl-oxazol-4-yl)-vinyl]-oxacyclohexadec-13-ene-2,6-dione;
(4S,7R,8S,9S,13Z,16S(Z))-4,8-Dihydroxy-16-[2-(2-hydroxymethyl-oxazol-4-yl)-1-chloro-vinyl]-5,5,7,9,13-pentamethyl-oxacyclohexadeo-13-ene-2,6-dione;
(4S,7R,8S,9S,13Z,16S(Z))-16-[2-(2-Aminomethyl-oxazol-4-yl)-chloro-vinyl]-4,8-dihydroxy-5,5,7,9,13-pentamethyl-oxacyclohexadeo-13-ene-2,6-dione;
(1S,3S(Z),7S,10R,11S,12S,16R)-7,11-Dihydroxy-8,8,10,12,16-pentamethyl-3-[1-chloro-2-(2-methyl-oxazol-4-yl)-vinyl]-4,17-dioxa-bicyclo[14.1.0]heptadecane-5,9-dione;
(1S,3S(Z),7S,10R,11S,12S,16R)-7,11-Dihydroxy-3-[2-(2-hydroxymethyl-oxazol-4-yl)-1-chloro-vinyl]-8,8,10,12,16-pentamethyl-4,17-dioxa-bicyclo[14.1.0]-heptadecane-5,9-dione;
(1 S,3S(Z),7S,10R,11S,12S,16R)-3-[2-(2-Aminomethyl-oxazol-4-yl)-1-chloro-vinyl]-7,1 1-dihydroxy-8,8,10,12,16-pentamethyl-4,17-dioxa-bicyclo[14.1.0]-heptadecane-5,9-dione;
(4S,7R,8S,9S,13Z,16S(Z))-4,8-Dihydroxy-7-ethyl-5,5,9,13-tetramethyl-16-[1-fluoro-2-(2-methyl-oxazol-4-yl)-vinyl]-oxacyclohexadec-13-ene-2,6-dione;
(4S,7R,8S,9S,13Z,16S(Z))-4,8-Dihydroxy-16-[2-(2-hydroxymethyl-oxazol-4-yl)-1-fluoro-vinyl]-7-ethyl-5,5,9,13-tetramethyl-oxacyclohexadec-13-ene-2,6-dione;
(4S,7R,8S,9S,13Z,16S(Z))-16-[2-(2-Aminomethyl-oxazol-4-yl)-1-fluoro-vinyl]-4,8-dihydroxy-7-ethyl-5,5,9,13-tetramethyl-oxacyclohexadec-13-ene-2,6-dione;
(1S,3S(Z),7S,10R,11S,12S,16R)-7,11-Dihydroxy-10-ethyl-8,8,12,16-tetramethyl-3-[1-fluoro-2-(2-methyl-oxazol-4-yl)-vinyl]-4,17-dioxa-bicyclo[14.1.0]-heptadecane-5,9-dione;
(1S,3S(Z),7S,10R,11S,12S,16R)-7,11-Dihydroxy-3-[2-(2-hydroxymethyl-oxazol-4-yl)-1-fluoro-vinyl]-10-ethyl-8,8,12,16-tetramethyl-4,17-dioxa-bicyclo[14.1.0]heptadecane-5,9-dione;
(1S,3S(Z),7S,10R,11S,12S,16R)-3-[2-(2-Aminomethyl-oxazol-4-yl)-1-fluorovinyl]-7,11-dihydroxy-10-ethyl-8,8,12,16-tetramethyl-4,17-dioxa-bicyclo[14.1.0]-heptadecane-5,9-dione;
(4S,7R,8S,9S,13Z,16S(Z))-4,8-Dihydroxy-7-ethyl-5,5,9,13-tetramethyl-16-[1-chloro-2-(2-methyl-oxazol-4-yl)-vinyl]-oxacyclohexadec-13-ene-2,6-dione;
(4S,7R,8S,9S,13Z,16S(Z))-4,8-Dihydroxy-16-[2-(2-hydroxymethyl-oxazol-4-yl)-1-chloro-vinyl]-7-ethyl-5,5,9,13-tetramethyl-oxacyclohexadec-13-ene-2,6-dione;
(4S,7R,8S,9S,13Z,16S(Z))-16-[2-(2-Aminomethyl-oxazol-4-yl)-1-chloro-vinyl]-4,8-dihydroxy-7-ethyl-5,5,9,13-tetramethyl-oxacyclohexadec-13-ene-2,6-dione;
(1S,3S(Z),7S,10R,11S,12S,16R)-7,11-Dihydroxy-10-ethyl-8,8,12,16-tetramethyl-3-[1-chloro-2-(2-methyl-oxazol-4-yl)-vinyl]-4,17-dioxa-bicyclo[14.1.0]-heptadecane-5,9-dione;
(1S,3S(Z),7S,10R,11S,12S,16R)-7,11-Dihydroxy-3-[2-(2-hydroxymethyl-oxazol-4-yl)-1-chloro-vinyl]-10-ethyl-8,8,12,16-tetramethyl-4,17-dioxa-bicyclo[14.1.0]-heptadecane-5,9-dione;
(1S,3S(Z),7S,10R,11S,12S,16R)-3-[2-(2-Aminomethyl-oxazol-4-yl)-1-chloro-vinyl]-7,11-dihydroxy-10-ethyl-8,8,12,16-tetramethyl-4,17-dioxa-bicyclo[14.1.0]-heptadecane-5,9-dione;
(4S,7R,8S,9S,13Z,16S(E))-4,8-Dihydroxy-5,5,7,9,13-pentamethyl-16-[2-(2-methyl-thiazol-4-yl)-vinyl]-oxacyclohexadeo- 13-ene-2,6-dione;
(4S,7R,8S,9S,13Z,16S(E))-4,8-Dihydroxy-16-[2-(2-hydroxymethyl-thiazol-4-yl)-vinyl]-5,5,7,9,13-pentamethyl-oxacyclohexadec-13-ene-2,6-dione;
(4S,7R,8S,9S,13Z,16S(E))-16-[2-(2-Aminomethyl-thiazol-4-yl)-vinyl]-4,8-dihydroxy-5,5,7,9,13-pentamethyl-oxacyclohexadec-13-ene-2,6-dione;
(1S,3S(E),7S,10R,11S,12S,16R)-7,11-Dihydroxy-8,8,10,12,16-pentamethyl-3-[2-(2-methyl-thiazol-4-yl)-vinyl]-4,17-dioxa-bicyclo[14.1.0]heptadecane-5,9-dione;
(1S,3S(E),7S,10R,11S,12S,16R)-7,11-Dihydroxy-3-[2-(2-hydroxymethyl-thiazol-4-yl)-vinyl]-8,8,10,12,16-pentamethyl-4,17-dioxa-bicyclo[14.1.0]heptadecane-5,9-dione;
(1S,3S(E),7S,10R,11S,12S,16R)-3-[2-(2-Aminomethyl-thiazol-4-yl)vinyl]-7,11-dihydroxy-8,8,10,12,16-pentamethyl-4,17-dioxa-bicyclo[14.1.0]heptadecane-5,9-dione;
(4S,7R, 8 S,9S,13Z,16S (E))-4,8-Dihydroxy-7-ethyl-5,5,9,13-tetramethyl-16-[2-(2-methyl-thiazol-4-yl)-vinyl]-oxacyclohexadec-13-ene-2,6-dione;
(4S,7R,8S,9S,13Z,16S(E))-4,8-Dihydroxy-16-[2-(2-hydroxymethyl-thiazol-4-yl)-vinyl]-7-ethyl-5,5, 9,13-tetramethyl-oxacyclohexadec-13-ene-2,6-dione;
(4S,7R,8S,9S,13Z,16S(E))-16-[2-(2-Aminomethyl-thiazol-4-yl)-vinyl]-4,8-dihydroxy-7-ethyl-5,5,9,13-tetramethyl-oxacyclohexadec-13-ene-2,6-dione;
(1S,3S(E),7S,10R,11S,12S,16R)-7,11-Dihydroxy-10-ethyl-8,8,12,16-tetramethyl-3-[2-(2-methyl-thiazol-4-yl)-vinyl]-4,17-dioxa-bicyclo [14.1.0]heptadecane-5,9-dione;
(1S,3S(E),7S,10R,11S,12S,16R)-7,11-Dihydroxy-3-[2-(2-hydroxymethyl-thiazol-4-yl)-vinyl]-10-ethyl-8,8,12,16-tetramethyl-4,17-dioxa-bicyclo[14.1.0]-heptadecane-5,9-dione;
(1S,3S(E),7S,10R,11S,12S,16R)-3-[2-(2-Aminomethyl-thiazol-4-yl)vinyl]-7,11-dihydroxy-10-ethyl-8,8,12,16-tetramethyl-4,17-dioxa-bicyclo[14.1.0]heptadecane-5,9-dione;
(4S,7R,8S,9S,13Z,16S(E))-4;8-Dihydroxy-5,5,7,9,13-pentamethyl-16-[2-(2-pyridyl)-vinyl]-oxacyclohexadec-13-ene-2,6-dione;
(1S,3S(E),7S,10R,11S,12S,16R)-7,11-Dihydroxy-8,8,10,12,16-pentamethyl-3-[2-(2-pyridyl)-vinyl]-4,17-dioxa-bicyclo[14.1.0]heptadecane-5,9-dione;
(4S,7R,8S,9S,13Z,16S(E))-4,8-Dihydroxy-7-ethyl-5,5,9,13-tetramethyl-16-[2-(2-pyridyl)-vinyl]-oxacyclohexadec-13-ene-2,6-dione;
(1S,3S(E),7S,10R,11S,12S,16R)-7,11-Dihydroxy-10-ethyl-8,8,12,16-tetramethyl-3-[2-(2-pyridyl)-vinyl]-4,17-dioxa-bicyclo[14.1.0]heptadecane-5,9-dione;
(4S,7R,8S,9S,13Z,16S)-4,8-Dihydroxy-5,5,7,9,13-pentamethyl-16-(2-methyl-benzothiazol-5-yl)-oxacyclohexadec-13-ene-2,6-dione;
(4S,7R,8S,9S,13Z,16S)-4,8-Dihydroxy-16-(2-hydroxymethyl-benzothiazol-5-yl)-5,5,7,9,13-pentamethyl-oxacyclohexadec-13-ene-2,6-dione;
(4S,7R,8S,9S,13Z,16S)-16-(2-Aminomethyl-benzothiazol-5-yl)-4,8-dihydroxy-5,5,7,9,13-pentamethyl-oxacyclohexadec-13-ene-2,6-dione;
(1S,3S,7S,10R,11S,12S,16R)-7,11-Dihydroxy 8,8,10,12,16-pentamethyl-3-(2-methyl-benzothiazol-5-yl)-4,17-dioxa-bicyclo[14.1.0]heptadecane-5,9-dione;
(1S,3S,7S,10R,11S,12S,16R)-7,11-Dihydroxy-3-(2-hydroxymethyl-benzothiazol-5-yl)-8,8,10,12,16-pentamethyl-4,17-dioxa-bicyclo[14.1.0]heptadecane-5,9-dione;
(1S,3S,7S,10R,11S,12S,16R)-3-(2-Aminomethyl-benzothiazol-5-yl)-7,11-dihydroxy-8,8,10,12,16-pentamethyl-4,17-dioxa-bicyclo[14.1.0]heptadecane-5,9-dione;
(4S,7R,8S,9S,13Z,16S)-4,8-Dihydroxy-7-ethyl-5,5,9,13-tetramethyl-16-(2-methyl-benzothiazol-5-yl)-oxacyclohexadec-13-ene-2,6-dione;
(4S,7R,8S,9S,13Z,16S)-4,8-Dihydroxy-16-(2-hydroxymethyl-benzothiazol-5-yl)-7-ethyl-5,5,9,13-tetramethyl-oxacyclohexadec-13-ene-2,6-dione;
(4S,7R,8S,9S,13Z,16S)-16-(2-Aminomethyl-benzothiazol-5-yl)-4,8-dihydroxy-7-ethyl-5,5,9,13-tetramethyl-oxacyclohexadec-13-ene-2,6-dione;
(1S,3S,7S,10R,11S,12S,16R)-7,11-Dihydroxy-10-ethyl-8,8,12,16-tetramethyl-3-(2-methyl-benzothiazol-5-yl)-4,17-dioxa-bicyclo[14.1.0]heptadecane-5,9-dione;
(1S,3S,7S,10R,11S,12S,16R)-7,11-Dihydroxy-3-(2-hydroxymethyl-benzothiazol-5-yl)-10-ethyl-8,8,12,16-tetramethyl-4,17-dioxa-bicyclo[14.1.0]-heptadecane-5,9-dione;
(1S,3S,7S, 10R,11S,12S,16R)-3-(2-Aminomethyl-benzothiazol-5-yl)-7,11-dihydroxy-10-ethyl-8,8,12,16-tetramethyl-4,17-dioxa-bicyclo[14.1.0]heptadecane-5,9-dione;
(4S,7R,8S,9S,13Z,16S)-4,8-Dihydroxy-7-propyl-5,5,9,13-tetramethyl-16-(2-methyl-benzothiazol-5-yl)-oxacyclohexadec-13-ene-2,6-dione;
(4S,7R,8S,9S,13Z,16S)-4,8-Dihydroxy-16-(2-hydroxymethyl-benzothiazol-5-yl)-7-propyl-5,5,9,13-tetramethyl-oxacyclohexadec-13-ene-2,6-dione;
(4S,7R,8S,9S,13Z,16S)-16-(2-Aminomethyl-benzothiazol-5-yl)-4,8-dihydroxy-7-propyl-5,5,9,13-tetramethyl-oxacyclohexadeo-l3-ene-2,6-dione;
(1S,3S,7S,10R,11S,12S,16R)-7,11-Dihydroxy-10-propyl-8,8,12,16-tetramethyl-3-(2-methyl-benzothiazol-5-yl)-4,17-dioxa-bicyclo[14.1.0]heptadecane-5,9-dione;
(1S,3S,7S,10R,11S,12S,16R)-7,11-Dihydroxy-3-(2-hydroxymethyl-benzothiazol-5-yl)-10-propyl-8,8,12,16-tetramethyl-4,17-dioxa-bicyclo[14.1.0]-heptadecane-5,9-dione;
(1S,3S,7S,10R,11S,12S,16R)-3-(2-Aminomethyl-benzothiazol-5-yl)-7,11-dihydroxy-10-propyl-8,8,12,16-tetramethyl-4,17-dioxa-bicyclo[14.1.0]heptadecane-5,9-dione;
(4S,7R,8S,9S,13Z,16S)-4,8-Dihydroxy-7-butyl-5,5,9,13-tetramethyl-16-(2-methyl-benzothiazol-5-yl)-oxacyclohexadec-13-ene-2,6-dione;
(4S,7R,8S,9S,13Z,16S)-4,8-Dihydroxy-16-(2-hydroxymethyl-benzothiazol-5-yl)-7-butyl-5,5,9,13-tetramethyl-oxacyclohexadec-13-ene-2,6-dione;
(4S,7R,8S,9S,13Z,16S)-16-(2-Aminomethyl-benzothiazol-5-yl)-4,8-dihydroxy-7-butyl-5,5,9,13-tetramethyl-oxacyclohexadec-13-ene-2,6-dione;
(1S,3S,7S,10R,11S,12S,16R)-7,11-Dihydroxy-10-butyl-8,8,12,16-tetramethyl-3-(2-methyl-benzothiazol-5-yl)-4,17-dioxa-bicyclo[14.1.0]heptadecane-5,9-dione;
(1S,3S,7S,10R,11S,12S,16R)-7,11-Dihydroxy-3-(2-hydroxymethyl-benzothiazol-5-yl)-10-butyl-8,8,12,16-tetramethyl-4,17-dioxa-bicyclo[14.1.0]-heptadecane-5,9-dione;
(1S,3S,7S,10R,11S,12S,16R)-3-(2-Aminomethyl-benzothiazol-5-yl)-7,11-dihydroxy-10-butyl-8,8,12,16-tetramethyl-4,17-dioxa-bicyclo[14.1.0]heptadecane-5,9-dione;
(4S,7R,8S,9S,13Z,16S)-4,8-Dihydroxy-7-allyl-5,5,9,13-tetramethyl-16-(2-methyl-benzothiazol-5-yl)-oxacyclohexadec-13-ene-2,6-dione;
(4S,7R,8S,9S,13Z,16S)-4,8-Dihydroxy-16-(2-hydroxymethyl-benzothiazol-5-yl)-7-allyl-5,5,9,13-tetramethyl-oxacyclohexadec-13-ene-2,6-dione;
(4S,7R,8S,9S,13Z,16S)-16-(2-Aminomethyl-benzothiazol-5-yl)-4,8-dihydroxy-7-allyl-5,5,9,13-tetramethyl-oxacyclohexadec-13-ene-2,6-dione;
(1S,3S,7S,10R,11 S,12S,16R)-7,11-Dihydroxy-10-alkyl-8,8,12,16-tetramethyl-3-(2-methyl-benzothiazol-5-yl)-4,17-dioxa-bicyclo[14.1.0]heptadecane-5,9-dione;
(1S,3S,7S,10R,11S,12S,16R)-7,11-Dihydroxy-3-(2-hydroxymethyl-benzothiazol-5-yl)-10-allyl-8,8,12,16-tetramethyl-4,17-dioxa-bicyclo[14.1.0]-heptadecane-5,9-dione;
(1S,3S,7S,10R,11S,12S,16R)-3-(2-Aminomethyl-benzothiazol-5-yl)-7,11-dihydroxy-10-allyl-8,8,12,16-tetramethyl-4,17-dioxa-bicyclo[14.1.0]heptadecane-5,9-dione;
(4S,7R,8S,9S,13Z,16S)-4,8-Dihydroxy-7-prop-2-inyl-5,5,9,13-tetramethyl-16-(2-methyl-benzothiazol-5-yl)-oxacyclohexadec-13-ene-2,6-dione;
(4S,7R,8S,9S,13Z,16S)-4,8-Dihydroxy-16-(2-hydroxymethyl-benzothiazol-5-yl)-7-prop-2-inyl-5,5,9,13-tetramethyl-oxacyclohexadec-13-ene-2,6-dione;
(4S,7R,8S,9S,13Z,16S)-16-(2-Aminomethyl-benzothiazol-5-yl)-4,8-dihydroxy-7-prop-2-inyl-5,5,9,13-tetramethyl-oxacyclohexadec-13-ene-2,6-dione;
(1S,3S,7S,10R,11S,12S,16R)-7,11-Dihydroxy-10-prop-2-inyl-8,8,12,16-tetramethyl-3-(2-methyl-benzothiazol-5-yl)-4,17-dioxa-bicyclo[14.1.0]heptadecane-5,9-dione;
(1S,3S,7S,10R,11S,12S,16R)-7,11-Dihydroxy-3-(2-hydroxymethyl-benzothiazol-5-yl)-10-prop-2-inyl-8,8,12,16-tetramethyl-4,17-dioxa-bicyclo[14.1.0]-heptadecane-5,9-dione;
(1S,3S,7S,10R,11S,12S,16R)-3-(2-Aminomethyl-benzothiazol-5-yl)-7,11-dihydroxy-10-prop-2-inyl-8,8,12,16-tetramethyl-4,17-dioxa-bicyclo[14.1.0]-heptadecane-5,9-dione;
(4S,7R,8S,9S,13Z,16S)-4,8-Dihydroxy-7-but-3-enyl-5,5,9,13-tetramethyl-16-(2-methyl-benzothiazol-5-yl)-oxacyclohexadeo-13-ene-2,6-dione;
(4S,7R,8S,9S,13Z,16S)-4,8-Dihydroxy-16-(2-hydroxymethyl-benzothiazol-5-yl)-7-but-3-enyl-5,5,9,13-tetramethyl-oxacyclohexadec-13-ene-2,6-dione;
(4S,7R,8S,9S,13Z,16S)-16-(2-Aminomethyl-benzothiazol-5-yl)-4,8-dihydroxy-7-but-3-enyl-5,5,9,13-tetramethyl-oxacyclohexadec-13-ene-2,6-dione;
(1S,3S,7S,10R,11S,12S,16R)-7,11-Dihydroxy-10-but-3-enyl-8,8,12,16-tetramethyl-3-(2-methyl-benzothiazol-5-yl)-4,17-dioxa-bicyclo[14.1.0]heptadecane-5,9-dione;
(1S,3S,7S,10R,11S,12S,16R)-7,11-Dihydroxy-3-(2-hydroxymethyl-benzothiazol-5-yl)-10-but-3-enyl-8,8,12,16-tetramethyl-4,17-dioxa-bicyclo[14.1.0]-heptadecane-5,9-dione;
(1S,3S,7S,10R,11S,12S,16R)-3-(2-Aminomethyl-benzothiazol-5-yl)-7,11-dihydroxy-10-but-3-enyl-8,8,12,16-tetramethyl-4,17-dioxa-bicyclo[14.1.0]heptadecane-5,9-dione;
(4S,7R,8S,9S,13Z,16S)-4,8-Dihydroxy-7-but-3-inyl-5,5,9,13-tetramethyl-16-(2-methyl-benzothiazol-5-yl)-oxacyclohexadec-13-ene-2,6-dione;
(4S,7R,8S,9S,13Z,16S)-4,8-Dihydroxy-16-(2-hydroxymethyl-benzothiazol-5-yl)-7-but-3-inyl-5,5,9,13-tetramethyl-oxacyclohexadec-13-ene-2,6-dione;
(4S,7R,8S,9S,13Z,16S)-16-(2-Aminomethyl-benzothiazol-5-yl)-4,8-dihydroxy-7-but-3-inyl-5,5,9,13-tetramethyl-oxacyclohexadec-13-ene-2,6-dione;
(1S,3S,7S,10R,11S,12S,16R)-7,11-Dihydroxy-10-but-3-inyl-8,8,12,16-tetramethyl-3-(2-methyl-benzothiazol-5-yl)-4,17-dioxa-bicyclo [14.1.0]heptadecane-5,9-dione;
(1S,3S,7S,10R,11S,12S,16R)-7,11-Dihydroxy-3-(2-hydroxymethyl-benzothiazol-5-yl)-10-but-3-inyl-8,8,12,16-tetramethyl-4,17-dioxa-bicyclo[14.1.0]-heptadecane-5,9-dione;
(1S,3S,7S,10R,11S,12S,16R)-3-(2-Aminomethyl-benzothiazol-5-yl)-7,11-dihydroxy-10-but-3-inyl-8,8,12,16-tetramethyl-4,17-dioxa-bicyclo[14.1.0]heptadecane-5,9-dione;
(4S,7R,8S,9S,13Z,16S)-4,8-Dihydroxy-5,5,7,9,13-pentamethyl-16-(2-methyl-benzoxazol-5-yl)-oxacyclohexadec-13-ene-2,6-dione;
(4S,7R,8S,9S,13Z,16S)-4,8-Dihydroxy-16-(2-hydroxymethyl-benzoxazol-5-yl)-5,5,7,9,13-pentamethyl-oxacyclohexadec-13-ene-2,6-dione;
(4S,7R,8S,9S,13Z,16S)-16-(2-Aminomethyl-benzoxazol-5-yl)-4,8-dihydroxy 5,5,7,9,13-pentamethyl-oxacyclohexadec-13-ene-2,6-dione;
(1S,3S,7S,10R,11S,12S,16R)-7,11-Dihydroxy-8,8,10,12,16-pentamethyl-3-(2-methyl-benzoxazol-5-yl)-4,17-dioxa-bicyclo[14.1.0]heptadecane-5,9-dione;
(1S,3S,7S,10R,11S,12S,16R)-7,11-Dihydroxy-3-(2-hydroxymethyl-benzoxazol-5-yl)-8,8,10,12,16-pentamethyl-4,17-dioxa-bicyclo[14.1.0]heptadecane-5,9-dione;
(1S,3S,7S,10R,11S,12S,16R)-3-(2-Aminomethyl-benzoxazol-5-yl)-7,11-dihydroxy-8,8,10,12,16-pentamethyl-4,17-dioxa-bicyclo[14.1.0]heptadecane-5,9-dione;
(4S,7R,8S,9S,13Z,16S)-4,8-Dihydroxy-7-ethyl-5,5,9,13-tetramethyl-16-(2-methyl-benzoxazol-5-yl)-oxacyclohexadec-13-ene-2,6-dione;
(4S,7R,8S,9S,13Z,16S)-4,8-Dihydroxy-16-(2-hydroxymethyl-benzoxazol-5-yl)-7-ethyl-5,5,9,13-tetramethyl-oxacyclohexadec-13-ene-2,6-dione;
(4S,7R,8S,9S,13Z,16S)-16-(2-Aminomethyl-benzoxazol-5-yl)-4,8-dihydroxy-7-ethyl-5,5,9,13-tetramethyl-oxacyclohexadeo-13-ene-2,6-dione;
(1S,3S,7S,10R,11S,12S,16R)-7,11-Dihydroxy-10-ethyl-8,8,12,16-tetramethyl-3-(2-methyl-benzoxazol-5-yl)-4,17-dioxa-bicyclo[14.1.0]heptadecane-5,9-dione;
(1S,3S,7S,10R,11S,12S,16R)-7,11-Dihydroxy-3-(2-hydroxymethyl-benzoxazol-5-yl)-10-ethyl-8,8,12,16-tetramethyl-4,17-dioxa-bicyclo[14.1.0]heptadecane-5,9-dione;
(1S,3S,7S,10R,1S,12S,16R)-3-(2-Aminomethyl-benzoxazol-5-yl)-7,1-dihydroxy-10-ethyl-8,8,12,16-tetramethyl-4,17-dioxa-bicyclo[14.1.0]heptadecane-5,9-dione;
(4S,7R,8S,9S,13Z,16S)-4,8-Dihydroxy-7-propyl-5,5,9,13-tetramethyl-16-(2-methyl-benzoxazol-5-yl)-oxacyclohexadec-13-ene-2,6-dione;
(4S,7R,8S,9S,13Z,16S)-4,8-Dihydroxy 16-(2-hydroxymethyl-benzoxazol-5-yl)-7-propyl-5,5,9,13-tetramethyl-oxacyclohexadec-13-ene-2,6-dione;
(4S,7R,8S,9S,13Z,16S)-16-(2-Aminomethyl-benzoxazol-5-yl)-4,8-dihydroxy-7-propyl-5,5,9,13-tetramethyl-oxacyclohexadeo- 13-ene-2,6-dione;
(1S,3S,7S,10R,11S,12S,16R)-7,11-Dihydroxy-10-propyl-8, 8,12,16-tetramethyl-3-(2-methyl-benzoxazol-5-yl)-4,17-dioxa-bicyclo[14.1.0]heptadecane-5,9-dione;
(1S,3S,7S,10R,11S,12S,16R)-7,11-Dihydroxy-3-(2-hydroxymethyl-benzoxazol-5-yl)-10-propyl-8,8,12,16-tetramethyl-4,17-dioxa-bicyclo[14.1.0]heptadecane-5,9-dione;
(1S,3S, 7S, 10R, 11S, 12S, 16R)-3-(2-Aminomethyl-benzoxazol-5-yl)-7,11-dihydroxy-10-propyl-8,8,12,16-tetramethyl-4,17-dioxa-bicyclo[14.1.0]heptadecane-5,9-dione;
(4S,7R,8S,9S,13Z,16S)-4,8-Dihydroxy-7-butyl-5,5,9,13-tetramethyl-16-(2-methyl-benzoxazol-5-yl)-oxacyclohexadec-13-ene-2,6-dione;
(4S,7R,8S,9S,13Z,16S)-4,8-Dihydroxy-16-(2-hydroxymethyl-benzoxazol-5-yl)-7-butyl-5,5,9,13-tetramethyl-oxacyclohexadec-13-ene-2,6-dione;
(4S,7R,8S,9S,13Z,16S)-16-(2-Aminomethyl-benzoxazol-5-yl)-4,8-dihydroxy-7-butyl-5,5,9,13-tetramethyl-oxacyclohexadec-13-ene-2,6-dione;
(1S,3S,7S,10R,11S,12S,16R)-7,11-Dihydroxy-10-butyl-8,8,12,16-tetramethyl-3-(2-methyl-benzoxazol-5-yl)-4,17-dioxa-bicyclo[14.1.0]heptadecane-5,9-dione;
(1S,3S,7S,10R,11S,12S,16R)7,11-Dihydroxy-3-(2-hydroxymethyl-benzoxazol-5-yl)-10-butyl-8,8,12,16-tetramethyl-4,17-dioxa-bicyclo[14.1.0]heptadecane-5,9-dione;
(1S,3S,7S,10R,11S,12S,16R)-3-(2-Aminomethyl-benzoxazol-5-yl)-7,11-dihydroxy-10-butyl-8, 8,12,16-tetramethyl-4,17-dioxa-bicyclo[14.1.0]heptadecane-5,9-dione;
(4S,7R,8S,9S,13Z,16S)-4,8-Dihydroxy-7-allyl-5,5,9,13-tetramethyl-16-(2-methyl-benzoxazol-5-yl)-oxacyclohexadec-13-ene-2,6-dione;
(4S,7R,8S,9S,13Z,16S)-4,8-Dihydroxy-16-(2-hydroxymethyl-benzoxazol-5-yl)-7-allyl-5,5,9,13-tetramethyl-oxacyclohexadec-13-ene-2,6-dione;
(4S,7R,8S,9S,13Z,16S)-16-(2-Aminomethyl-benzoxazol-5-yl)-4,8-dihydroxy-7-allyl-5,5,9,13-tetramethyl-oxacyclohexadec-13-ene-2,6-dione;
(1S,3S,7S,10R,11S,12S,16R)-7,11-Dihydroxy-10-allyl-8,8,12,16-tetramethyl-3-(2-methyl-benzoxazol-5-yl)-4,17-dioxa-bicyclo[14.1.0]heptadecane-5,9-dione;
(1S,3S,7S,10R,11S,12S,16R)-7,11-Dihydroxy-3-(2-hydroxymethyl-benzoxazol-5-yl)-10-allyl-8,8,12,16-tetramethyl-4,17-dioxa-bicyclo[14.1.0]heptadecane-5,9-dione;
(1S,3S,7S, 10R, 11S,12S,16R)-3-(2-Aminomethyl-benzoxazol-5-yl)-7,11-dihydroxy-10-allyl-8,8,12,16-tetramethyl-4,17-dioxa-bicyclo[14.1.0]heptadecane-5,9-dione;
(4S,7R,8S,9S,13Z,16S)-4,8-Dihydroxy-7-prop-2-inyl-5,5,9,13-tetramethyl-16-(2-methyl-benzoxazol-5-yl)-oxacyclohexadec-13-ene-2,6-dione;
(4S,7R,8S,9S,13Z,16S)-4,8-Dihydroxy-16-(2-hydroxymethyl-benzoxazol-5-yl)-7-prop-2-inyl-5,5,9,13-tetramethyl-oxacyclohexadec-13-ene-2,6-dione;
(4S,7R,8S,9S,13Z,16S)-16-(2-Aminomethyl-benzoxazol-5-yl)-4,8-dihydroxy-7-prop-2-inyl-5,5,9,13-tetramethyl-oxacyclohexadec-13-ene-2,6-dione;
(1S,3S,7S,10R,11S,12S,16R)7,11-Dihydroxy-10-prop-2-inyl-8,8,12,16-tetramethyl-3-(2-methyl-benzoxazol-5-yl)-4,17-dioxa-bicyclo[14.1.0]heptadecane-5,9-dione;
(1S,3S,7S,10R,11S,12S,16R)-7,11-Dihydroxy-3-(2-hydroxymethyl-benzoxazol-5-yl)-10-prop-2-inyl-8,8,12,16-tetramethyl-4,17-dioxa-bicyclo[14.1.0]heptadecane-5,9-dione;
(1S,3S,7S,10R,11 S,12S,16R)-3-(2-Aminomethyl-benzoxazol-5-yl)-7,11-dihydroxy-10-prop-2-inyl-8,8,12,16-tetramethyl-4,17-dioxa-bicyclo[14.1.0]-heptadecane-5,9-dione;
(4S,7R,8S,9S,13Z,16S)-4,8-Dihydroxy-7-but-3-enyl-5,5,9,13-tetramethyl-16-(2-methyl-benzoxazol-5-yl)-oxacyclohexadec-13-ene-2,6-dione;
(4S,7R,8S,9S,13Z,16S)-4,8-Dihydroxy-16-(2-hydroxymethyl-benzoxazol-5-yl)-7-but-3-enyl-5,5,9,13-tetramethyl-oxacyclohexadec-13-ene-2,6-dione;
(4S,7R,8S,9S,13Z,16S)-16-(2-Aminomethyl-benzoxazol-5-yl)-4,8-dihydroxy-7-but-3-enyl-5,5,9,13-tetramethyl-oxacyclohexadec-13-ene-2,6-dione;
(1S,3S,7S,10R,11S,12S,16R)-7,11-Dihydroxy-10-but-3-enyl-8,8,12,16-tetramethyl-3-(2-methyl-benzoxazol-5-yl)-4,17-dioxa-bicyclo[14.1.0]heptadecane-5,9-dione;
(1S,3S,7S,10R,11S,12S,16R)-7,11-Dihydroxy-3-(2-hydroxymethyl-benzoxazol-5-yl)-10-but-3-enyl-8,8,12,16-tetramethyl-4,17-dioxa-bicyclo[14.1.0]heptadecane-5,9-dione;
(1S,3S,7S,10R,11S,12S,16R)-3-(2-Aminomethyl-benzoxazol-5-yl)-7,11-dihydroxy-10-but-3-enyl-8,8,12,16-tetramethyl-4,17-dioxa-bicyclo[14.1.0]heptadecane-5,9-dione;
(4S,7R,8S,9S,13Z,16S)-4,8-Dihydroxy-7-but-3-inyl-5,5,9,13-tetramethyl-16-(2-methyl-benzoxazol-5-yl)-oxacyclohexadec-13-ene-2,6-dione;
(4S,7R,8S,9S,13Z,16S)-4,8-Dihydroxy-16-(2-hydroxymethyl-benzoxazol-S-yl)-7-but-3-inyl-5,5,9,13-tetramethyl-oxacyclohexadec-13-ene-2,6-dione;
(4S,7R,8S,9S,13Z,16S)-16-(2-Aminomethyl-benzoxazol-5-yl)-4,8-dihydroxy-7-but-3-inyl-5,5,9,13-tetramethyl-oxacyclohexadec-13-ene-2,6-dione;
(1S,3S,7S,10R,11S,12S,16R)-7,11-Dihydroxy-10-but-3-inyl-8,8,12,16-tetramethyl-3-(2-methyl-benzoxazol-5-yl)-4,17-dioxa-bicyclo[14.1.0]heptadecane-5,9-dione;
(1S,3S,7S,10R,11S,12S,16R)-7,11-Dihydroxy 3-(2-hydroxymethyl-benzoxazol-5-yl)-10-but-3-inyl-8,8,12,16-tetramethyl-4,17-dioxa-bicyclo[14.1.0]heptadecane-5,9-dione;
(1S,3S,7S,10R,11S,12S,16R)-3-(2-Aminomethyl-benzoxazol-5-yl)-7,11-dihydroxy-10-but-3-inyl-8,8,12,16-tetramethyl-4,17-dioxa-bicyclo[14.1.0]heptadecane-5,9-dione.

In a compound of general formula (I) according to the invention that contains one of the above-mentioned elements, the hydrogen atoms in the above-mentioned elements are replaced by radicals L¹-L³ in the positions that are indicated in formula (I), whereby radicals L¹-L³ have the above-indicated meanings.

The invention also relates to linkers of general formula III¹: 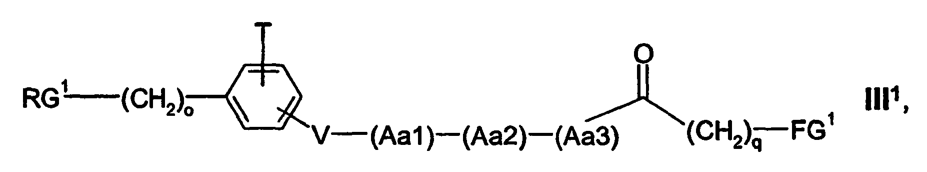 in which
- RG¹: can be an O=C=N group, and o, T, V, Aa1, Aa2, Aa3, q and FG¹ have the already mentioned meanings,
as well as linkers of general formula III² 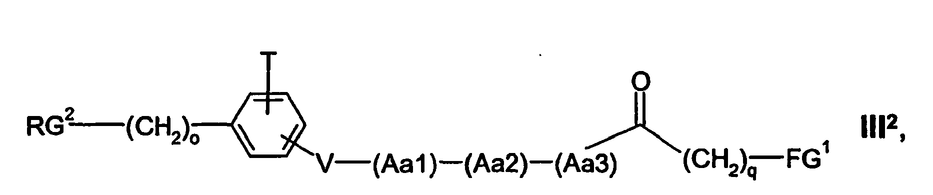 in which
- RG²: is a Hal-C(=O)-CHR²² group or a Hal-C(=O)-CHR²²-NR²³-C(=O) group or an R²⁶-C(=O)-O-C(=O)-CHR²² group or an R²⁶-C(=O)-O-C(=O)-CHR²²-NR²³-C(=O) group, whereby R²⁶ is C₁-C₁₀ alkyl, aryl, or aralkyl, and o, V, q, T, Aa1, Aa2, Aa3 and FG¹ have the meanings that are mentioned in Claim 1;
as well as linkers of general formula III³ 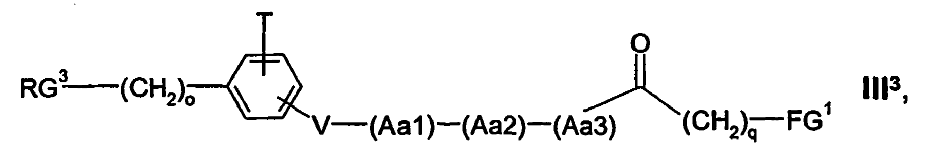 in which
RG³ can be an OH group or an NHR^{24a} group or a COOH group, and o, T, V, Aa1, Aa2, Aa3, q and FG¹ have the meanings that were already mentioned above.

Especially preferred according to the invention are linkers of general formulas III¹, III² or III³, whereby Aa1 represents a bond.

The invention also relates to processes
for reacting a linker of general formula III¹
with a compound of general formula I, in which the condition that at least one group L¹, L² or L⁴ represent a linker need not be met, and in which L¹ and/or L² and/or L⁴ have the meaning of a hydrogen atom, and free hydroxyl groups and/or amino groups that are not required for the reaction optionally are protected,
for reacting a linker of general formula III²
with a compound of general formula I, in which the condition that at least one group L¹, L² or L⁴ represent a linker need not be met, and in which L¹ and/or L² and/or L⁴ have the meaning of a hydrogen atom, and free hydroxyl groups and/or amino groups that are not required for the reaction are optionally protected,
for reacting a linker of general formula III³
with a compound of general formula I, in which the condition that at least one group L¹, L² or L⁴ represent a linker need not be met, and L¹ and/or L² and/or L⁴ have the meaning of a C(=O)Hal group, and free hydroxyl groups and/or amino groups that are not required for the reaction are optionally protected.

The invention also relates to the use of a compound of general formula I, whereby the substituents have the above-mentioned meanings, but the condition that at least one substituent L¹, L² or L⁴ represent a linker of general formula III need not be met, and at least one substituent L¹, L² or L⁴ represents hydrogen or a group C(=O)Cl in a process as described above.

The invention also relates to the use of a compound of general formula I, whereby the substituents have the above-mentioned meanings, but the condition that at least one substituent L¹, L² or L⁴ represent a linker of general formula III need not be met, and at least one substituent L¹, L² or L⁴ represents hydrogen or a group C(=O)Cl for the production of an effector-recognition unit conjugate as described above.

The invention also relates to the use of a linker of general formula III¹, III² or III³ for the production of an effector conjugate, as described above.

The invention also relates to the use of a linker of general formula III¹, III² or III³ for the production of an effector-recognition unit conjugate as described above.

The invention also relates to the use of a recognition unit, as described above, in one of the processes according to the invention for the production of an effector-recognition unit conjugate, as described above.

The invention also relates to the effector-recognition unit conjugates according to the invention for use as medications or for the production of a medication or a pharmaceutical composition.

Finally, the invention relates to the use of the effector-recognition unit conjugates according to the invention for the production of medications for the treatment of diseases that are linked with proliferative processes, such as tumors, inflammatory and/or neurodegenerative diseases, multiple sclerosis, Alzheimer's disease, or for the treatment of angiogenesis-associated diseases, such as the growth of tumors, rheumatoid arthritis, or diseases of the ocular fundus.

### Examples of Synthesis of Linkers (L)

### Example L1

### (2S,5S)-[5-{2-[11-(2,5-Dioxo-2,5-dihydro-pyrrol-1-yl)-undecanoylamino]-3-phenylpropionylamino}-5-(4-hydroxymethyl-phenylcarbamoyl)-pentyl]-carbamic acid allyl ester

### Example L1a

### 11-(2,5-Dioxo-2,5-dihydro-pyrrol-l-yl)-undecanoyl chloride

2.0 g (7.11 mmol) of 11-(2,5-dioxo-2,5-dihydro-pyrrol-1-yl)-undecanoic acid is mixed with 15.5 ml of thionyl chloride and refluxed for 15 minutes. After cooling, it is mixed with toluene and evaporated to the dry state. 2.13 g (max. 7.11 mmol) of the title compound, which is further reacted without purification, is isolated.

### Example L1b

### (S)-2-[11-(2,5-Dioxo-2,5-dihydro-pyrrol-1-yl)-undecanoylamino]-3-phenyl-propionic acid

The solution of 2.13 g (max. 7.11 mmol) of the compound, produced according to Example L1a, in 20 ml of dichloromethane is added in drops to the solution of 1.08 g of L-phenylalanine in 20 ml of pyridine within 10 minutes at 0°C. It is allowed to heat to 23°C and stirred for 1.5 more hours. It is poured into water, set at a pH of 4 to 5 by adding a 4M hydrochloric acid solution and extracted several times with dichloromethane. The combined organic extracts are washed with water and saturated sodium chloride solution and dried on sodium sulfate. The residue that is obtained after filtration and removal of the solvent is purified by chromatography. 1.34 g (3.13 mmol, 44%) of the title compound is isolated.

### Example L1c

### (S)-6-Allyloxycarbonylamino-2-(9H-fluoren-9-ylmethoxycarbonylamino)-hexanoic acid

The solution of 25 g (61.7 mmol) of (S)-6-amino-2-(9*H*-fluoren-9-ylmethoxycarbonylamino)-hexanoic acid in 275 ml of dioxane is mixed with 25 ml of a 25% potassium carbonate solution, and 6.55 ml of allyl chloroformate is added. It is stirred for 20 hours at 23°C, diluted with water and extracted with methyl-tert-butyl ether. The separated aqueous phase is acidified with a 2N hydrochloric acid and extracted several times with dichloromethane. The combined dichloromethane phases are dried on sodium sulfate. After filtration and removal of the solvent, 26.3 g (58.1 mmol, 94%) of the title compound, which is further reacted without purification, is isolated.

### Example L1d

### (S)-[5-Allyloxycarbonylamino-1-(4-hydroxymethyl-phenylcarbamoyl)-pentyl]-carbamic acid 9H-fluoren-9-yl methyl ester

The solution of 7.24 g (16.0 mmol) of the compound, produced according to Example L1c, in 72 ml of pyridine is mixed with 1.97 g of 4-aminobenzyl alcohol, 3.68 ml of di-tert.-butyldicarbonate, and it is stirred for 16 hours at 23°C. It is concentrated by evaporation, mixed with toluene and concentrated by evaporation again. The residue is mixed with 150 ml of ethyl acetate, and the suspension is stirred for another 16 hours. It is suctioned off, the residue is rewashed with a little cold ethyl acetate and diethyl ether, and dried. 6.22 g (11.2 mmol, 70%) of the title compound is isolated as a colorless, amorphous solid.

### Example L1e

### (S)-[5-Amino-5-(4-hydroxymethyl-phenylcarbamoyl)-pentyl]-carbamic acid allyl ester

6.22 g (11.2 mmol) of the compound that is produced according to Example L1d is mixed with 2.54 ml of diethylamine and heated for 3 hours to 50°C. It is concentrated by evaporation, and the residue is purified by chromatography on fine silica gel. 3.26 g (9.7 mmol, 87%) of the title compound is isolated.

### Example L1

### (2S,5S)-[5-{2-[11-(2,5-Dioxo-2,5-dihydro-pyrrol-1-yl)-undecanoylamino]-3-phenylpropionylamino}-5-(4-hydroxymethyl-phenylcarbamoyl)-pentyl]-carbamic acid allyl ester

The solution of 2.58 g (7.7 mmol) of the compound, produced according to Example L1e, in 130 ml of dimethylformamide is mixed with 3.3 g of the compound that is produced according to Example L1b, 3.46 ml of 2,4,6-trimethylpyridine, 2.81 g of (O-(7-azabenzotriazol-1-yl)-N,N',N',N'-tetramethyluroniumbexafluorophosphate), and it is stirred for 16 hours at 23°C. It is poured into 2N hydrochloric acid, extracted several times with ethyl acetate, the combined organic extracts are washed with water and dried on sodium sulfate. The residue that is obtained after filtration and removal of the solvent is purified by chromatography on fine silica gel. Subsequent recrystallization from ethyl acetate yields 2.04 g (2.73 mmol, 36%) of the title compound as a colorless solid.

¹H-NMR (d6-DMSO): = 0.94-1.83 (22H), 2.02 (2H), 2.74 (1H), 2.91-3.11 (3H), 3.38 (2H), 4.31-4.51 (5H), 4.57 (1H), 5.10 (1H), 5.14 (1H), 5.24 (1H), 5.88 (1H), 7.00 (2H), 7.09-7.32 (8H), 7.55 (2H), 8.00 (1H), 8.12 (1H), 9.94 (1H) ppm.

### Examples of Synthesis of Effector-Linker Units (EL)

### Example EL1

### Carboxylic acid 4-(6-allyloxycarbonylamino-2(S)-{2(S)-[11-(2,5-dioxo-2,5-dihydro-pyrrol-1-yl)-undecanoylamino]-3-phenyl-propionylamino}-hexanoylamino)-benzyl ester (4S,7R,8S,9S,13Z,16S)-[7-allyl-8-hydroxy-5,5,9,13-tetramethyl-16-(2-methylbenzothiazol-5-yl)-2,6-dioxo-oxacyclohexadec-13-en-4-yl]-ester

### Example EL1a

### Carboxylic acid 4-(6-allyloxycarbonylamino-2(S)-{2(S)-[11-(2,5-dioxo-2,5-dihydropyrrol-1-yl)-undecanoylamino]-3-phenyl-propionylamino}-hexanoylamino)-benzyl ester 4-(4S,7R,8S,9S,13Z,16S)-[7-allyl-8-(tert-butyl-dimethyl-silanyloxy)-5,5,9,13-tetramethyl-16-(2-methyl-benzothiazol-5-yl)-2,6-dioxo-oxacyclohexadec-13-en-4-yl]-ester

The solution of 375 mg (532 µmol) of (4S,7R,8S,9S,13Z,16S)-chloroformic acid-7-allyl-8-(*tert*-butyl-dimethyl-silanyloxy)-5,5,9,13-tetramethyl-16-(2-methyl-benzothiazol-5-yl)-2,6-dioxo-oxacyclohexadec-13-en-4-yl ester, which was produced analogously to the process described under DE 10234975.4 and DE 10305098.1, in 11.4 ml of dimethylformamide, is mixed with the solution of 2.07 g of the compound, produced according to Example L1, in 4.3 ml of dichloromethane, 274 mg of copper(I)chloride is added, and it is stirred for 5 hours at 23°C. It is poured into water, mixed with ethyl acetate, undissolved solid is filtered out, and it is extracted several times with ethyl acetate. The combined organic extracts are washed with saturated sodium chloride solution and dried on sodium sulfate. The residue that is obtained after filtration and removal of the solvent is purified by chromatography on fine silica gel. 66 mg (46.7 µmol, 9%) of the title compound is isolated.

### Example EL1

### Carboxylic acid 4-(6-allyloxycarbonylamino-2(S)-{2(S)-[11-(2,5-dioxo-2,5-dihydropyrrol-1-yl)-undecanoylamino]-3-phenyl-propionylamino}-hexanoylamino)-benzyl ester (4S,7R,8S,9S,13Z,16S)-[7-allyl-8-hydroxy-5,5,9,13-tetramethyl-16-(2-methylbenzothiazol-5-yl)-2,6-dioxo-oxacyclohexadec-13-en-4-yl]-ester

The solution of 66 mg (46.7 µmol) of the compound that is produced according to Example EL1 a in a mixture that consists of 0.77 ml of tetrahydrofuran and 0.77 ml of acetonitrile is mixed with 403 µl of hexafluorosilicic acid and 403 µl of hydrogen fluoride-pyridine complex. It is stirred for 23 hours at 23°C, mixed with water and extracted several times with ethyl acetate. The combined organic extracts are washed with saturated sodium bicarbonate solution and saturated sodium chloride solution and dried on sodium sulfate. The residue that is obtained after filtration and removal of the solvent is purified by chromatography on fine silica gel. 32 mg (24.6 µmol, 53%) of the title compound is isolated.

¹H-NMR (CDCl₃): δ = 0.99 (3H), 1.11 (3H), 1.14 (3H), 1.17-1.89 (26H), 1.70 (3H), 1.96 (2H), 2.18 (2H), 2.25-2.55 (5H), 2.67 (1H), 2.77 (3H), 2.93-3.26 (5H), 3.39 (1H), 3.50 (2H), 3.68 (1H), 4.38-4.78 (6H), 4.96-5.09 (3H), 5.13-5.23 (2H), 5.29 (1H), 5.55 (1H), 5.70 (1H), 5.90 (1H), 5.98 (1H), 6.09 (1H), 6.65 (1H), 6.68 (2H), 7.04 (2H), 7.11-7.34 (6H), 7.37 (1H), 7.47 (2H), 7.77 (1H), 7.96 (1H), 8.43 (1H) ppm.

### Example EL2

### Carboxylic acid 4-(6-allyloxycarbonylamino-2(S)-{2(S)-[11-(2,5-dioxo-2,5-dihydro-pyrrol-1-yl)-undecanoylamino]-3-phenyl-propionylamino}-hexanoylamino)-benzyl ester (1S,3S,7S,lOR,11S,12S,16R)-(10-allyl-11-hydroxy 8,8,12,16-tetramethyl-3-(2-methyl-benzothiazol-5-yl)-5,9-dioxo-4,17-dioxa-bicyclo[14.1.0]heptadec-7-yl]-ester

The solution of 27 mg (20.8 µmol) of the compound, produced according to Example EL1, in 1.2 ml of dichloromethane is mixed at -40°C under an atmosphere of dry argon with 0.5 ml of a 0.1 M solution of dimethyl dioxiram in acetone, and it is stirred for 3 hours at -40°C to -20°C. It is poured into a saturated sodium thiosulfate solution, diluted with water, and extracted several times with dichloromethane. The combined orgnaic extracts are washed with water and dried on sodium sulfate. The residue that is obtained after filtration and removal of the solvent is purified by chromatography on a silica gel plate. 13.3 mg (10.1 µmol, 49%) of the title compound as well as 7.6 mg (5.8 µmol, 28%) of carboxylic acid 4-(6-allyloxycarbonylamino-2(S)-{2(S)-[11-(2,5-dioxo-2,5-dihydro-pyrrol-1-yl)-undecanoylamino]-3-phenyl-propionylamino}-hexanoylamino)-benzyl ester (1R,3S,7S,10R,11S,12S,16S)-[10-allyl-11-hydroxy-8,8,12,16-tetramethyl-3-(2-methyl-benzothiazol-5-yl)-5,9-dioxo-4,17-dioxa-bicyclo[14.1.0]heptadec-7-yl]-ester are isolated.

¹H-NMR (CDCl₃): δ =1.00 (3H), 1.07-1.84 (33H), 1.48 (3H), 1.93 (1H), 2.13-2.57 (8H), 2.70 (1H), 2.79 (3H), 2.85 (1H), 3.02 (1H), 3.08-3.21 (3H), 3.45 (1H), 3.50 (2H), 3.69 (1H), 4.40-4.62 (4H), 4.67-4.76 (2H), 4.99 (1H), 5.04 (1H), 5.13 (1H), 5.19 (1H), 5.28 (1H), 5.44 (1H), 5.69 (1H), 5.90 (1H), 6.07 (1H), 6.27 (1H), 6.68 (2H), 6.87 (1H), 7.07 (2H), 7.10-7.21 (5H), 7.31 (1H), 7.47 (2H), 7.78 (1H), 7.93 (1H), 8.50 (1H), 9.67 (1H) ppm.

### Example EL3

### Carboxylic acid 4-(6-amino-2(S)-{2(S)-[11-(2,5-dioxo-2,5-dihydro-pyrrol-1-yl)-undecanoylamino]-3-phenyl-propionylamino}-hexanoylamino)-benzyl ester (1S,3S,7S,10R,11S,12S,16R)-[10-allyl-11-hydroxy-8,8,12,16-tetramethyl-3-(2-methyl-benzothiazol-5-yl)-5,9-dioxo-4,17-dioxa-bicyclo[14.1.0]heptadeo-7-yl]-ester

The solution of 13 mg (10.1 µmol) of the compound, produced according to Example EL2, in 0.5 ml of dichloromethane is mixed with 2.53 µl of phenylsilane, the solution of 772 µg of tetrakis-triphenylphosphonium palladium (0) in 1.0 ml of dichloromethane, and it is stirred for 2.5 hours at 23°C. It is concentrated by evaporation, and the residue is purified by chromatography on a silica gel plate. 4.2 mg (3.4 µmol, 34%) of the title compound is isolated.
MS: m/z = 1231

### Examples of the Synthesis of Effector-Linker-Recognition Unit Conjugates (ELE)

### Example ELE1

### Carboxylic acid 4-(6-amino-2(S)-{2(S)-[11-(3-(AP39r)-sulfanyl-2,5-dioxo-pyrrolidin-1-yl)-undecanoylamino]-3-phenyl-propionylamino}-hexanoylamino)-benzyl ester (1S,3S,7S,10R,11S,12S,16R)-[10-allyl-11-hydroxy-8,8,12,16-tetramethyl-3-(2-methyl-benzothiazol-5-yl)-5,9-dioxo-4,17-dioxa-bicyclo[14.1.0]heptadec-7-yl]-ester

50 µl of a 1.5 mmol solution of the effector-linker conjugate, produced according to Example EL3, in DMSO is added to 400 µl of a solution of the reduced antibody fragment AP39r with a content of 0.7 mg/ml, which was produced analogously to the process that is described under DE 10234975.4 and DE 10305098.1, mixed with 170 µl of PBS and incubated at 25°C for 1 hour. It is desalinated with a pre-equilibrated NAP5 column at a concentration with 400 µl of the reaction solution. After elution with PBS, the solution of the title compound is isolated. The dilution factor relative to the antibody fragment is about 1.8.
m/z = 26710 ± 20

## Claims

1. Effector conjugate of general formula (I): 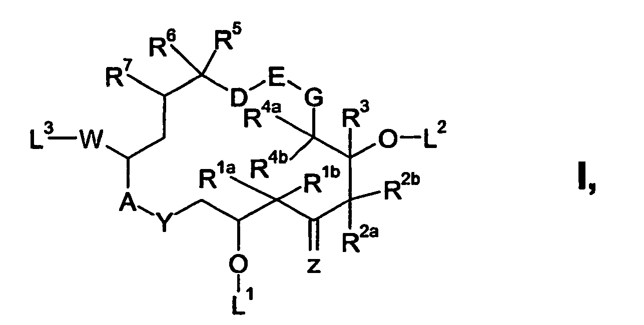 in which
R^{1a}, R^{1b}, independently of one another, are hydrogen, C₁-C₁₀ alkyl, aryl, aralkyl, or together a -(CH₂)ₘ group, in which m is 2 to 5,
R^{2a}, R^{2b}, independently of one another, are hydrogen, C₁-C₁₀ alkyl, aryl, aralkyl, or together a -(CH₂)ₙ group, in which n is 2 to 5, or C₂-C₁₀ alkenyl, or C₂-C₁₀ alkinyl,
R³ is hydrogen, C₁-C₁₀ alkyl, aryl or aralkyl, and
R^{4a}, R^{4b}, independently of one another, are hydrogen, C₁-C₁₀ alkyl, aryl, aralkyl, or together a -(CH₂)ₚ group, in which p is 2 to 5,
R⁵ is hydrogen, C₁-C₁₀ alkyl, aryl, aralkyl, CO₂H, CO₂alkyl, CH₂OH, CH₂Oalkyl, CH₂Oacyl, CN, CH₂NH₂, CH₂N(alkyl, acyl)_{1,2}, or CH₂Hal,
Hal is a halogen atom,
R⁶, R⁷, in each case, are hydrogen, or together an additional bond or together an oxygen atom, or together an NH group, or together an N-alkyl group, or together a CH₂ group, and
G is an oxygen atom or CH₂,
D-E is a group H₂C-CH₂, HC=CH, C≡C, CH(OH)-CH(OH), CH(OH)-CH₂, CH₂-CH(OH), 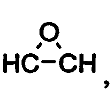 O-CH₂, or, if G represents a CH₂ group, D-E is also CH₂-O,
W is a group C(=X)R⁸, or a bi- or tricyclic aromatic or heteroaromatic radical,
L³ is hydrogen, or, if a radical in W contains a hydroxyl group, forms a group O-L⁴ with the latter, or, if a radical in W contains an amino group, forms a group NR²⁵-L⁴ with the latter,
R²⁵ is hydrogen or C₁-C₁₀ alkyl,
X is an oxygen atom, or two OR²⁰ groups, or a C₂-C₁₀ alkylenedioxy group, which should be straight-chain or branched, or H/OR⁹, or a CR¹⁰R¹¹ group,
R⁸ is hydrogen, C₁-C₁₀ alkyl, aryl, aralkyl, halogen or CN, and
R⁹ is hydrogen or a protective group PG^{X},
R¹⁰, R¹¹, in each case independently of one another, are hydrogen, C₁-C₂₀ alkyl, aryl, or aralkyl, or together with a methylene carbon atom form a 5- to 7-membered carbocyclic ring,
Z can represent oxygen or H/OR¹²,
R12 can represent hydrogen or a protective group PG^{Z},
A-Y can represent a group O-C(=O), O-CH₂, CH₂-C(=O), NR²¹-C(=O) or NR²¹-SO₂,
R²⁰ can represent C₁-C₂₀ alkyl,
R²¹ can represent a hydrogen atom or C₁-C₁₀ alkyl,
PG^{X}, PG^{Y}, and PG^{Z} can represent a protective group PG, and
L¹, L², L⁴. independently of one another, can represent hydrogen, a group C(=O)Cl, a group C(=S)Cl, a group PG^{Y} or a linker of general formula (III);
with the condition that at least one substituent L¹, L² or L⁴ represents a linker of general formula (III);
the linker of general formula (III) has the following structure, 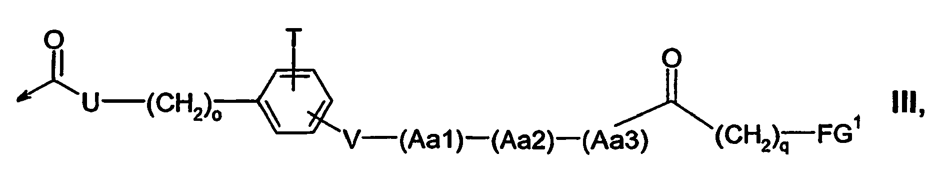 in which
T represents halogen, OH, O-alkyl, CO₂H, CO₂-alkyl, -NHR²³, -NO₂, -N₃, -CN, C₁-C₂₀-alkyl, C₁-C₂₀-acyl or C₁-C₂₀-acyloxy groups,
U represents a bond, oxygen, or NR^{24a},
o represents 0 to 5,
V represents oxygen or NR^{24b},
Aa1 represents a bond or a group of general formula IV and
Aa2, Aa3, independently of one another, represent a group of general formula IV 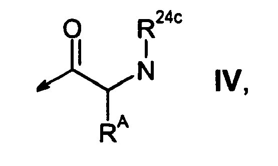 which is derived from a natural or unnatural amino acid HO-Aa1-H, HO-Aa2-H, or HO-Aa3-H of general formula IV' 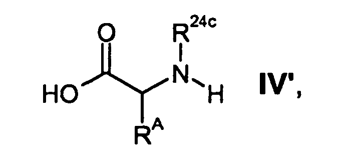
in which R^{A} can be the same or different in HO-Aa1-H, HO-Aa2-H, or HO-Aa3-H, and the Aa-substituent represents a natural or unnatural amino acid,
R²² can represent hydrogen, C₁-C₁₀ alkyl, aryl or aralkyl,
R²³ can represent hydrogen, C₁-C₁₀ alkyl, or C₁-C₁₀ acyl,
R^{24a}, R^{24b}, R^{24c}, independently of one another, can represent hydrogen or C₁-C₁₀ alkyl,
q can represent 1 to 20,
FG¹ can represent C₁-C₁₀ alkyl-S₃, 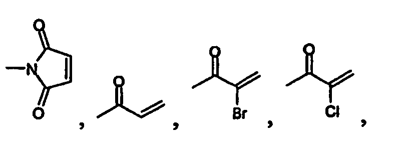 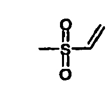 or CO₂H;
as a uniform isomer or a mixture of different isomers and/or as a pharmaceutically acceptable salt thereof.

2. Effector conjugate according to claim 1, whereby:
A-Y represents O-C(=O) or NR²¹-C(=O);
D-E represents an H₂C-CH₂ group or an HC=CH group;
G represents a CH₂ group;
Z represents an oxygen atom;
R^{1a}, R^{1b} in each case represent C₁-C₁₀ alkyl or together a -(CH₂)ₚ group with p equal to 2 or 3 or 4;
R^{2a}, R^{2b}, independently of one another, represent hydrogen, C₁-C₁₀ alkyl, C₂-C₁₀ alkenyl, or C₂-C₁₀ alkinyl;
R³ represents hydrogen;
R^{4a}, R^{4b}, independently of one another, represent hydrogen or C₁-C₁₀ alkyl;
R⁵ represents hydrogen or C₁-C₄ alkyl or CH₂OH or CH₂NH₂ or CH₂N(alkyl, acyl)_{1,2} or CH₂Hal;
R⁶ and R⁷ together represent an additional bond or together an NH group or together an N-alkyl group or together a CH₂ group or together an oxygen group;
W represents a group C(=X)R⁸ or a 2-methylbenzothiazol-5-yl radical or a 2-methylbenzoxazol-5-yl radical or a quinolin-7-yl radical or a 2-aminomethylbenzothiazol-5-yl radical or a 2-hydroxymethylbenzothiazol-5-yl radical or a 2-aminomethylbenzoxazol-5-yl radical or a 2-hydroxymethyl-benzoxazol-5-yl radical;
X represents a CR¹⁰R¹¹ group;
R⁸ represents hydrogen or C₁-C₄ alkyl or a fluorine atom or a chlorine atom or a bromine atom;
R¹⁰/R¹ represent hydrogen/2-methylthiazol-4-yl or hydrogen/2-pyridyl or hydrogen/2-methyloxazol-4-yl or hydrogen/2-aminomethylthiazol-4-yl or hydrogen/2-aminomethyloxazol-4-yl or hydrogen/2-hydroxymethylthiazol-4-yl or hydrogen/2-hydroxymethyloxazol-4-yl.

3. Effector conjugate according to claim 1 or 2, whereby the effector element is selected from the group that consists of:
(4S,7R,8S,9S,13Z,16S(E))-4,8-Dihydroxy-5,5,7,9,13-pentamethyl-16-[1-methyl-2-(2-methyl-thiazol-4-yl)-vinyl]-oxacyclohexadec-13-ene-2,6-dione;
(4S,7R,8S,9S,13Z,16S(B))-4,8-Dihydroxy-16-[2-(2-hydroxymethyl-thiazol-4-yl)-1-methyl-vinyl]-5,5,7,9,13-pentamethyl-oxacyclohexadeo-13-ene-2,6-dione;
(4S,7R,8S,9S,13Z,16S(E))-16-[2-(2-Aminomethyl-thiazol-4-yl)-1-methyl-vinyl]-4,8-dihydroxy-5,5,7,9,13-pentamethyl-oxacyclohexadec-13-ene-2,6-dione;
(1S,3S(E),7S,10R,11S,12S,16R)-7,11-Dihydroxy-8,8,10,12,16-pentamethyl-3-[1-methyl-2-(2-methyl-thiazol-4-yl)-vinyl]-4,17-dioxa-bicyclo[14.1.0]heptadecane-5,9-dione;
(1S,3S(E),7S,10R,11S,12S,16R)-7,11-Dihydroxy-3-[2-(2-hydroxymethyl-thiazol-4-yl)-1-methyl-vinyl]-8,8,10,12,16-pentamethyl-4,17-dioxa-bicyclo[14.1.0]-heptadecane-5,9-dione;
(1 S,3S(E),7S,10R,11S,12S,16R)-3-[2-(2-Aminomethyl-thiazol-4-yl)-1-methylvinyl]-7,11-dihydroxy-8,8,10,12,16-pentamethyl-4,17-dioxa-bicyclo[14.1.0]-heptadecane-5,9-dione;
(4S,7R,8S,9S,13Z,16S(E))-4,8-Dihydroxy-7-ethyl-5,5,9,13-tetramethyl-16-[1-methyl-2-(2-methyl-thiazol-4-yl)-vinyl]-oxacyclohexadec-13-ene-2,6-dione;
(4S,7R,8S,9S,13Z,16S(E))-4,8-Dihydroxy-16-[2-(2-hydroxymethyl-thiazol-4-yl)-1-methyl-vinyl]-7-ethyl-5,5,9,13-tetramethyl-oxacyclohexadec-13-ene-2,6-dione;
(4S,7R,8S,9S,13Z,16S(E))-16-[2-(2-Aminomethyl-thiazol-4-yl)-1-methyl-vinyl]-4,8-dihydroxy-7-ethyl-5,5,9,13-tetramethyl-oxacyclohexadec-13-ene-2,6-dione;
(1S,3S(E),7S,10R,11S,12S,16R)-7,11-Dihydroxy-10-ethyl-8,8,12,16-tetramethyl-3-[1-methyl-2-(2-methyl-thiazol-4-yl)-vinyl]-4,17-dioxa-bicyclo[14.1.0]-heptadecane-5,9-dione;
(1S,3S(E),7S,10R,11S,12S,16R)7,11-Dihydroxy-3-[2-(2-hydroxymethyl-thiazol-4-yl)-1-methyl-vinyl]-10-ethyl-8,8,12,16-tetramethyl-4,17-dioxa-bicyclo[14.1.0]heptadecane-5,9-dione ;
(1S,3S(E),7S,10R,11S,12S,16R)-3-[2-(2-Aminomethyl-thiazol-4-yl)-1-methylvinyl]-7,11-dihydroxy-10-ethyl-8,8,12,16-tetramethyl-4,17-dioxa-bicyclo[14.1.0]-heptadecane-5,9-dione;
(4S,7R,8S,9S,13Z,16S(Z))-4,8-Dihydroxy-5,5,7,9,13-pentamethyl-16-[1-fluoro-2-(2-methyl-thiazol-4-yl)-vinyl]-oxacyclohexadec-13-ene-2,6-dione;
(4S,7R,8S,9S,13Z,16S(Z))-4,8-Dihydroxy-16-[2-(2-hydroxymethyl-thiazol-4-yl)-1-fluoro-vinyl]-5,5,7,9,13-pentamethyl-oxacyclohexadec-13-ene-2,6-dione;
(4S,7R,8S,9S,13Z,16S(Z))-16-[2-(2-Aminomethyl-thiazol-4-yl)-1-fluoro-vinyl]-4,8-dihydroxy-5,5,7,9,13-pentamethyl-oxacyclohexadec-13-ene-2,6-dione;
(1S,3S(Z),7S,10R,11S,12S,16R)-7,11-Dihydroxy-8,8,10,12,16-pentamethyl-3-[1-fluoro-2-(2-methyl-thiazol-4-yl)-vinyl]-4,17-dioxa-bicyclo[14.1.0]heptadecane-5,9-dione;
(1S,3S(Z),7S,10R,11S,12S,16R)-7,11-Dihydroxy-3-[2-(2-hydroxymethyl-thiazol-4-yl)-1-fluoro-vinyl]-8,8,10,12,16-pentamethyl-4,17-dioxa-bicyclo[14.1.0]-heptadecane-5,9-dione;
(1S,3S(Z),7S,10R,11S,12S,16R)-3-[2-(2-Aminomethyl-thiazol-4-yl)-1-fluoro-vinyl]-7,11-dihydroxy-8,8,10,12,16-pentamethyl-4,17-dioxa-bicyclo[14.1.0]-heptadecane-5,9-dione;
(4S,7R,8S,9S,13Z,16S(Z))-4,8-Dihydroxy-5,5,7,9,13-pentamethyl-16-[1-chloro-2-(2-methyl-thiazol-4-yl)-vinyl]-oxacyclohexadeo-13-ene-2,6-dione;
(4S,7R,8S,9S,13Z,16S(Z))-4,8-Dihydroxy-16-[2-(2-hydroxymethyl-thiazol-4-yl)-1-chloro-vinyl]-5,5,7,9,13-pentamethyl-oxacyclohexadec-13-ene-2,6-dione;
(4S,7R,8S,9S,13Z,16S(Z))-16-[2-(2-Aminomethyl-thiazol-4-yl)-1-chloro-vinyl]-4,8-dihydroxy-5,5,7,9,13-pentamethyl-oxacyclohexadec-13-ene-2,6-dione;
(1S,3S(Z),7S,10R,11S,12S,16R)-7,11-Dihydroxy-8,8,10,12,16-pentamethyl-3-[1-chloro-2-(2-methyl-thiazol-4-yl)-vinyl]-4,17-dioxa-bicyclo[14.1.0]heptadecane-5,9-dione;
(1S,3S(Z),7S,10R,11S,12S,16R)-7,11-Dihydroxy-3-[2-(2-hydroxymethyl-thiazol-4-yl)-1-chloro-vinyl]-8,8,10,12,16-pentamethyl-4,17-dioxa-bicyclo[14.1.0]-heptadecane-5,9-dione;
(1S,3S(Z),7S,10R,11S,12S,16R)-3-[2-(2-Aminomethyl-thiazol-4-yl)-1-chloro-vinyl]-7,11-dihydroxy-8,8,10,12,16-pentamethyl-4,17-dioxa-bicyclo[14.1.0]-heptadecane-5,9-dione;
(4S,7R,8S,9S,13Z,16S(Z))-4,8-Dihydroxy-7-ethyl-5,5,9,13-tetramethyl-16-[1-fluoro-2-(2-methyl-thiazol-4-yl)-vinyl]-oxacyclohexadec-13-ene-2,6-dione;
(4S,7R,8S,9S,13Z,16S(Z))-4,8-Dihydroxy-16-[2-(2-hydroxymethyl-thiazol-4-yl)-1-fluoro-vinyl]-7-ethyl-5,5,9,13-tetramethyl-oxacyclohexadec-13-ene-2,6-dione;
(4S,7R,8S,9S,13Z,16S(Z))-16-[2-(2-Aminomethyl-thiazol-4-yl)-1-fluoro-vinyl]-4,8-dihydroxy-7-ethyl-5,5,9,13-tetramethyl-oxacyclohexadec-13-ene-2,6-dione;
(1S,3S(Z),7S,10R,11S,12S,16R)-7,11-Dihydroxy-10-ethyl-8, 8,12,16-tetramethyl-3-[1-fluoro-2-(2-methyl-thiazol-4-yl)-vinyl]-4,17-dioxa-bicyclo[14.1.0]-heptadecane-5,9-dione;
(1S,3S(Z),7S,10R,11S,12S,16R)-7,11-Dihydroxy-3-[2-(2-hydroxymethyl-thiazol-4-yl)-1-fluoro-vinyl]-10-ethyl-8,8,12,16-tetramethyl-4,17-dioxa-bicyclo[14.1.0]-heptadecane-5,9-dione;
(1 S,3S(Z),75,10R,11S,12S,16R)-3-[2-(2-Aminomethyl-thiazol-4-yl)-1-fluoro-vinyl]-7,1 1-dihydroxy-10-ethyl-8,8,12,16-tetramethyl-4,17-dioxa-bicyclo[14.1.0]-heptadecane-5,9-dione;
(4S,7R,8S,9S,13Z,16S(Z))-4,8-Dihydroxy-7-ethyl-5,5,9,13-tetramethyl-16-[1-chloro-2-(2-methyl-thiazol-4-yl)-vinyl]-oxacyclohexadec-13-ene-2,6-dione;
(4S,7R,8S,9S,13Z,16S(Z))-4,8-Dihydroxy-16-[2-(2-hydroxymethyl-thiazol-4-yl)-1-chloro-vinyl]-7-ethyl-5,5,9,13-tetramethyl-oxacyclohexadec-13-ene-2,6-dione;
(4S,7R,8S,9S,13Z,16S(Z))-16-[2-(2-Aminomethyl-thiazol-4-yl)-1-chloro-vinyl]-4,8-dihydroxy-7-ethyl-5,5,9,13-tetramethyl-oxacyclohexadec-13-ene-2,6-dione;
(1S,3S(Z),7S,10R,11S,12S,16R)-7,11-Dihydroxy-10-ethyl-8,8,12,16-tetramethyl-3-[1-chloro-2-(2-methyl-thiazol-4-yl)-vinyl]-4,17-dioxa-bicyclo[14.1.0]-heptadecane-5,9-dione;
(1S,3S(Z),7S,10R,11S,12S,16R)-7,11-Dihydroxy-3-[2-(2-hydroxymethyl-thiazol-4-yl)-1-chloro-vinyl]-10-ethyl-8,8,12,16-tetramethyl-4,17-dioxa-bicyclo[14.1.0]-heptadecane-5,9-dione;
(1S,3S(Z),75,10R,11S,12S,16R)-3-[2-(2-Aminomethyl-thiazol-4-yl)-1-chloro-vinyl]-7,11-dihydroxy-10-ethyl-8,8,12,16-tetramethyl-4,17-dioxa-bicyclo[14.1.0]-heptadecane-5,9-dione;
(4S,7R,8S,9S,13Z,16S(E))-4,8-Dihydroxy-5,5,7,9,13-pentamethyl-16-[1-methyl-2-(2-pyridyl)-vinyl]-oxacyclohexadec-13-ene-2,6-dione;
(1S,3S(E),7S,10R,11S,12S,16R)-7,11-Dihydroxy-8,8,10,12,16-pentamethyl-3-[1-methyl-2-(2-pyridyl)-vinyl]-4,17-dioxa-bicyclo[14.1.0]heptadecane-5,9-dione;
(4S,7R,8S,9S,13Z,16S(E))-4,8-Dihydroxy-7-ethyl-5,5,9,13-tetramethyl-16-[1-methyl-2-(2-pyridyl)-vinyl]-oxacyclohexadec-13-ene-2,6-dione;
(1S,3S(E),7S,10R,11S,12S,16R)-7,11-Dihydroxy-10-ethyl-8,8,12,16-tetramethyl-3-[1-methyl-2-(2-pyridyl)-vinyl]-4,17-dioxa-bicyclo[14.1.0]heptadecane-5,9-dione;
(4S,7R,8S,9S,13Z,16S(Z))-4,8-Dihydroxy-5,5,7,9,13-pentamethyl-16-[1-fluoro-2-(2-pyridyl)-vinyl]-oxacyclohexadec-13-ene-2,6-dione;
(1S,3S(Z),7S,10R,1S,12S,16R)-7,11-Dihydroxy-8,8,10,12,16-pentamethyl-3-[1-fluoro-2-(2-pyridyl)-vinyl]-4,17-dioxa-bicyclo[14.1.0]heptadecane-5,9-dione;
(4S,7R,8S,9S,13Z,16S(Z))-4,8-Dihydroxy-5,5,7,9,13-pentamethyl-16-[1-chloro-2-(2-pyridyl)-vinyl]-oxacyclohexadec-13-ene-2,6-dione;
(1S,3S(Z),75,10R,11S,12S,16R)-7,11-Dihydroxy-8,8,10,12,16-pentamethyl-3-[1-chloro-2-(2-pyridyl)-vinyl]-4,17-dioxa-bicyclo[14.1.0]heptadecane-5,9-dione;
(4S,7R,8S,9S,13Z,16S(Z))-4,8-Dihydroxy-7-ethyl-5,5,9,13-tetramethyl-16-[1-fluoro-2-(2-pyridyl)-vinyl]-oxacyclohexadec-13-ene-2,6-dione;
(1S,3S(Z),7S,10R,11S,12S,16R)-7,11-Dihydroxy-10-ethyl-8,8,12,16-tetramethyl-3-[1-fluoro-2-(2-pyridyl)-vinyl]-4,17-dioxa-bicyclo[14.1.0]heptadecane-5,9-dione;
(4S,7R,8S,9S,13Z,16S(Z))-4,8-Dihydroxy-7-ethyl-5,5,9,13-tetramethyl-16-[1-chloro-2-(2-pyridyl)-vinyl]-oxacyclohexadec-13-ene-2,6-dione;
(1S,3S(Z),7S,10R,11S,12S,16R)-7,11-Dihydroxy-10-ethyl-8,8,12,16-tetramethyl-3-[1-chloro-2-(2-pyridyl)-vinyl]-4,17-dioxa-bicyclo[14.1.0]heptadecane-5,9-dione;
(4S,7R,8S,9S,13Z,16S(E))-4,8-Dihydroxy-5,5,7,9,13-pentamethyl-16-[1-methyl-2-(2-methyl-oxazol-4-yl)-vinyl]-oxacyclohexadec-13-ene-2,6-dione;
(4S,7R,8S,9S,13Z,16S(E))-4,8-Dihydroxy-16-[2-(2-hydroxymethyl-oxazol-4-yl)-1-methyl-vinyl]-5,5,7,9,13-pentamethyl-oxacyclohexadec-13-ene-2,6-dione;
(4S,7R,8S,9S,13Z,16S(E))-16-[2-(2-Aminomethyl-oxazol-4-yl)-1-methyl-vinyl]-4,8-dihydroxy-5,5,7,9,13-pentamethyl-oxacyclohexadec-13-ene-2,6-dione;
(1S,3S(E),7S,10R,11S,12S,16R)-7,11-Dihydroxy-8,8,10,12,16-pentamethyl-3-[1-methyl-2-(2-methyl-oxazol-4-yl)-vinyl]-4,17-dioxa-bicyclo[14.1.0]heptadecane-5,9-dione;
(1S,3S(E),7S,10R,11S,12S,16R)-7,11-Dihydroxy-3-[2-(2-hydroxymethyl-oxazol-4-yl)-1-methyl-vinyl]-8,8,10,12,16-pentamethyl-4,17-dioxa-bicyclo[14.1.0]-heptadecane-5,9-dione;
(1S,3S(E),7S,10R,11S,12S,16R)-3-[2-(2-Aminomethyl-oxazol-4-yl)-1-methylvinyl]-7,11-dihydroxy-8,8,10,12,16-pentamethyl-4,17-dioxa-bicyclo[14.1.0]-heptadecane-5,9-dione;
(4S,7R,8S,9S,13Z,16S(E))-4,8-Dihydroxy-7-ethyl-5,5,9,13-tetramethyl-16-[1-methyl-2-(2-methyl-oxazol-4-yl)-vinyl]-oxacyclohexadec-13-ene-2,6-dione;
(4S,7R,8S,9S,13Z,16S(E))-4,8-Dihydroxy-16-[2-(2-hydroxymethyl-oxazol-4-yl)-1-methyl-vinyl]-7-ethyl-5,5,9,13-tetramethyl-oxacyclohexadec-13-ene-2,6-dione;
(4S,7R,8S,9S,13Z,16S(E))-16-[2-(2-Aminomethyl-oxazol-4-yl)-1-methyl-vinyl]-4,8-dihydroxy-7-ethyl-5,5,9,13-tetramethyl-oxacyclohexadec-13-ene-2,6-dione;
(1S,3S(E),7S,10R,11S,12S,16R)-7,11-Dihydroxy-10-ethyl-8,8,12,16-tetramethyl-3-[1-methyl-2-(2-methyl-oxazol-4-yl)-vinyl]-4,17-dioxa-bicyclo[14.1.0]-heptadecane-5,9-dione;
(1S,3S(E),7S,10R, 11S,12S,16R)-7,11-Dihydroxy-3-[2-(2-hydroxymethyl-oxazol-4-yl)-1-methyl-vinyl]-10-ethyl-8,8,12,16-tetramethyl-4,17-dioxa-bicyclo[14.1.0]heptadecane-5,9-dione;
(1S,3S(E),7S,10R,11S,12S,16R)-3-[2-(2-Aminomethyl-oxazol-4-yl)-1-methylvinyl]-7,11-dihydroxy-10-ethyl-8,8,12, 16-tetramethyl-4,17-dioxa-bicyclo[14.1.0]-heptadecane-5,9-dione;
(4S,7R,8S,9S,13Z,16S(Z))-4,8-Dihydroxy-5,5,7,9,13-pentamethyl-16-[1-fluoro-2-(2-methyl-oxazol-4-yl)-vinyl]-oxacyclohexadec-13-ene-2,6-dione;
(4S,7R,8S,9S,13Z,16S(Z))-4,8-Dihydroxy-16-[2-(2-hydroxymethyl-oxazol-4-yl)-1-fluoro-vinyl]-5,5,7,9,13-pentamethyl-oxacyclohexadeo-13-ene-2,6-dione;
(4S,7R,8S,9S,13Z,16S(Z))-16-[2-(2-Aminomethyl-oxazol-4-yl)-1-fluoro-vinyl]-4,8-dihydroxy-5,5,7,9,13-pentamethyl-oxacyclohexadeo-13-ene-2,6-dione;
(1S,3S(Z),7S,10R,11S,12S,16R)-7,11-Dihydroxy-8,8,10,12,16-pentamethyl-3-[1-fluoro-2-(2-methyl-oxazol-4-yl)-vinyl]-4,17-dioxa-bicyclo[14.1.0]heptadecane-5,9-dione;
(1S,3 S(Z),7S,10R,11S,12S,16R)-7,11-Dihydroxy-3-[2-(2-hydroxymethyl-oxazol-4-yl)-1-fluoro-vinyl]-8, 8,10,12,16-pentamethyl-4,17-dioxa-bicyclo[14.1.0]-heptadecane-5,9-dione;
(1S,3S(Z),7S,10R,11S,12S,16R)3-[2-(2-Aminomethyl-oxazol-4-yl)-1-fluorovinyl]-7,11-dihydroxy-8,8,10,12,16-pentamethyl-4,17-dioxa-bicyclo[14.1.0]-heptadecane-5,9-dione;
(4S,7R,8S,9S,13Z,16S(Z))-4,8-Dihydroxy-5,5,7,9,13-pentamethyl-16-[1-chloro-2-(2-methyl-oxazol-4-yl)-vinyl]-oxacyclohexadec-13-ene-2,6-dione;
(4S,7R,8S,9S,13Z,16S(Z))-4,8-Dihydroxy-16-[2-(2-hydroxymethyl-oxazol-4-yl)-1-chloro-vinyl]-5,5,7,9,13-pentamethyl-oxacyclohexadec-13-ene-2,6-dione;
(4S,7R,8S,9S,13Z,16S(Z))-16-[2-(2-Aminomethyl-oxazol-4-yl)-1-chloro-vinyl]-4,8-dihydroxy-5,5,7,9,13-pentamethyl-oxacyclohexadec-13-ene-2,6-dione;
(1 S,3 S(Z),7S,10R,11S,12S,16R)-7,11-Dihydroxy-8,8,10,12,16-pentamethyl-3-[1-chloro-2-(2-methyl-oxazol-4-yl)-vinyl]-4,17-dioxa bicyclo[14.1.0]heptadecane-5,9-dione;
(1S,3S(Z),7S,10R,11S,12S,16R)-7,11-Dihydroxy-3-[2-(2-hydroxymethyl-oxazol-4-yl)-1-chloro-vinyl]-8,8,10,12,16-pentamethyl-4,17-dioxa-bicyclo[14.1.0]-heptadecane-5,9-dione;
(1S,3S(Z),7S,10R,11S,12S,16R)-3-[2-(2-Aminomethyl-oxazol-4-yl)-1-chloro-vinyl]-7,11-dihydroxy-8,8,10,12,16-pentamethyl-4,17-dioxa-bicyclo [14.1.0]-heptadecane-5,9-dione ;
(4S,7R,8S,9S,13Z,16S(Z))-4,8-Dihydroxy-7-ethyl-5,5,9,13-tetramethyl-16-[1-fluoro-2-(2-methyl-oxazol-4-yl)-vinyl]-oxacyclohexadec-13-ene-2,6-dione ;
(4S,7R,8S,9S,13Z,16S(Z))-4,8-Dihydroxy-16-[2-(2-hydroxymethyl-oxazol-4-yl)-1-fluoro-vinyl]-7-ethyl-5,5,9,13-tetramethyl-oxacyclohexadec-13-ene-2,6-dione;
(4S,7R,8S,9S,13Z,16S(Z))-16-[2-(2-Aminomethyl-oxazol-4-yl)-1-fluoro-vinyl]-4,8-dihydroxy-7-ethyl-5,5,9,13-tetramethyl-oxacyclohexadec-13-ene-2,6-dione;
(1S,3S(Z),7S,10R,11S,12S,16R)-7,11-Dihydroxy-10-ethyl-8,8,12,16-tetramethyl-3-[1-fluoro-2-(2-methyl-oxazol-4-yl)-vinyl]-4,17-dioxa-bicyclo[14.1.0]-heptadecane-5,9-dione;
(1S,3S(Z),7S,10R,11S,12S,16R)-7,11-Dihydroxy-3-[2-(2-hydroxymethyl-oxazol-4-yl)-1-fluoro-vinyl]-10-ethyl-8,8,12,16-tetramethyl-4,17-dioxa-bicyclo[14.1.0]heptadecane-5,9-dione;
(1S,3S(Z),7S,10R,11S,12S,16R)-3-[2-(2-Aminomethyl-oxazol-4-yl)-1-fluorovinyl]-7,11-dihydroxy 10-ethyl-8,8,12,16-tetramethyl-4,17-dioxa-bicyclo[14.1.0]-heptadecane-5,9-dione;
(4S,7R,8S,9S,13Z,16S(Z))-4,8-Dihydroxy-7-ethyl-5,5,9,13-tetramethyl-16-[1-chloro-2-(2-methyl-oxazol-4-yl)-vinyl]-oxacyclohexadec-13-ene-2,6-dione;
(4S,7R,8S,9S,13Z,16S(Z))-4,8-Dihydroxy-16-[2-(2-hydroxymethyl-oxazol-4-yl)-1-chloro-vinyl]-7-ethyl-5,5,9,13-tetramethyl-oxacyclohexadec-13-ene-2,6-dione;
(4S,7R,8S,9S,13Z,16S(Z))-16-[2-(2-Aminomethyl-oxazol-4-yl)-1-chloro-vinyl]-4,8-dihydroxy-7-ethyl-5,5,9,13-tetramethyl-oxacyclohexadeo-13-ene-2,6-dione;
(1S,3S(Z),7S,10R,11S,12S,16R)-7,11-Dihydroxy-10-ethyl-8,8,12,16-tetramethyl-3-[1-chloro-2-(2-methyl-oxazol-4-yl)-vinyl]-4,17-dioxa-bicyclo[14.1.0]-heptadecane-5,9-dione;
(1S,3S(Z),7S,10R,11S,12S,16R)-7,11-Dihydroxy-3-[2-(2-hydroxymethyl-oxazol-4-yl)-1-chloro-vinyl]-10-ethyl-8,8,12,16-tetramethyl-4,17-dioxa-bicyclo[14.1.0]heptadecane-5,9-dione;
(1S,3S(Z),7S,10R,11S,12S,16R)3-[2-(2-Aminomethyl-oxazol-4-yl)-1-chloro-vinyl]-7,11-dihydroxy-10-ethyl-8,8,12,16-tetramethyl-4,17-dioxa-bicyclo[14.1.0]-heptadecane-5,9-dione;
(4S,7R,8S,9S,13Z,16S(E))-4,8-Dihydroxy-5,5,7,9,13-pentamethyl-16-[2-(2-methyl-thiazol-4-yl)-vinyl]-oxacyclohexadec-13-ene-2,6-dione;
(4S,7R,8S,9S,13Z,16S(E))-4,8-Dihydroxy-16-[2-(2-hydroxymethyl-thiazol-4-yl)-vinyl]-5,5,7,9,13-pentamethyl-oxacyclohexadec-13-ene-2,6-dione;
(4S,7R,8S,9S,13Z,16S(E))-16-[2-(2-Aminomethyl-thiazol-4-yl)-vinyl]-4,8-dihydroxy-5,5,7,9,13-pentamethyl-oxacyclohexadec-13-ene-2,6-dione;
(1S,3S(E),7S,10R,11S,12S,16R)-7,11-Dihydroxy-8,8,10,12,16-pentamethyl-3-[2-(2-methyl-thiazol-4-yl)-vinyl]-4,17-dioxa-bicyclo[ 14.1.0]heptadecane-5,9-dione;
(1S,3S(E),75,10R,11S,12S,16R)-7,11-Dihydroxy-3-[2-(2-hydroxymethyl-thiazol-4-yl)-vinyl]-8,8,10,12,16-pentamethyl-4,17-dioxa-bicyclo[14.1.0]heptadecane-5,9-dione;
(1S,3S(E),7S,10R,11S,12S,16R)-3-[2-(2-Aminomethyl-thiazol-4-yl)-vinyl]-7,11-dihydroxy-8,8,10,12,16-pentamethyl-4,17-dioxa-bicyclo[14.1.0]heptadecane-5,9-dione;
(4S,7R,8S,9S,13Z,16S(E))-4,8-Dihydroxy-7-ethyl-5,5,9,13-tetramethyl-16-[2-(2-methyl-thiazol-4-yl)-vinyl]-oxacyclohexadec-13-ene-2,6-dione;
(4S,7R,8S,9S,13Z,16S(E))-4,8-Dihydroxy-16-[2-(2-hydroxymethyl-thiazol-4-yl)-vinyl]-7-ethyl-5,5,9,13-tetramethyl-oxacyclohexadec-13-ene-2,6-dione;
(4S,7R,8S,9S,13Z,16S(E))-16-[2-(2-Aminomethyl-thiazol-4-yl)-vinyl]-4,8-dihydroxy-7-ethyl-5,5,9,13-tetramethyl-oxacyclohexadec-13-ene-2,6-dione;
(1S,3S(E),7S,10R,11S,12S,16R)-7,11-Dihydroxy-10-ethyl-8,8,12,16-tetramethyl-3-[2-(2-methyl-thiazol-4-yl)-vinyl]-4,17-dioxa-bicyclo[14.1.0]heptadecane-5,9-dione;
(1S,3S(E),7S,10R,11S,12S,16R)-7,11-Dihydroxy-3-[2-(2-hydroxymethyl-thiazol-4-yl)-vinyl]-10-ethyl-8,8,12,16-tetramethyl-4,17-dioxa-bicyclo[14.1.0]-heptadecane-5,9-dione;
(1S,3S(E),7S,10R,11S,12S,16R)-3-[2-(2-Aminomethyl-thiazol-4-yl)-vinyl]-7,11-dihydroxy-10-ethyl-8,8,12,16-tetramethyl-4,17-dioxa-bicyclo[14.1.0]heptadecane-5,9-dione;
(4S,7R,8S,9S,13Z,16S(E))-4,8-Dihydroxy 5,5,7,9,13-pentamethyl-16-[2-(2-pyridyl)-vinyl]-oxacyclohexadec-13-ene- 2,6-dione;
(1S,3S(E),7S,10R,11S,12S,16R)-7,11-Dihydroxy-8,8,10,12,16-pentamethyl-3-[2-(2-pyridyl)-vinyl]-4,17-dioxa-bicyclo[14.1.0]heptadecane-5,9-dione;
(4S,7R,8S,9S,13Z,16S(E))-4,8-Dihydroxy-7-ethyl-5,5,9,13-tetramethyl-16-[2-(2-pyridyl)-vinyl]-oxacyclohexadec-13-ene-2,6-dione;
(1S,3S(E),75,10R,11S,12S,16R)-7,11-Dihydroxy-10R-ethyl-8,8,12,16-tetramethyl-3-[2-(2-pyridyl)-vinyl]-4,17-dioxa-bicyclo[14.1.0]heptadecane-5,9-dione;
(4S,7R,8S,9S,13Z,16S)-4,8-Dihydroxy-5,5,7,9,13-pentamethyl-16-(2-methyl-benzothiazol-5-yl)-oxacyclohexadec-13-ene-2,6-dione;
(4S,7R,8S,9S,13Z,16S)-4,8-Dihydroxy-16-(2-hydroxymethyl-benzothiazol-5-y!)-5,5,7,9,13-pentamethyl-oxacyclohexadec-13-ene-2,6-dione;
(4S,7R,8S,9S,13Z,16S)-16-(2-Aminomethyl-benzothiazol-5-yi)-4,8-dihydroxy-5,5,7,9,13-pentamethyl-oxacyclohexadec-13-ene-2,6-dione;
(1S,3S,7S,10R,11S,12S,16R)-7,11-Dihydroxy-8,8,10,12,16-pentamethyl-3-(2-methyl-benzothiazol-5-yl)-4,17-dioxa-bicyclo[14.1.0]heptadecane-5,9-dione;
(1S,3S,7S,10R,11S,12S,16R)-7,11-Dihydroxy-3-(2-hydroxymethyl-benzothiazol-5-yl)-8,8,10,12,16-pentamethyl-4,17-dioxa-bicyclo[14.1.0]heptadecane-5,9-dione;
(1S,3S,7S,10R,11S,12S,16R)-3-(2-Aminomethyl-benzothiazol-5-yl)-7,11-dihydroxy-8,8,10,12,16-pentamethyl-4,17-dioxa-bicyclo[14.1.0]heptadecane-5,9-dione;
(4S,7R,8S,9S,13Z,16S)-4,8-Dihydroxy-7-ethyl-5,5,9,13-tetramethyl-16-(2-methyl-benzothiazol-5-yl)-oxacyclohexadec-13-ene-2,6-dione;
(4S,7R,8S,9S,13Z,16S)-4,8-Dihydroxy-16-(2-hydroxymethyl-benzothiazol-5-yl)-7-ethyl-5,5,9,13-tetramethyl-oxacyclohexadec-13-ene-2,6-dione;
(4S,7R,8S,9S,13Z,16S)-16-(2-Aminomethyl-benzothiazol-5-yl)-4,8-dihydroxy-7-ethyl-5,5,9,13-tetramethyl-oxacyclohexadec-13-ene-2,6-dione;
(1S,3S,7S,10R,11S,12S,16R)-7,11-Dihydroxy-10-ethyl-8,8,12,16-tetramethyl-3-(2-methyl-benzothiazol-5-yl)-4,17-dioxa-bicyclo[14.1.0]heptadecane-5,9-dione;
(1S,3S,7S,10R,11S,12S,16R)-7,11-Dihydroxy-3-(2-hydroxymethyl-benzothiazol-5-yl)-10-ethyl-8,8,12,16-teh-amethyl-4,17-dioxa-bicyclo[14.1.0]-heptadecane-5,9-dione;
(1S,3S,7S,10R,1 11S,12S,16R)-3-(2-Aminomethyl-benzothiazol-5-yl)-7,11-dihydroxy-10-ethyl-8,8,12,16-tetramethyl-4,17-dioxa-bicyclo[14.1.0]heptadecane-5,9-dione;
(4S,7R,8S,9S,13Z,16S)-4,8-Dihydroxy-7-propyl-5,5,9,13-tetramethyl-16-(2-methyl-benzothiazol-5-yl)-oxacyclohexadec-13-ene-2,6-dione;
(4S,7R,8S,9S,13Z,16S)-4,8-Dihydroxy-16-(2-hydroxymethyl-benzothiazol-5-yl)-7-propyl-5,5,9,13-tetramethyl-oxacyclohexadec-13-ene-2,6-dione;
(4S,7R,8S,9S,13Z,16S)-16-(2-Aminomethyl-benzothiazol-5-yl)-4,8-dihydroxy-7-propyl-5,5,9,13-tetramethyl-oxacyclohexadec-13-ene-2,6-dione;
(1S,3S,7S,10R,11S,12S,16R)-7,11-Dihydroxy-10-propyl-8,8,12,16-tetramethyl-3-(2-methyl-benzothiazol-5-yl)-4,17-dioxa-bicyclo[14.1.0]heptadecane-5,9-dione;
(1S,3S,7S,10R,11S,12S,16R)-7,11-Dihydroxy-3-(2-hydroxymethyl-benzothiazol-5-yl)-10-propyl-8,8,12,16-tetramethyl-4,17-dioxa-bicyclo[14.1.0]-heptadecane-5,9-dione;
(1S,3S,7S,10R,11S,12S,16R)-3-(2-Aminomethyl-benzothiazol-5-yl)-7,11-dihydroxy-10-propyl-8,8,12,16-tetramethyl-4,17-dioxa-bicyclo[14.1.0]heptadecana-5,9-dione;
(4S,7R,8S,9S,13Z,16S)-4,8-Dihydroxy-7-butyl-5,5,9,13-tetramethyl-16-(2-methyl-benzothiazol-5-yl)oxacyclohexadec-13-ene-2,6-dione;
(4S,7R,8S,9S,13Z,16S)-4,8-Dihydroxy-16-(2-hydroxymethyl-benzothiazol-5-yl)-7-butyl-5,5,9,13-tetramethyl-oxacyclohexadec-13-ene-2,6-dione;
(4S,7R,8S,9S,13Z,16S)-16-(2-Aminomethyl-benzothiazol-5-yl)-4,8-dihydroxy-7-butyl-5,5,9,13-tetramethyl-oxacyclohexadec-13-ene-2,6-dione;
(1S,3S,7S,10R,11S,12S,16R)-7,11-Dihydroxy-10-butyl-8,8,12,16-tetramethyl-3-(2-methyl-benzothiazol-5-yl)-4,17-dioxa-bicyclo[14.1.0]heptadecane-5,9-dione;
(1S,3S,7S,10R,11S,12S,16R)-7,11-Dihydroxy-3-(2-hydroxymethyl-benzothiazol-5-yl)-10-butyl-8,8,12,16-tetramethyl-4,17-dioxa-bicyclo[14.1.0]-heptadecane-5,9-dione;
(1S,3S,7S,10R,11S,12S,16R)-3-(2-Aminomethyl-benzothiazol-5-yl)-7,11-dihydroxy- 10-butyl-8,8,12,16-tetramethyl-4,17-dioxa-bicyclo[14.1.0]heptadecane-5,9-dione;
(4S,7R,8S,9S,13Z,16S)-4,8-Dihydroxy-7-allyl-5,5,9,13-tetramethyl-16-(2-methyl-benzothiazol-5-yl)-oxacyclohexadec-13-ene-2,6-dione;
(4S,7R,8S,9S,13Z,16S)-4,8-Dihydroxy-16-(2-hydroxymethyl-benzothiazol-5-yl)-7-allyl-5,5,9,13-tetramethyl-oxacyclohexadec-13-ene-2,6-dione;
(4S,7R,8S,9S,13Z,16S)-16-(2-Aminomethyl-benzothiazol-5-yl)-4,8-dihydroxy-7-allyl-5,5,9,13-tetramethyl-oxacyclohexadec-13-ene-2,6-dione;
(1S,3S,7S,10R,11S,12S,16R)-7,11-Dihydroxy-10-allyl-8,8,12,16-tetramethyl-3-(2-methyl-benzothiazol-5-yl)-4,17-dioxa-bicyclo[14.1.0]heptadecane-5,9-dione;
(1S,3S,7S,10R,11S,12S,16R)-7,11-Dihydroxy-3-(2-hydroxymethyl-benzothiazol-5-yl)-10-allyl-8,8,12,16-tetramethyl-4,17-dioxa-bicyclo[14.1.0]-heptadecane-5,9-dione;
(1S,3S,7S,10R,11S,12S,16R)-3-(2-Aminomethyl-benzothiazol-5-yl)-7,11-dihydroxy-10-allyl-8,8,12,16-tetramethyl-4,17-dioxa-bicyclo[14.1.0]heptadecane-5,9-dione;
(4S,7R,8S,9S,13Z,16S)-4,8-Dihydroxy-7-prop-2-inyl-5,5,9,13-tetramethyl-16-(2-methyl-benzothiazol-5-yl)-oxacyclohexadec-13-ene-2,6-dione;
(4S,7R,8S,9S,13Z,16S)-4,8-Dihydroxy-16-(2-hydroxymethyl-benzothiazol-5-yl)-7-prop-2-inyl-5,5,9,13-tetramethyl-oxacyclohexadec-13-ene-2,6-dione;
(4S,7R,8S,9S,13Z,16S)-16-(2-Aminomethyl-benzothiazol-5-yl)-4,8-dihydroxy-7-prop-2-inyl-5,5,9,13-tetramethyl-oxacyclohexadec-13-ene-2,6-dione;
(1S,3S,7S,10R,11S,125,16R)-7,11-Dihydroxy-10-prop-2-inyl-8,8,12,16-tetramethyl-3-(2-methyl-benzothiazol-5-yl)-4,17-dioxa-bicyclo [14.1.0]heptadecane-5,9-dione;
(1S,3S,7S,10R,11S, 12S,16R)-7,11-Dihydroxy-3-(2-hydroxymethyl-benzothiazol-5-yl)-10-prop-2-inyl-8,8,12,16-tetramethyl-4,17-dioxa-bicyclo[14.1.0]heptadecane-5,9-dione;
(1S,3S,7S,10R,11S, 1S,12S,16R)-3-(2-Aminomethyl-benzothiazol-5-yl)-7,11-dihydroxy-10-prop-2-inyl-8,8,12,16-tetramethyl-4,17-dioxa-bicyclo[14.1.0]-heptadecane-5,9-dione;
(4S,7R,8S,9S,13Z,16S)-4,8-Dihydroxy-7-but-3-enyl-5,5,9,13-tetramethyl-16-(2-methyl-benzothiazol-5-yl)-oxacyclohexadec-13-ene-2,6-dione;
(4S,7R,8S,9S,13Z,16S)-4,8-Dihydroxy-16-(2-hydroxymethyl-benzothiazol-5-yl)-7-but-3-enyl-5,5,9,13-tetramethyl-oxacyclohexadec-13-ene-2,6-dione;
(4S,7R,8S,9S,13Z,16S)-16-(2-Aminomethyl-benzothiazol-5-yl)-4,8-dihydroxy-7-but-3-enyl-5,5,9,13-tetramethyl-oxacyclohexadec-13-ene-2,6-dione;
(1S,3S,7S,10R,11S,12S,16R)-7,11-Dihydroxy-10-but-3-enyl-8,8,12,16-tetramethyl-3-(2-methyl-benzothiazol-5-yl)-4,17-dioxa-bicyclo[14.1.0]heptadecane-5,9-dione;
(1S,3S,7S,10R,11S,12S,16R)-7,11-Dihydroxy-3-(2-hydroxymethyl-benzothiazol-5-yl)-10-but-3-enyl-8,8,12,16-tetramethyl-4,17-dioxa-bicyclo[14.1.0]-heptadecane-5,9-dione;
(1S,3S,7S,10R,11S,12S,16R)-3-(2-Aminomethyl-benzothiazol-5-yl)-7,11-dihydroxy-10-but-3-enyl-8,8,12,16-tetramethyl-4,17-dioxa-bicyclo[14.1.0]heptadecane-5,9-dione;
(4S,7R,8S,9S,13Z,16S)-4,8-Dihydroxy-7-but-3-inyl-5,5,9,13-tetramethyl-16-(2-methyl-benzothiazol-5-yl)-oxacyclohexadec-13-ene-2,6-dione;
(4S,7R,8S,9S,13Z,16S)-4,8-Dihydroxy-16-(2-hydroxymethyl-benzothiazol-5-yl)-7-but-3-inyl-5,5,9,13-tetramethyl-oxacyclohexadec-13-ene-2,6-dione;
(4S,7R,8S,9S,13Z,16S)-16-(2-Aminomethyl-benzothiazol-5-yl)-4,8-dihydroxy-7-but-3-inyl-5,5,9,13-tetramethyl-oxacyclohexadec-13-ene-2,6-dione;
(1S,3S,7S,10R,11S,12S,16R)-7,11-Dihydroxy-10-but-3-inyl-8,8,12,16-tetramethyl-3-(2-methyl-benzothiazol-5-yl)-4,17-dioxa-bicyclo[14.1.0]heptadecane-5,9-dione;
(1S,3S,7S,10R,11S,12S,16R)-7,11-Dihydroxy-3-(2-hydroxymethyl-benzothiazol-5-yl)-10-but-3-inyl-8,8,12,16-tetramethyl-4,17-dioxa-bicyclo[14.1.0]-heptadecane-5,9-dione;
(1S,3S,7S,10R,11S,12S,16R)-3-(2-Aminomethyl-benzothiazol-5-yl)-7,11-dihydroxy-10-but-3-inyl-8,8,12,16-tetramethyl-4,17-dioxa-bicyclo[14.1.0]heptadecane-5,9-dione;
(4S,7R,8S,9S,13Z,16S)-4,8-Dihydroxy-5,5,7,9,13-pentamethyl-16-(2-methyl-benzoxazol-5-yl)-oxacyclohexadec-13-ene-2,6-dione;
(4S,7R,8S,9S,13Z,16S)-4,8-Dihydroxy-16-(2-hydroxymethyl-benzoxazol-5-yl)-5,5,7,9,13-pentamethyl-oxacyclohexadec-13-ene-2,6-dione;
(4S,7R,8S,9S,13Z,16S)-16-(2-Aminomethyl-benzoxazol-5-yl)-4,8-dihydroxy-5,5,7,9,13-pentamethyl-oxacyclohexadec-13-ene-2,6-dione;
(1S,3S,7S,10R,11S,12S,16R)-7,11-Dihydroxy-8,8,10,12,16-pentamethyl-3-(2-methyl-benzoxazol-5-yl)-4,17-dioxa-bicyclo[14.1.0]heptadecane-5,9-dione;
(1S,3S,7S,10R,11S,12S,16R)-7,11-Dihydroxy-3-(2-hydroxymethyl-benzoxazol-5-yl)-8,8,10,12,16-pentamethyl-4,17-dioxa-bicyclo[14.1.0]heptadecane-5,9-dione;
(1S,3S,7S,10R,11S,12S,16R)-3-(2-Aminomethyl-benzoxazol-5-yl)-7,11-dihydroxy-8,8,10,12,16-pentamethyl-4,17-dioxa-bicyclo[14.1.0]heptadecane-5,9-dione;
(4S,7R,8S,9S,13Z,16S)-4,8-Dihydroxy-7-ethyl-5,5,9,13-tetramethyl-16-(2-methyl-benzoxazol-5-yl)-oxacyclohexadec-13-ene-2,6-dione;
(4S,7R,8S,9S,13Z,16S)-4,8-Dihydroxy-16-(2-hydroxymethyl-benzoxazol-5-yl)-7-ethyl-5,5,9,13-tetramethyl-oxacyclohexadec-13-ene-2,6-dione;
(4S,7R,8S,9S,13Z,16S)-16-(2-Aminomethyl-benzoxazol-5-yl)-4,8-dihydroxy-7-ethyl-5,5,9,13-tetramethyl-oxacyclohexadec-13-ene-2,6-dione;
(1S,3S,7S,10R,11S,12S,16R)-7,11-Dihydroxy-10-ethyl-8,8,12,16-tetramethyl-3-(2-methyl-benzoxazol-5-yl)-4,17-dioxa-bicyclo[14.1.0]heptadecane-5,9-dione;
(1S,3S,7S,10R,11S,12S,16R)-7,11-Dihydroxy-3-(2-hydroxymethyl-benzoxazol-5-yl)-10-ethyl-8,8,12,16-tetramethyl-4,17-dioxa-bicyclo[14.1.0]heptadecane-5,9-dione;
(1S,3S,7S,10R,11S,12S,16R)-3-(2-Aminomethyl-benzoxazol-5-yl)-7,11-dihydroxy-10-ethyl-8,8,12,16-tetramethyl-4,17-dioxa-bicyclo[14.1.0]heptadecane-5,9-dione;
(4S,7R,8S,9S,13Z,16S)-4,8-Dihydroxy-7-propyl-5,5,9,13-tetramethyl-16-(2-methyl-benzoxazol-5-yl)-oxacyclohexadec-13-ene-2,6-dione;
(4S,7R,8S,9S,13Z,16S)-4,8-Dihydroxy-16-(2-hydroxymethyl-benzoxazol-5-yl)-7-propyl-5,5,9,13-tetramethyl-oxacyclohexadec-13-ene-2,6-dione;
(4S,7R,8S,9S,13Z,16S)-16-(2-Aminomethyl-benzoxazol-5-yl)-4,8-dihydroxy-7-propyl-5,5,9,13-tetramethyl-oxacyclohexadec-13-ene-2,6-dione;
(1S,3S,7S,10R,11S,12S,16R)7,11-Dihydroxy-10-propyl-8,8,12,16-tetramethyl-3-(2-methyl-benzoxazol-5-yl)-4,17-dioxa-bicyclo[14.1.0]heptadecane-5,9-dione;
(1S,3S,7S,10R,11S,12S,16R)-7,11-Dihydroxy-3-(2-hydroxymethyl-benzoxazol-5-yl)-10-propyl-8,8,12,16-tetramethyl-4,17-dioxa-bicyclo[14.1.0]heptadecane-5,9-dione;
(1S,3S,7S,10R,11S,12S,16R)-3-(2-Aminomethyl-benzoxazol-5-yl)-7,11-dihydroxy-10-propyl-8,8,12,16-tetramethyl-4,17-dioxa-bicyclo[14.1.0]heptadecane-5,9-dione;
(4S,7R,8S,9S,13Z,16S)-4,8-Dihydroxy-7-butyl-5,5,9,13-tetramethyl-16-(2-methyl-benzoxazol-5-yl)-oxacyclohexadec-13-ene-2,6-dione;
(4S,7R,8S,9S,13Z,16S)-4,8-Dihydroxy-16-(2-hydroxymethyl-benzoxazol-5-yl)-7-butyl-5,5,9,13-tetramethyl-oxacyclohexadec-13-ene-2,6-dione;
(4S,7R,8S,9S,13Z,16S)-16-(2-Aminomethyl-benzoxazol-5-yl)-4,8-dihydroxy-7-butyl-5,5,9,13-tetramethyl-oxacyclohexadec-13-ene-2,6-dione;
(1S,3S,7S,10R,11S,12S,16R)7,11-Dihydroxy-10-butyl-8,8,12,16-tetramethyl-3-(2-methyl-benzoxazol-5-yl)-4,17-dioxa-bicyclo[14.1.0]heptadecane-5,9-dione;
(1S,3S,7S,10R,11S,12S,16R)-7,11-Dihydroxy-3-(2-hydroxymethyl-benzoxazol-5-yl)-10-butyl-8,8,12,16-tetramethyl-4,17-dioxa-bicyclo[14.1.0]heptadecane-5,9-dione;
(1S,3S,7S,10R,11S,12S,16R)-3-(2-Aminomethyl-benzoxazol-5-yl)-7,11-dihydroxy-10-butyl-8,8,12,16-tetramethyl-4,17-dioxa-bicyclo[14.1.0]heptadecane-5,9-dione;
(4S,7R,8S,9S,13Z,16S)-4,8-Dihydroxy-7-allyl-5,5,9,13-tetramethyl-16-(2-methyl-benzoxazol-5-yl)-oxacyclohexadec-13-ene-2,6-dione;
(4S,7R,8S,9S,13Z,16S)-4,8-Dihydroxy-16-(2-hydroxymethyl-benzoxazol-5-yl)-7-allyl-5,5,9,13-tetramethyl-oxacyclohexadec-13-ene-2,6-dione;
(4S,7R,8S,9S,13Z,16S)-16-(2-Aminomethyl-benzoxazol-5-yl)-4,8-dihydroxy-7-allyl-5,5,9,13-tetrannethyl-oxacyclohexadec-13-ene-2,6-dione;
(1S,3S,7S,10R,11S,12S,16R)-7,11-Dihydroxy-10-allyl-8,8,12,16-tetramethyl-3-(2-methyl-benzoxazol-5-yl)-4,17-dioxa-bicyclo[14.1.0]heptadecane-5,9-dione;
(1S,3S,7S,10R,11S,12S,16R)-7,11-Dihydroxy-3-(2-hydroxymethyl-benzoxazol-5-yl)-10-allyl-8,8,12,16-tetramethyl-4,17-dioxa-bicyclo[14.1.0]heptadecane-5,9-dione;
(1S,3S,7S,10R,11S,12S,16R)-3-(2-Aminomethyl-benzoxazol-5-yl)-7,11-dihydroxy-10-allyl-8,8,12,16-tetramethyl-4,17-dioxa-bicyclo[14.1.0]heptadecane-5,9-dione;
(4S,7R,8S,9S,13Z,16S)-4,8-Dihydroxy-7-prop-2-inyl-5,5,9,13-tetramethyl-16-(2-methyl-benzoxazol-5-yl)-oxacyclohexadec-13-ene-2,6-dione;
(4S,7R,8S,9S,13Z,16S)-4,8-Dihydroxy-16-(2-hydroxymethyl-benzoxazol-5-yl)-7-prop-2-inyl-5,5,9,13-tetramethyl-oxacyclohexadeo-13-ene-2,6-dione;
(4S,7R,8S,9S,13Z,16S)-16-(2-Aminomethyl-benzoxazol-5-yl)-4,8-dihydroxy-7-prop-2-inyl-5,5,9,13-tetramethyl-oxacyclohexadec-13-ene-2,6-dione;
(1S,3S,7S,10R,11S,12S,16R)-7,11-Dihydroxy-10-prop-2-inyl-8,8,12,16-tetramethyl-3-(2-methyl-benzoxazol-5-yl)-4,17-dioxa-bicyclo[14.1.0]heptadecane-5,9-dione;
(1S,3S,7S,10R,11S,12S,16R)-7,11-Dihydroxy-3-(2-hydroxymethyl-benzoxazol-5-yl)-10-prop-2-inyl-8,8,12,16-tetramethyl-4,17-dioxa-bicyclo[14.1.0]heptadecane-5,9-dione;
(1S,3S,7S,10R,11S,12S,16R)-3-(2-Aminomethyl-benzoxazol-5-yl)-7,11-dihydroxy-10-prop-2-inyl-8,8,12,16-tetramethyl-4,17-dioxa-bicyclo[14.1.0]-heptadecane-5,9-dione;
(4S,7R,8S,9S,13Z,16S)-4,8-Dihydroxy-7-but-3-enyl-5,5,9,13-tetramethyl-16-(2-methyl-benzoxazol-5-yl)-oxacyclohexadec-13-ene-2,6-dione;
(4S,7R,8S,9S,13Z,16S)-4,8-Dihydroxy-16-(2-hydroxymethyl-benzoxazol-5-yl)-7-but-3-enyl-5,5,9,13-tetramethyl-oxacyclohexadec-13-ene-2,6-dione;
(4S, 7R,8S,9S, 13Z, 16S)-16-(2-Aminomethyl-benzoxazol-5-yl)-4,8-dihydroxy-7-but-3-enyl-5,5,9,13-tetramethyl-oxacyclohexadec-13-ene-2,6-dione;
(1S,3S,7S,10R,11S,12S,16R)-7,11-Dihydroxy-10-but-3-enyl-8,8,12,16-tetramethyl-3-(2-methyl-benzoxazol-5-yl)-4,17-dioxa-bicyclo[14.1.0]heptadecane-5,9-dione;
(1S,3S,7S,10R,11S,12S,16R)-7,11-Dihydroxy-3-(2-hydroxymethyl-benzoxazol-5-yl)-10-but-3-enyl-8,8,12,16-tetramethyl-4,17-dioxa-bicyclo[14.1.0]heptadecane-5,9-dione;
(1S,3S,7S,10R,1S,12S,16R)-3-(2-Aminomethyl-benzoxazol-5-yl)-7,11-dihydroxy-10-but-3-enyl-8,8,12,16-tetramethyl-4,17-dioxa-bicyclo[14.1.0]heptadecane-5,9-dione;
(4S,7R,8S,9S,13Z,16S)-4,8-Dihydroxy-7-but-3-inyl-5,5,9,13-tetramethyl-16-(2-methyl-benzoxazol-5-yl)-oxacyclohexadeo-l3-ene-2,6-dione;
(4S,7R,8S,9S,13Z,16S)-4,8-Dihydroxy-16-(2-hydroxymethyl-benzoxazol-5-yl)-7-but-3-inyl-5,5,9,13-tetramethyl-oxacyclohexadec-13-ene-2,6-dione;
(4S,7R,8S,9S,13Z,16S)-16-(2-Aminomethyl-benzoxazol-5-yl)-4,8-dihydroxy-7-but-3-inyl-5,5,9,13-tetramethyl-oxacyclohexadec-13-ene-2,6-dione;
(1S,3S,7S,10R,11S,12S,16R)-7,11-Dihydroxy-10-but-3-inyl-8,8,12,16-tetramethyl-3-(2-methyl-benzoxazol-5-yl)-4,17-dioxa-bicyclo[14.1.0]heptadecane-5,9-dione;
(1S,3S,7S,10R,11S,12S,16R)-7,11-Dihydroxy-3-(2-hydroxymethyl-benzoxazol-5-yl)-10-but-3-inyl-8,8,12,16-tetramethyl-4,17-dioxa-bicyclo[14.1.0]heptadecane-5,9-dione;
(1S,3S,7S, 10R, 11S,12 S,16R)-3-(2-Aminomethyl-benzoxazol-5-yl)-7,11-dihydroxy-10-but-3-inyl-8,8,12,16-tetramethyl-4,17-dioxa-bicyclo[14.1.0]heptadecane-5,9-dione;
whereby the hydrogen atoms in the above-mentioned effector elements are replaced by radicals L¹-L³ in the positions indicated in formula (I).

4. Effector conjugate according to one of claims 1-3, whereby the linker is selected from the group that consists of compounds of general formula (III), whereby Aa1 represents a bond.

5. Effector conjugate according to one of claims 1-4, whereby
FG¹ is C₁-C₁₀ alkyl-S₃ or

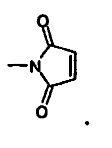

6. Effector-recognition unit conjugate of general formula (I), 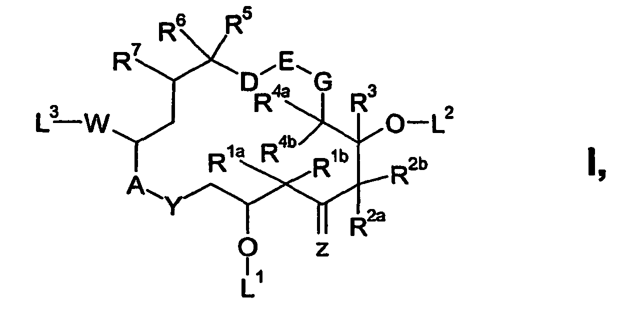 whereby the substituents here have the meanings that are mentioned in claim 1, but at least one group FG¹ is replaced by a group FG^{2a} or FG^{2b}, whereby FG^{2a} or FG^{2b} can have the following meanings:
FG^{2a} is -S-S-, 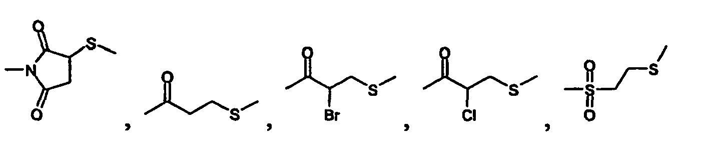 FG^{2b} is -CONH-
and whereby a recognition unit is conjugated via a sulfur atom with the group FG^{2a}, whereby the sulfur atom that is shown is a component of the recognition unit, or via an amide group with group FG^{2b}, whereby the nitrogen atom that is shown is a component of the recognition unit;
whereby the recognition unit is selected from the group that consists of peptides, soluble receptors, cytokines, lymphokines, aptamers, spiegelmers, recombinant proteins, new framework structures, monoclonal antibodies, and fragments of monoclonal antibodies;
as a uniform isomer or a mixture of different isomers and/or as a pharmaceutically acceptable salt thereof.

7. Effector-recognition unit conjugate according to claim 6, whereby the conjugate contains more than one recognition unit, and whereby the recognition units are identical.

8. Effector-recognition unit conjugate according to claim 6 or 7, whereby the recognition unit is an antibody or an antigen-binding fragment of the same, which is specific to an antigen that is selected from the group that consists of the antigens that are indicated in Table 1, as well as CD19, CD20, CD40, CD22, CD25, CD5, CD52, CD10, CD2, CD7, CD33, CD38, CD40, CD72, CD4, CD21, CD37, CD30, VCAM, CD31, ELAM, endoglin, VEGFRI/II, VEGFRvIII, scFv(14E1), αᵥβ₃, Tie1/2, TES23 (CD44ex6), phosphatidylserine, PSMA, VEGFR/VEGF complex and ED-B-fibronectin.

9. Linkers of general formula (III¹): 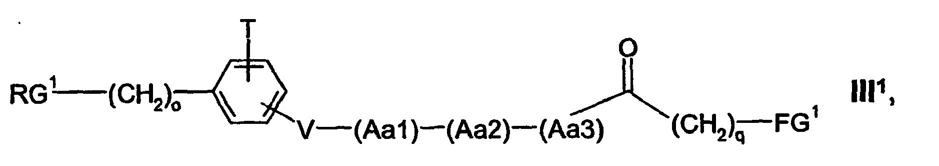 in which
RG¹ is an O=C=N group, and o, V, q, T, Aa1, Aa2, Aa3 and FG¹ have the meanings that are mentioned in Claim 1;
or linkers of general formula (III²): 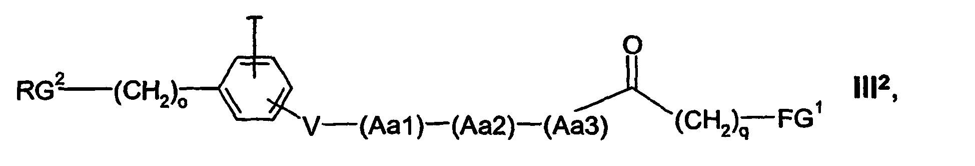 in which
RG² is a Hal-C(=O)-CHR²² group or a Hal-C(=O)-CHR²²-NR²³-C(=O) group, or an R²⁶-C(=O)-O-C(=O)-CHR²² group or an R²⁶-C(=O)-O-C(=O)-CHR²²-NR²³-C(=O) group, whereby R²⁶ is C₁-C₁₀ alkyl, aryl, or aralkyl, and o, V, q, T, Aa1, Aa2, Aa3 and FG¹ have the meanings that are mentioned in claim 1;
or linkers of general formula (III³): 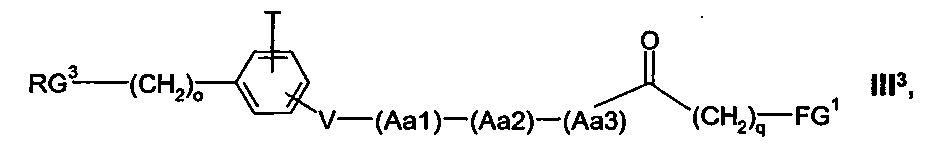 in which
RG³ is an OH group or an NHR^{24a} group or a COOH group, and o, V, q, T, Aa1, Aa2, Aa3 and FG¹ have the meanings that are mentioned in claim 1.

10. Process for the production of effector conjugates according to one of claims 1-5, whereby a compound of general formula (I), whereby the substituents have the meanings that are mentioned in claim 1, but
the condition that at least one group L¹, L² or L⁴ represent a linker of general formula (III) need not be met, and at least one substituent L¹, L² or L⁴ represents hydrogen or a group C(=O)Cl;
is reacted with a linker that is selected from the group that consists of a linker of general formula (III¹) or (III²) or (III³), as described in claim 9.

11. Process for the production of effector-recognition unit conjugates according to one of claims 6 to 8, whereby an effector conjugate is reacted according to one of claims 1-5 with at least one recognition unit, as defined in claims 6 and 8.

12. Use of a compound of general formula (I), whereby the substituents have the meanings that are mentioned in claim 1, but
the condition that at least one group L¹, L² or L⁴ represent a linker of general formula (III) need not be met, and at least one substituent L¹, L² or L⁴ represents hydrogen or a group C(=O)Cl;
in a process according to claim 10.

13. Use of a compound of general formula (I) for the production of an effector-recognition unit conjugate according to claims 6 to 8.

14. Use of a linker of general formula (III¹), (III²) or (III³) in a process according to claim 10.

15. Use of a linker of general formula (III¹), (III²) or (III³) for the production of an effector-recognition unit conjugate according to one of claims 6 to 8.

16. Use of a recognition unit, as defined in claim 6 or 8, in a process according to claim 11.

17. Effector-recognition unit conjugate according to one of claims 6 to 8 for use as a medication.

18. Effector-recognition unit conjugate according to one of claims 6 to 8 for use as a medication for treating diseases that are associated with proliferative processes.

19. Effector-recognition unit conjugate according to one of claims 6 to 8 for use as a medication for treating a disease, which is selected from the group that consists of tumors, inflammatory diseases, neurodegenerative diseases, angiogenesis-associated diseases, multiple sclerosis, Alzheimer's disease and rheumatoid arthritis.

## Patentansprüche

1. Effektor-Konjugat der allgemeinen Formel (I): 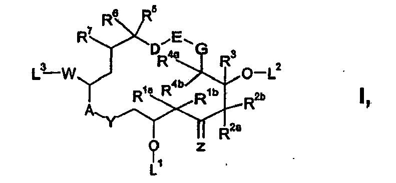 worin
R^{1a}, R^{1b} unabhängig voneinander Wasserstoff, C₁-C₁₀ Alkyl, Aryl, Aralkyl, oder gemeinsam eine -(CH₂)ₘ-Gruppe sind, worin m 2 bis 5 ist,
R^{2a}, R^{2b} unabhängig voneinander Wasserstoff, C₁-C₁₀ Alkyl, Aryl, Aralkyl, oder gemeinsam eine -(CH₂)ₙ-Gruppe sind, worin n 2 bis 5 ist, oder C₂-C₁₀ Alkenyl, oder C₂-C₁₀ Alkinyl, R³ Wasserstoff, C₁-C₁₀ Alkyl, Aryl oder Aralkyl, und
R^{4a}, R^{4b} unabhängig voneinander Wasserstoff, C₁-C₁₀ Alkyl, Aryl, Aralkyl, oder gemeinsam eine -(CH₂)ₚ-Gruppe sind, worin p 2 bis 5 ist,
R⁵ Wasserstoff, C₁-C₁₀ Alkyl, Aryl, Aralkyl, CO₂H, CO₂Alkyl, CH₂OH, CH₂OAlkyl, CH₂OAcyl, CN, CH₂NH₂, CH₂N(Alkyl, Acyl)_{1,2}, oder CH₂Hal,
Hal ein Halogen-Atom,
R⁶, R⁷ jeweils Wasserstoff, oder gemeinsam eine zusätzliche Bindung, oder gemeinsam ein Sauerstoff-Atom, oder gemeinsam eine NH-Gruppe, oder gemeinsam eine N-Alkyl-Gruppe, oder gemeinsam eine CH₂-Gruppe, und
G ein Sauerstoffatom oder CH₂ sind,
D-E eine Gruppe H₂C-CH₂, HC=CH, C=C, CH(OH)-CH(OH), CH(OH)-CH₂, CH₂-CH(OH), 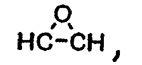 O-CH₂, oder, falls G eine CH₂-Gruppe darstellt, D-E auch CH₂-O ist,
W eine Gruppe C(=X)R⁸, oder ein bi- oder tricyclischer aromatischer oder heteroaromatischer Rest ist,
L³ Wasserstoff ist, oder, falls ein Rest in W eine Hydroxyl-Gruppe enthält, mit dieser eine Gruppe O-L⁴ bildet, oder, falls ein Rest in W eine Amino-Gruppe enthält, mit dieser eine Gruppe NR²⁵-L⁴ bildet,
R²⁵ Wasserstoff oder C₁-C₁₀ Alkyl ist,
X ein Sauerstoffatom, oder zwei OR²⁰-Gruppen, oder eine C₂-C₁₀ Alkylendioxy-Gruppe, die geradkettig oder verzweigt sein darf, oder H/OR⁹, oder eine CR¹⁰R¹¹-Gruppe, R⁸ Wasserstoff, C₁-C₁₀ Alkyl, Aryl, Aralkyl, Halogen oder CN, und
R⁹ Wasserstoff oder eine Schutzgruppe PG^{x} sind,
R¹⁰, R¹¹ jeweils unabhängig voneinander Wasserstoff, C₁-C₂₀ Alkyl, Aryl, Aralkyl sind, oder gemeinsam mit einem Methylenkohlenstoffatom einen 5- bis 7-gliedrigen carbocyclischen Ring bilden,
Z Sauerstoff oder H/OR¹²,
R¹² Wasserstoff oder eine Schutzgruppe PG^{z},
A-Y eine Gruppe O-C(=O), O-CH₂, CH₂-C(=O), NR²¹-C(=O) oder NR²¹-SO₂,
R²⁰ C₁-C₂₀ Alkyl,
R²¹ ein Wasserstoffatom oder C₁-C₁₀ Alkyl,
PG^{x}, PG^{y}, PG^{z} eine Schutzgruppe PG, und
L¹, L², L⁴ unabhängig voneinander Wasserstoff, eine Gruppe C(=O)Cl, eine Gruppe C(=S)Cl, eine Gruppe PG^{y} oder einen Linker der allgemeinen Formel (III)
darstellen können;
mit der Bedingung, dass mindestens ein Substituent L¹, L² oder L⁴ einen Linker der allgemeinen Formel (III) darstellt;
der Linker der allgemeinen Formel (III) folgende Struktur hat, 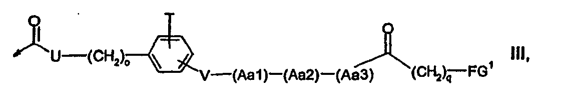 worin
T Halogen, OH, O-Alkyl, CO₂H, CO₂-Alkyl, -NHR²³, -NO₂, -N₃, -CN, C₁-C₂₀-Alkyl, C₁-C₂₀-Acyl oder C₁-C₂₀-Acyloxy-Gruppen,
U eine Bindung, Sauerstoff, oder NR^{24a},
o 0 bis 5,
V Sauerstoff oder NR^{24b},
Aa1 eine Bindung oder eine Gruppe der allgemeinen Formel IV und
Aa2, Aa3 unabhängig voneinander eine Gruppe der allgemeinen Formel IV darstellt 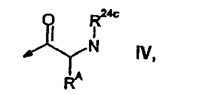 die sich aus einer natürlichen oder nicht natürlichen Aminosäure HO-Aa1-H, HO-Aa2-H, oder HO-Aa3-H der allgemeinen Formel IV' 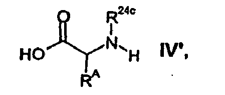 ableitet
worin R^{A} in HO-Aa1-H, HO-Aa2-H, oder HO-Aa3-H gleich oder verschieden sein kann und den Aa-Substituenten eine natürliche oder nicht natürliche Aminosäure repräsentiert,
R²² Wasserstoff, C₁-C₁₀ Alkyl, Aryl oder Aralkyl,
R²³ Wasserstoff, C₁-C₁₀ Alkyl, oder C₁-C₁₀ Acyl,
R^{24a} R^{24b} , R^{24c} unabhängig voneinander Wasserstoff oder C₁-C₁₀ Alkyl,
q 1 bis 20,
FG¹ C₁-C₁₀ Alkyl-S₃, 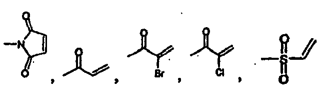 oder CO₂H
darstellen können;
als ein einheitliches Isomer oder eine Mischung unterschiedlicher Isomere und/oder als ein pharmazeutisch akzeptables Salz hiervon.

2. Effektor-Konjugat gemäss Anspruch 1, wobei:
A-Y O-C(=O) oder NR²¹-C(=O),
D-E eine H₂C-CH₂-Gruppe oder eine HC=CH-Gruppe,
G eine CH₂-Gruppe,
Z ein Sauerstoffatom,
R^{1a}, R^{1b} jeweils C₁-C₁₀ Alkyl oder zusammen eine -(CH₂)ₚ-Gruppe mit p gleich 2 oder 3 oder 4,
R^{2a}, R^{2b} unabhängig voneinander Wasserstoff, C₁-C₁₀ Alkyl, C₂-C₁₀ Alkenyl, oder C₂-C₁₀ Alkinyl,
R³ Wasserstoff;
R^{4a}, R^{4b} unabhängig voneinander Wasserstoff oder C₁-C₁₀ Alkyl;
R⁵ Wasserstoff, oder C₁-C₄ Alkyl oder CH₂OH oder CH₂NH₂ oder CH₂N(Alkyl, Acyl)_{1,2} oder CH₂Hal,
R⁶ und R⁷ gemeinsam eine zusätzliche Bindung oder gemeinsam eine NH-Gruppe oder gemeinsam eine N-Alkyl-Gruppe oder gemeinsam eine CH₂-Gruppe, oder gemeinsam eine Sauerstoff Gruppe,
W eine Gruppe C(=X)R⁸ oder ein 2-Methylbenzothiazol-5-yl-Radikal oder ein 2-Methylbenzoxazol-5-yl-Radikal oder ein Chinolin-7-yl-Radikal oder ein 2-Aminomethylbenzothiazol-5-yl-Radikal oder ein 2-Hydroxymethylbenzothiazol-5-yl-Radikal oder ein 2-Aminomethylbenzoxazol-5-yl-Radikal oder ein 2-Hydroxymethylbenzoxazol-5-yl-Radikal,
X eine CR¹⁰R¹¹-Gruppe,
R⁸ Wasserstoff oder C₁-C₄ Alkyl oder ein Fluoratom oder ein Chloratom oder ein Bromatom, R¹⁰/R¹¹ Wasserstoff/2-Methylthiazol-4-yl oder Wasserstoff/2-Pyridyl oder Wasserstoff/2-Methyloxazol-4-yl oder Wasserstoff/2-Aminomethylthiazol-4-yl oder Wasserstoff/2-Aminomethyloxazol-4-yl oder Wasserstoff/2-Hydroxymethylthiazol-4-yl oder Wasserstoff/2-Hydroxymethyloxazol-4-yl darstellen.

3. Effektor-Konjugat gemäss Anspruch 1 oder 2, wobei der Effektor-Grundkörper ausgewählt ist aus der Gruppe bestehend aus:
(4S,7R,8S,9S,13Z,16S(E))-4,8-Dihydroxy-5,5,7,9,13-pentamethyl-16-[1-methyl-2-(2-methyl-thiazol-4-yl)-vinyl]-oxacyclohexadec-13-en-2,6-dion;
(4S,7R,8S,9S,13Z,16S(E))-4,8-Dihydroxy-16-[2-(2-hydroxymethyl-thiazol-4-yl)-1-methyl-vinyl]-5,5,7,9,13-pentamethyl-oxacyclohexadec-13-en-2,6-dion;
(4S,7R,8S,9S,13Z,16S(E))-16-[2-(2-Aminomethyl-thiazol-4-yl)-1-methyl-vinyl]-4,8-dihydroxy-5,5,7,9,13-pentamethyl-oxacyclohexadec-13-en-2,6-dion;
(1S,3S(E),7S,10R,11S,12S,16R)-7,11-Dihydroxy-8,8,10,12,16-pentamethyl-3-[1-methyl-2-(2-methyl-thiazol-4-yl)-vinyl]-4,17-dioxa-bicyclo[14.1.0]heptadecan-5,9-dion;
(1S,3S(E),7S,10R,11S,12S,16R)-7,11-Dihydroxy-3-[2-(2-hydroxymethyl-thiazol-4-yl)-1-methyl-vinyl]-8,8,10,12,16-pentamethyl-4,17-dioxa-bicyclo[14.1.0]heptadecan-5,9-dion;
(1S,3S(E),7S,10R,11S,12S,16R)-3-[2-(2-Aminomethyl-thiazol-4-yl)-1-methyl-vinyl]-7,11-dihydroxy-8,8,10,12,16-pentamethyl-4,17-dioxa-bicyclo[14.1.0]heptadecan-5,9-dion;
(4S,7R,8S,9S,13Z,16S(E))-4,8-Dihydroxy-7-ethyl-5,5,9,13-tetramethyl-16-[1-methyl-2-(2-methyl-thiazol-4-yl)-vinyl]-oxacyclohexadec-13-en-2,6-dion;
(4S,7R,8S,9S,13Z,16S(E))-4,8-Dihydroxy-16-[2-(2-hydroxymethyl-thiazol-4-yl)-1-methyl-vinyl]-7-ethyl-5,5,9,13-tetramethyl-oxacyclohexadec-13-en-2,6-dion;
(45,7R,85,95,13Z,165(E))-16-[2-(2-Aminomethyl-thiazol-4-yl)-1-methyl-vinyl]-4,8-dihydroxy-7-ethyl-5,5,9,13-tetramethyl-oxacyclohexadec-13-en-2,6-dion;
(1S,3S(E),7S,10R,11S,12S,16R)-7,11-Dihydroxy-10-ethyl-8,8,12,16-tetramethyl-3-[1-methyl-2-(2-methyl-thiazol-4-yl)-vinyl]-4,17-dioxa-bicyclo[14.1.0]heptadecan-5,9-dion;
(1 S,3S(E),7S,10R,11S,12S,16R)-7,11-Dihydroxy-3-[2-(2-hydroxymethyl-thiazol-4-yl)-1-methyl-vinyl]-10-ethyl-8,8,12,16-tetramethyl-4,17-dioxa-bicyclo[14.1.0]heptadecan-5,9-dion;
(1S,3S(E),7S,10R,11S,12S,16R)-3-[2-(2-Aminomethyl-thiazol-4-yl)-1-methyl-vinyl]-7,11-dihydroxy-10-ethyl-8,8,12,16-tetramethyl-4,17-dioxa-bicyclo[14.1.0]heptadecan-5,9-dion;
(4S,7R,8S,9S,13Z,16S(Z))-4,8-Dihydroxy-5,5,7,9,13-pentamethyl-16-[1-fluor-2-(2-methyl-thiazol-4-yl)-vinyl]-oxacyclohexadec-13-en-2,6-dion;
(4S,7R,8S,9S,13Z,16S(Z))-4,8-Dihydroxy-16-[2-(2-hydroxymethyl-thiazol-4-yl)-1-fluor-vinyl]-5,5,7,9,13-pentamethyl-oxacyclohexadec-13-en-2,6-dion;
(4S,7R,8S,9S,13Z, 16S(Z))-16-[2-(2-Aminomethyl-thiazol-4-yl)-1-fluor-vinyl]-4,8-dihydroxy-5,5,7,9,13-pentamethyl-oxacyclohexadec-13-en-2,6-dion;
(1S,3S(Z),7S, 10R,11S,12S,16R)-7,11-Dihydroxy-8,8,10,12,16-pentamethyl-3-[1-fluor-2-(2-methyl-thiazol-4-yl)-vinyl]-4,17-dioxa-bicyclo[14.1.0]heptadecan-5,9-dion;
(1S,3S(Z),7S,10R,11S,12S,16R)-7,11-Dihydroxy-3-[2-(2-hydroxymethyl-thiazol-4-yl)-1-fluor-vinyl]-8,8,10,12,16-pentamethyl-4,17-dioxa-bicyclo[14.1.0]heptadecan-5,9-dion;
(1S,3S(Z),7S,10R,11S,12S,16R)-3-[2-(2-Aminomethyl-thiazol-4-yl)-1-fluor-vinyl]-7,11-dihydroxy-8,8,10,12,16-pentamethyl-4,17-dioxa-bicyclo[14.1.0]heptadecan-5,9-dion;
(4S,7R,8S,9S,13Z,16S(Z))-4,8-Dihydroxy-5,5,7,9,13-pentamethyl-16-[1-chlor-2-(2-methyl-thiazol-4-yl)-vinyl]-oxacyclohexadec-13-en-2,6-dion;
(4S,7R,8S,9S, 13Z,16S(Z))-4,8-Dihydroxy-16-[2-(2-hydroxymethyl-thiazol-4-y1)-1-chlor-vinyl]-5,5,7,9,13-pentamethyl-oxacyclohexadec-13-en-2,6-dion;
(4S,7R,8S,9S,13Z,16S(Z))-16-[2-(2-Aminomethyl-thiazol-4-yl)-1-chlor-vinyl]-4,8-dihydroxy-5,5,7,9,13-pentamethyl-oxacyclohexadec-13-en-2,6-dion;
(1S,3S(Z),7S,10R,11S,12S,16R)-7,11-Dihydroxy-8,8,10,12,16-pentamethyl-3-[1-chlor-2-(2-methyl-thiazol-4-yl)-vinyl]-4,17-dioxa-bicyclo[14.1.0]heptadecan-5,9-dion;
(1 S,3S(Z),7S,10R,11S,12S,16R)-7,11-Dihydroxy-3-[2-(2-hydroxymethyl-thiazol-4-yl)-1-chlor-vinyl]-8,8,10,12,16-pentamethyl-4,17-dioxa-bicyclo[14.1.0]heptadecan-5,9-dion;
(1S,3S(Z),7S,10R,11S,12S,16R)-3-(2-(2-Aminomethyl-thiazol-4-yl)-1-chlor-vinyl]-7,11-dihydroxy-8,8,10,12,16-pentamethyl-4,17-dioxa-bicyclo[14.1.0]heptadecan-5,9-dion;
(4S,7R,8S,9S,13Z,16S(Z))-4,8-Dihydroxy-7-ethyl-5,5,9,13-tetramethyl-16-[1-fluor-2-(2-methyl-thiazol-4-yl)-vinyl]-oxacyclohexadec-13-en-2,6-dion;
(4S,7R,8S,9S, 13Z, 16S(Z))-4,8-Dihydroxy-16-[2-(2-hydroxymethyl-thiazol-4-yl)-1-fluor-vinyl]-7-ethyl-5,5,9,13-tetramethyl-oxacyclohexadec-13-en-2,6-dion;
(4S,7R,8S,9S,13Z,16S(Z))-16-[2-(2-Aminomethyl-thiazol-4-yl)-1-fluor-vinyl]-4,8-dihydroxy-7-ethyl-5,5,9,13-tetramethyl-oxacyclohexadec-13-en-2,6-dion;
(S,3S(Z),7S,10R,11S,12S,16R)-7,11-Dihydroxy-10-ethyl-8,8,12,16-tetramethyl-3-[1-fluor-2-(2-methyl-thiazol-4-yl)-vinyl]-4,17-dioxa-bicyclo[14.1.0]heptadecan-5,9-dion;
(1S,3S(Z),7S,10R,11S,12S,16R)-7,11-Dihydroxy-3-[2-(2-hydroxymethyl-thiazol-4-yl)-1-fluor-vinyl]-10-ethyl-8,8,12,16-tetramethyl-4,17-dioxa-bicyclo[14.1.0]heptadecan-5,9-dion;
(1 S,3S(Z),7S,10R,11S,12S,16R)-3-[2-(2-Aminomethyl-thiazol-4-yl)-1-fluor-vinyl]-7,11-dihydroxy-10-ethyl-8,8,12,16-tetramethyl-4,17-dioxa-bicyclo[14.1.0]heptadecan-5,9-dion;
(4S,7R,8S,9S,13Z,16S(Z))-4,8-Dihydroxy-7-ethyl-5,5,9,13-tetramethyl-16-[1-chlor-2-(2-methyl-thiazol-4-yl)-vinyl]-oxacyclohexadec-13-en-2,6-dion;
(4S,7R,8S,9S,13Z,16S(Z))-4,8-Dihydroxy-16-[2-(2-hydroxymethyl-thiazol-4-yl)-1-chlor-vinyl]-7-ethyl-5,5,9,13-tetramethyl-oxacyclohexadec-13-en-2,6-dion;
(4S,7R,8S,9S,13Z,16S(Z))-16-[2-(2-Aminomethyl-thiazol-4-yl)-1-chlor-vinyl]-4,8-dihydroxy-7-ethyl-5,5,9,13-tetramethyl-oxacyclohexadec-13-en-2,6-dion;
(1 S,3S(Z),7S,10R,11S,12S,16R)-7,11-Dihydroxy-10-ethyl-8,8,12,16-tetramethyl-3-[1-chlor-2-(2-methyl-thiazol-4-yl)-vinyl]-4,17-dioxa-bicyclo[14.1.0]heptadecan-5,9-dion-,
(1S,3S(Z),7S,10R,11S,12S,16R)-7,11-Dihydroxy-3-[2-(2-hydroxymethyl-thiazol-4-yl)-1-chlor-vinyl]-10-ethyl-8,8,12,16-tetramethyl-4,17-dioxa-bicyclo[14.1.0]heptadecan-5,9-dion;
(1S,3S(Z),7S,10R,11S,12S, 16R)-3-[2-(2-Aminomethyl-thiazol-4-yl)-1-chlor-vinyl]-7,11-dihydroxy-10-ethyl-8,8,12,16-tetramethyl-4,17-dioxa-bicyclo[14.1.0]heptadecan-5,9-dion;
(4S,7R,8S,9S,13Z,16S(E))-4,8-Dihydroxy-5,5,7,9,13-pentamethyl-16-[1-methyl-2-(2-pyridyl)-vinyl]-oxacyclohexadec-13-en-2,6-dion;
(1S,3S(E),7S,10R,11S,12S,16R)-7,11-Dihydroxy-8,8,10,12,16-pentamethyl-3-[1-methyl-2-(2-pyridyl)-vinyl]-4,17-dioxa-bicyclo[14.1.0]heptadecan-5,9-dion;
(4S,7R,8S,9S,13Z,16S(E))-4,8-Dihydroxy-7-ethyl-5,5,9,13-tetramethyl-16-[1-methyl-2-(2-pyridyl)-vinyl]-oxacyctohexadec-13-en-2,6-dion;
(1S,3S(E),7S,10R,11S,12S,16R)-7,11-Dihydroxy-10-ethyl-8,8,12,16-tetramethyl-3-[1-methyl-2-(2-pyridyl)-vinyl]-4,17-dioxa-bicyclo[14.1.0]heptadecan-5,9-dion;
(4S,7R,8S,9S,13Z,16S(Z))-4,8-Dihydroxy-5,5,7,9,13-pentamethyl-16-[1-fluor-2-(2-pyridyl)-vinyl]-oxacyclohexadec-13-en-2,6-dion;
(1S,3S(Z),7S,10R,11S,12S,16R)-7,11-Dihydroxy-8,8,10,12,16-pentamethyl-3-[1-fluor-2-(2-pyridyl)-vinyl]-4,17-dioxa-bicyclo[14.1.0]heptadecan-5,9-dion;
(4S,7R,8S,9S,13Z,16S(Z))-4,8-Dihydroxy-5,5,7,9,13-pentamethyl-16-[1-chlor-2-(2-pyridyl)-vinyl]-oxacyclohexadec-13-en-2,6-dion;
(1S,3S(Z),7S,10R,11S,12S,16R)-7,11-Dihydroxy-8,8,10,12,16-pentamethyl-3-[1-chlor-2-(2-pyridyl)-vinyl]-4,17-dioxa-bicyclo[14.1.0]heptadecan-5,9-dion;
(4S,7R,8S,9S,13Z,16S(Z))-4,8-Dihydroxy-7-ethyl-5,5,9,13-tetramethyl-16-[1-fluor-2-(2-pyridyl)-vinyl]-oxacyclohexadec-13-en-2,6-dion;
(1S,3S(Z),7S,10R,11S,125,16R)-7,11-Dihydroxy-10-ethyl-8,8,12,16-tetramethyl-3-[1-fluor-2-(2-pyridyl)-vinyl]-4,17-dioxa-bicyclo[14.1.0]heptadecan-5,9-dion;
(4S,7R,8S,95,13Z,16S(Z))-4,8-Dihydroxy-7-ethyl-5,5,9,13-tetramethyl-16-[1-chlor-2-(2-pyridyl)-vinyl]-oxacyclohexadec-1 3-en-2,6-dion;
(1S,3S(Z),7S,10R,11S,12S,16R)-7,11-Dihydroxy-10-ethyl-8,8,12,16-tetramethyl-3-[1-chlor-2-(2-pyridyl)-vinyl]-4,17-dioxa-bicyclo[14.1.0]heptadecan-5,9-dion;
(4S,7R,8S,9S,13Z,16S(E))-4,8-Dihydroxy-5,5,7,9,13-pentamethyl-16-[1-methyl-2-(2-methyl-oxazol-4-yl)-vinyl]-oxacyclohexadec-13-en-2,6-dion;
(4S,7R,8S,9S,13Z,16S(E))-4,8-Dihydroxy-16-[2-(2-hydroxymethyl-oxazol-4-yl)-1-methyl-vinyl]-5,5,7,9,13-pentamethyl-oxacyclohexadec-13-en-2,6-dion;
(4S,7R,8S,9S,13Z,16S(E))-16-[2-(2-Aminomethyl-oxazol-4-yl)-1-methyl-vinyl]-4,8-dihydroxy-5,5,7,9,13-pentamethyl-oxacyclohexadec-13-en-2,6-dion;
(1S,3S(E),7S,10R,11S,12S,16R)-7,11-Dihydroxy-8,8,10,12, 16-pentamethyl-3-[1-methyl-2-(2-methyl-oxazol-4-yl)-vinyl]-4,17-dioxa-bicyclo[14.1.0]heptadecan-5,9-dion;
(1S,3S(E),7S,10R,11S,12S,16R)-7,11-Dihydroxy-3-[2-(2-hydroxymethyl-oxazol-4-yl)-1-methyl-vinyl]-8,8,10,12,16-pentamethyl-4,17-dioxa-bicyclo[14.1.0]heptadecan-5,9-dion;
(1S,3S(E),7S,1 0R,11S,125,16R)-3-[2-(2-Aminomethyl-oxazol-4-yl)-1-methyl-vinyl]-7,11-dihydroxy-8,8,10,12,16-pentamethyl-4,17-dioxa-bicyclo[14.1.0]heptadecan-5,9-dion;
(4S,7R,8S,9S,13Z,16S(E))-4,8-Dihydroxy-7-ethyl-5,5,9,13-tetramethyl-16-[1-methyl-2-(2-methyl-oxazol-4-yl)-vinyl]-oxacyclohexadec-13-en-2,6-dion;
(4S,7R,8S,9S,13Z,16S(E))-4,8-Dihydroxy-16-[2-(2-hydroxymethyl-oxazol-4-yl)-1-methyl-vinyl]-7-ethyl-5,5,9,13-tetramethyl-oxacyclohexadec-13-en-2,6-dion;
(4S,7R,8S,9S,13Z,16S(E))-16-[2-(2-Aminomethyl-oxazol-4-yl)-1-methyl-vinyl]-4,8-dihydroxy-7-ethyl-5,5,9,13-tetramethyl-oxacyclohexadec-13-en-2,6-dion;
(1S,3S(E),7S,10R,11S, 12S,16R)-7,11-Dihydroxy-10-ethyl-8,8,12,16-tetramethyl-3-[1-methyl-2-(2-methyl-oxazol-4-yl)-vinyl]-4,17-dioxa-bicyclo[14.1.0]heptadecan-5,9-dion;
(1S,3S(E),7S,10R,11S,12S,16R)-7,11-Dihydroxy-3-[2-(2-hydroxymethyl-oxazol-4-yl)-1-methyl-vinyl]-10-ethyl-8,8,12,16-tetramethyl-4,17-dioxa-bicyclo[14.1.0]heptadecan-5,9-dion;
(1S,3S(E),7S,10R,11S,12S,16R)-3-[2-(2-Aminomethyl-oxazol-4-yl)-1-methyl-vinyl]-7,11-dihydroxy-10-ethyl-8,8,12,16-tetramethyl-4,17-dioxa-bicyclo[14.1.0]heptadecan-5,9-dion;
(4S,7R,8S,9S,13Z,16S(Z))-4,8-Dihydroxy-5,5,7,9,13-pentamethyl-16-[1-fluor-2-(2-methyl-oxazol-4-yl)-vinyl]-oxacyclohexadec-13-en-2,6-dion;
(4S,7R,8S,9S,13Z,16S(Z))-4,8-Dihydroxy-16-[2-(2-hydroxymethyl-oxazol-4-yl)-1-fluor-vinyl]-5,5,7,9,13-pentamethyl-oxacyclohexadec-13-en-2,6-dion;
(4S,7R,8S,9S,13Z,16S(Z))-16-[2-(2-Aminomethyl-oxazol-4-yl)-1-fluor-vinyl]-4,8-dihydroxy-5,5,7,9,13-pentamethyl-oxacyclohexadec-13-en-2,6-dion;
(1S,3S(Z),7S,10R,11S,12S,16R)-7,11-Dihydroxy-8,8,10,12,16-pentamethyl-3-[1-fluor-2-(2-methyl-oxazol-4-yl)-vinyl]-4,17-dioxa-bicycio[14.1.0]heptadecan-5,9-dion;
(1S,3S(Z),7S,10R,11S,12S,16R)-7,11-Dihydroxy-3-[2-(2-hydroxymethyl-oxazol-4-yl)-1-fluor-vinyl]-8,8,10,12,16-pentamethyl-4,17-dioxa-bicyclo[14.1.0]heptadecan-5,9-dion;
(1S,3S(Z),7S,10R,11S,12S,16R)-3-[2-(2-Aminomethyl-oxazol-4-yl)-1-fluor-vinyl]-7,11-dihydroxy-8,8,10,12,16-pentamethyl-4,17-dioxa-bicycto[14.1.0]heptadecan-5,9-dion;
(4S,7R,8S,9S,13Z,16S(Z))-4,8-Dihydroxy-5,5,7,9,13-pentamethyl-16-[1-chlor-2-(2-methyl-oxazol-4-yl)-vinyl]-oxacyclohexadec-13-en-2,6-dion;
(4S,7R,8S,9S,13Z,16S(Z))-4,8-Dihydroxy-16-[2-(2-hydroxymethyl-oxazol-4-yl)-1-chlor-vinyl]-5,5,7,9,13-pentamethyl-oxacyclohexadec-13-en-2,6-dion;
(4S,7R,8S,9S,13Z,16S(Z))-16-[2-(2-Aminomethyl-oxazol-4-yl)-1-chlor-vinyl]-4,8-dihydroxy-5,5,7,9,13-pentamethyl-oxacyclohexadec-13-en-2,6-dion;
(1S,3S(Z),7S,10R,11S,12S,16R)-7,11-Dihydroxy-8,8,10,12,16-pentamethyl-3-[1-chlor-2-(2-methyl-oxazol-4-yl)-vinyl]-4,17-dioxa-bicyclo[14.1.0]heptadecan-5,9-dion;
(1S,3S(Z),7S,10R,11S,12S,16R)-7,11-Dihydroxy-3-[2-(2-hydroxymethyl-oxazol-4-yl)-1-chlor-vinyl]-8,8,10,12,16-pentamethyl-4,17-dioxa-bicyclo[14.1.0]heptadecan-5,9-dion;
(1S,3S(Z),7S,10R,11S,12S,16R)-3-[2-(2-Aminomethyl-oxazol-4-yl)-1-chlor-vinyl]-7,11-dihydroxy-8,8,10,12,16-pentamethyl-4,17-dioxa-bicyclo[14.1.0]heptadecan-5,9-dion;
(4S,7R,8S,9S,13Z,16S(Z))-4,8-Dihydroxy-7-ethyl-5,5,9,13-tetramethyl-16-[1-fluor-2-(2-methyl-oxazol-4-yl)-vinyl]-oxacyclohexadec-13-en-2,6-dion;
(4S,7R,8S,9S, 13Z,16S(Z))-4,8-Dihydroxy-16-[2-(2-hydroxymethyl-oxazol-4-yl)-1-fluor-vinyl]-7-ethyl-5,5,9,13-tetramethyl-oxacyclohexadec-13-en-2,6-dion;
(4S,7R,8S,9S, 13Z,165(Z))-16-[2-(2-Aminomethyl-oxazol-4-yl)-1-fluor-vinyl]-4,8-dihydroxy-7-ethyl-5,5,9,13-tetramethyl-oxacyclohexadec-13-en-2,6-dion;
(1S,3S(Z),7S,10R,11S,12S,16R)-7,11-Dihydroxy-10-ethyl-8,8,12,16-tetramethyl-3-[1-fluor-2-(2-methyl-oxazol-4-yl)-vinyl]-4,17-dioxa-bicyclo[14.1.0]heptadecan-5,9-dion;
(1S,3S(Z),7S,10R,11S,12S,16R)-7,11-Dihydroxy-3-[2-(2-hydroxymethyl-oxazol-4-yl)-1-fluor-vinyl]-10-ethyl-8,8,12,16-tetramethyl-4,17-dioxa-bicyclo[14.1.0]heptadecan-5,9-dion;
(1 S,3S(Z),7S,10R,11S,12S,16R)-3-[2-(2-Aminomethyl-oxazol-4-yl)-1-fluor-vinyl]-7,11-dihydroxy-10-ethyl-8,8,12,16-tetramethyl-4,17-dioxa-bicyclo[14.1.0]heptadecan-5,9-dion;
(4S,7R,8S,9S,13Z,16S(Z))-4,8-Dihydroxy-7-ethyl-5,5,9,13-tetramethyl-16-[1-chlor-2-(2-methyl-oxazol-4-yl)-vinyl]-oxacyclohexadec-13-en-2,6-dion;
(4S,7R,8S,9S,13Z,16S(Z))-4,8-Dihydroxy-16-[2-(2-hydroxymethyl-oxazol-4-yl)-1-chlor-vinyl]-7-ethyl-5,5,9,13-tetramethyl-oxacyclohexadec-13-en-2,6-dion;
(4S,7R,8S,9S,13Z,16S(Z))-16-[2-(2-Aminomethyl-oxazol-4-yl)-1-chlor-vinyl]-4,8-dihydroxy-7-ethyl-5,5,9,13-tetramethyl-oxacyclohexadec-13-en-2,6-dion;
(1S,3S(Z),7S,10R,11S,12S,16R)-7,11-Dihydroxy-10-ethyl-8,8,12,16-tetramethyl-3-[1-chlor-2-(2-methyl-oxazol-4-yl)-vinyl]-4,17-dioxa-bicyclo[14.1.0]heptadecan-5,9-dion;
(1S,3S(Z),7S,10R,11S,12S,16R)-7,11-Dihydroxy-3-[2-(2-hydroxymethyl-oxazol-4-yl)-1-chlor-vinyl]-10-ethyl-8,8,12,16-tetramethyl-4,17-dioxa-bicyclo[14.1.0]heptadecan-5,9-dion;
(1S,3S(Z),7S,10R,11S,12S,16R)-3-[2-(2-Aminomethyl-oxazol-4-yl)-1-chlor-vinyl]-7,11-dihydroxy-10-ethyl-8,8,12,16-tetramethyl-4,17-dioxa-bicyclo[14.1.0]heptadecan-5,9-dion;
(4S,7R,8S,9S,13Z,16S(E))-4,8-Dihydroxy-5,5,7,9,13-pentamethyl-16-[2-(2-methyl-thiazol-4-yl)-vinyl]-oxacyclohexadec-1 3-en-2,6-dion;
(4S,7R,8S,9S,13Z,16S(E))-4,8-Dihydroxy-16-[2-(2-hydroxymethyl-thiazol-4-yl)-vinyl]-5,5,7,9,13-pentamethyl-oxacyclohexadec-13-en-2,6-dion;
(4S,7R,8S,9S,13Z,16S(E))-16-[2-(2-Aminomethyl-thiazol-4-yl)-vinyl]-4,8-dihydroxy-5,5,7,9,13-pentamethyl-oxacyclohexadec-13-en-2,6-dion;
(1S,3S(E),7S,10R,11S,12S,16R)-7,11-Dihydroxy-8,8,10,12,16-pentamethyl-3-[2-(2-methyl-thiazol-4-yl)-vinyl]-4,17-dioxa-bicyclo[14.1.0]heptadecan-5,9-dion;
(1S,3S(E),7S,10R,11S,12S,16R)-7,11-Dihydroxy-3-[2-(2-hydroxymethyl-thiazol-4-yl)-vinyl]-8,8,10,12,16-pentamethyl-4,17-dioxa-bicyclo[14.1.0]heptadecan-5,9-dion;
(1S,3S(E),7S,10R,11S,12S,16R)-3-[2-(2-Aminomethyl-thiazol-4-yl)-vinyl]-7,11 -dihydroxy-8,8,10,12,16-pentamethyl-4,17-dioxa-bicyclo[14.1.0]heptadecan-5,9-dion;
(4S,7R,8S,9S,13Z,16S(E))-4,8-Dihydroxy-7-ethyl-5,5,9,13-tetramethyl-16-[2-(2-methyl-thiazol-4-yl)-vinyl]-oxacyclohexadec-13-en-2,6-dion;
(4S,7R,8S,9S,13Z,16S(E))-4,8-Dihydroxy-16-[2-(2-hydroxymethyl-thiazol-4-yl)-vinyl]-7-ethyl-5,5,9,13-tetramethyl-oxacyclohexadec-13-en-2,6-dion;
(4S,7R,8S,9S,13Z,16S(E))-16-[2-(2-Aminomethyl-thiazol-4-yl)-vinyl]-4,8-dihydroxy-7-ethyl-5,5,9,13-tetramethyl-oxacyclohexadec-13-en-2,6-dion;
(1S,3S(E),7S,10R,11S,12S,16R)-7,11-Dihydroxy-10-ethyl-8,8,12,16-tetramethyl-3-[2-(2-methyl-thiazol-4-yl)-vinyl]-4,17 -dioxa-bicyclo[14.1.0]heptadecan-5,9-dion;
(1S,3S(E), 7S,10R,11S,12S,16R)-7,11-Dihydroxy-3-[2-(2-hydroxymethyl-thiazol-4-yl)-vinyl]-10-ethyl-8,8,12,16-tetramethyl-4,17-dioxa-bicyclo[14.1.0]heptadecan-5,9-dion;
(1S,3S(E),7S,10R,11S,12S,16R)-3-[2-(2-Aminomethyl-thiazol-4-yl)-vinyl]-7,11-dihydroxy-10-ethyl-8,8,12,16-tetramethyl-4,17-dioxa-bicyclo[14.1.0]heptadecan-5,9-dion;
(4S,7R,8S,9S,13Z,16S(E))-4,8-Dihydroxy-5,5,7,9,13-pentamethyl-16-[2-(2-pyridyl)-vinyl]-oxacyclohexadec-13-en-2,6-dion;
(1S,3S(E),7S,10R,11S,12S,16R)-7,11-Dihydroxy-8,8,10,12,16-pentamethyl-3-[2-(2-pyridyl)-vinyl]-4,17-dioxa-bicyclo[14.1.0]heptadecan-5,9-dion;
(4S.7R.8S.9S, 13Z, 16S(E))-4,8-Dihydroxy-7-ethyl-5,5,9,13-tetramethyl-16-[2-(2-pyridyl)-vinyl]-oxacyclohexadec-13-en-2,6-dion;
(1S,3S(E),7S,10R,11S,12S,16R)-7,11-Dihydroxy-10-ethyl-8,8,12,16-tetramethyl-3-[2-(2-pyridyl)-vinyl]-4,17-dioxa-bicyclo[14.1.0]heptadecan-5,9-dion;
(4S,7R,8S,9S,13Z,16S)-4,8-Dihydroxy-5,5,7,9,13-pentamethyl-16-(2-methyl-benzothiazol-5-yl)-oxacyclohexadec-13-en-2,6-dion;
(4S,7R,8S,9S,13Z,16S)-4,8-Dihydroxy-16-(2-hydroxymethyl-benzothiazol-5-yl)-5,5,7,9,13-pentamethyl-oxacyclohexadec-13-en-2,6-dion;
(4S,7R,8S,9S,13Z,16S)-16-(2-Aminomethyl-benzothiazol-5-yl)-4,8-dihydroxy-5, 5, 7,9,13-pentamethyl-oxacyclohexadec-13-en-2,6-dion;
(1S,3S,7S,10R,11S,12S,16R)-7,11-Dihydroxy-8,8,10,12,16-pentamethyl-3-(2-methyl-benzothiazol-5-yl)-4,17-dioxa-bicyclo[14.1.0]heptadecan-5,9-dion;
(1S,3S,7S,10R,11S,12S,16R)-7,11-Dihydroxy-3-(2-hydroxymethyl-benzothiazol-5-yl)-8,8,10,12,16-pentamethyl-4,17-dioxa-bicyclo[14.1.0]heptadecan-5,9-dion;
(1S,3S,7S,10R,11S,12S,16R)-3-(2-Aminomethyl-benzothiazol-5-yl)-7,11-dihydroxy-8,8,10,12,16-pentamethyl-4,17-dioxa-bicyclo[14.1.0]heptadecan-5,9-dion;
(4S,7R,8S,9S,13Z,16S)-4,8-Dihydroxy-7-ethyl-5,5,9,13-tetramethyl-16-(2-methyl-benzothiazol-5-yl)-oxacyclohexadec-13-en-2,6-dion;
(4S,7R,8S,9S,13Z,16S)-4,8-Dihydroxy-16-(2-hydroxymethyl-benzothiazol-5-yl)-7-ethyl-5,5,9,13-tetramethyl-oxacyclohexadec-13-en-2,6-dion;
(4S,7R,8S,9S, 13Z, 16S)-16-(2-Aminomethyl-benzothiazol-5-yl)-4,8-dihydroxy-7-ethyl-5,5,9,13-tetramethyl-oxacyclohexadec-13-en-2,6-dion;
(1S,3S,7S,10R,11S,12S,16R)-7,11-Dihydroxy-10-ethyl-8,8,12,16-tetramethyl-3-(2-methyl-benzothiazol-5-yl)-4,17-dioxa-bicyclo[14.1.0]heptadecan-5,9-dion;
(1S,3S,7S,10R,11S,12S,16R)-7,11-Dihydroxy-3-(2-hydroxymethyl-benzothiazol-5-yl)-10-ethyl-8,8,12,16-tetramethyl-4,17-dioxa-bicyclo[14.1.0]heptadecan-5,9-dion;
(1S,3S,7S,10R,11S,12S,16R)-3-(2-Aminomethyl-benzothiazol-5-yl)-7,11-dihydroxy-10-ethyl-8,8,12,16-tetramethyl-4,17-dioxa-bicyclo[14.1.0]heptadecan-5,9-dion;
(4S,7R,8S,9S,13Z,16S)-4,8-Dihydroxy-7-propyl-5,5,9,13-tetramethyl-16-(2-methyl-benzothiazol-5-yl)-oxacyclohexadec-13-en-2,6-dion;
(4S,7R,8S,9S,13Z,16S)-4,8-Dihydroxy-16-(2-hydroxymethyl-benzothiazol-5-yl)-7-propyl-5,5,9,13-tetramethyl-oxacyclohexadec-13-en-2,6-dion;
(4S,7R,8S,9S,13Z,16S)-16-(2-Aminomethyl-benzothiazol-5-yl)-4,8-dihydroxy-7-propyl-5,5,9,13-tetramethyl-oxacyclohexadec-13-en-2,6-dion;
(1S,3S,7S,10R,11S,12S,16R)-7,11-Dihydroxy-10-propyl-8,8,12,16-tetramethyl-3-(2-methyl-benzothiazol-5-yl)-4,17-dioxa-bicyclo[14.1.0]heptadecan-5,9-dion;
(1S,3S,7S,10R,11S,12S,16R)-7,11-Dihydroxy-3-(2-hydroxymethyl-benzothiazol-5-yl)-10-propyl-8,8,12,16-tetramethyl-4,17-dioxa-bicyclo[14.1.0]heptadecan-5,9-dion;
(1S,3S,7S,10R,11S,12S,16R)-3-(2-Aminomethyl-benzothiazol-5-yl)-7,11-dihydroxy-10-propyl-8,8,12,16-tetramethyl-4,17-dioxa-bicyclo[14.1.0]heptadecan-5,9-dion;
(4S,7R,8S,9S,13Z,16S)-4,8-Dihydroxy-7-butyl-5,5,9,13-tetramethyl-16-(2-methyl-benzothiazol-5-yl)-oxacyclohexadec-13-en-2,6-dion;
(4S,7R,8S,9S,13Z,16S)-4,8-Dihydroxy-16-(2-hydroxymethyl-benzothiazol-5-yl)-7-butyl-5,5,9,13-tetramethyl-oxacyclohexadec-13-en-2,6-dion;
(4S,7R,8S,9S,13Z,16S)-16-(2-Aminomethyl-benzothiazol-5-yl)-4,8-dihydroxy-7-butyl-5,5,9,13-tetramethyl-oxacyclohexadec-13-en-2,6-dion;
(1S,3S,7S,10R,11S,12S,16R)-7,11-Dihydroxy-10-butyl-8,8,12,16-tetramethyl-3-(2-methyl-benzothiazol-5-yl)-4,17-dioxa-bicyclo[14.1.0]heptadecan-5,9-dion;
(1S,3S,7S,10R,11S,12S,16R)-7,11-Dihydroxy-3-(2-hydroxymethyl-benzothiazol-5-yl)-10-butyl-8,8,12,16-tetramethyl-4,17-dioxa-bicyclo[14.1.0]heptadecan-5,9-dion;
(1S,3S,7S,10R,11S,12S,16R)-3-(2-Aminomethyl-benzothiazol-5-yl)-7,11-dihydroxy-10-butyl-8,8,12,16-tetramethyl-4,17-dioxa-bicyclo[14.1.0]heptadecan-5,9-dion;
(4S,7R,8S,9S,13Z,16S)-4,8-Dihydroxy-7-allyl-5,5,9,13-tetramethyl-16-(2-methyl-benzothiazol-5-yl)-oxacyclohexadec-13-en-2,6-dion;
(4S,7R,8S,9S,13Z,16S)-4,8-Dihydroxy-16-(2-hydroxymethyl-benzothiazol-5-yl)-7-allyl-5,5,9,13-tetramethyl-oxacyclohexadec-13-en-2,6-dion;
(4S,7R,8S,9S,13Z,16S)-16-(2-Aminomethyl-benzothiazol-5-yl)-4,8-dihydroxy-7-allyl-5,5,9,13-tetramethyl-oxacyclohexadec-13-en-2,6-dion;
(1S,3S,7S,10R,11S,12S,16R)-7,11-Dihydroxy-10-allyl-8,8,12,16-tetramethyl-3-(2-methyl-benzothiazol-5-yl)-4,17-dioxa-bicyclo[14.1.0]heptadecan-5,9-dion;
(1S,3S,7S,10R,11S,12S,16R)-7,11-Dihydroxy-3-(2-hydroxymethyl-benzothiazol-5-yl)-10-allyl-8,8,12,16-tetramethyl-4,17-dioxa-bicyclo[14.1.O]heptadecan-5,9-dion;
(1S,3S,7S,10R,11S,12S,16R)-3-(2-Aminomethyl-benzothiazol-5-yl)-7,11-dihydroxy-10-allyl-8,8,12,16-tetramethyl-4,17-dioxa-bicyclo[14.1.0]heptadecan-5,9-dion;
(4S,7R,8S,9S,13Z,16S)-4,8-Dihydroxy-7-prop-2-inyl-5, 5,9,13-tetramethyl-16-(2-methyl-benzothiazol-5-yl)-oxacyclohexadec-13-en-2,6-dion;
(4S,7R,8S,9S,13Z,16S)-4,8-Dihydroxy-16-(2-hydroxymethyl-benzothiazol-5-yl)-7-prop-2-inyl-5,5,9,13-tetramethyl-oxacyclohexadec-13-en-2,6-dion;
(4S,7R,8S,9S,13Z,16S)-16-(2-Aminomethyl-benzothiazol-5-yl)-4,8-dihydroxy-7-prop-2-inyl-5,5,9,13-tetramethyl-oxacyclohexadec-13-en-2,6-dion;
(1S,3S,7S,10R,11S,12S,16R)-7,11-Dihydroxy-10-prop-2-inyl-8,8,12,16-tetramethyl-3-(2-methyl-benzothiazol-5-yl)-4,17-dioxa-bicyclo[14.1.0]heptadecan-5,9-dion;
(1S,3S,7S,10R,11S,12S,16R)-7,11-Dihydroxy-3-(2-hydroxymethyl-benzothiazol-5-yl)-10-prop-2-inyl-8,8,12,16-tetramethyl-4,17-dioxa-bicyclo[14.1.0]heptadecan-5,9-dion;
(1S,3S,7S,10R,11S,12S,16R)-3-(2-Aminomethyl-benzothiazol-5-yl)-7,11-dihydroxy-10-prop-2-inyl-8,8,12,16-tetramethyl-4,17-dioxa-bicyclo[14.1.0]heptadecan-5,9-dion;
(4S,7R,8S,9S,13Z,16S)-4,8-Dihydroxy-7-but-3-enyl-5,5,9,13-tetramethyl-16-(2-methyl-benzothiazol-5-yl)-oxacyclohexadec-13-en-2,6-dion;
(4S,7R,8S,9S,13Z,16S)-4,8-Dihydroxy-16-(2-hydroxymethyl-benzothiazol-5-yl)-7-but-3-enyl-5,5,9,13-tetramethyl-oxacyclohexadec-13-en-2,6-dion;
(4S,7R,8S,9S,13Z,16S)-16-(2-Aminomethyl-benzothiazol-5-yl)-4,8-dihydroxy-7-but-3-enyl-5,5,9,13-tetramethyl-oxacyclohexadec-13-en-2,6-dion;
(1S,3S,7S,10R,11S,12S,16R)-7,11-Dihydroxy-10-but-3-enyl-8,8,12,16-tetramethyl-3-(2-methyl-benzothiazol-5-yl)-4,17-dioxa-bicyclo[14.1.0]heptadecan-5,9-dion;
(1S,3S,7S,10R,11S,12S,16R)-7,11-Dihydroxy-3-(2-hydroxymethyl-benzothiazol-5-yl)-10-but-3-enyl-8,8,12,16-tetramethyl-4,17-dioxa-bicyclo[14.1.0]heptadecan-5,9-dion;
(1S,3S,7S,10R,11S,12S,16R)-3-(2-Aminomethyl-benzothiazol-5-yl)-7,11-dihydroxy-10-but-3-enyl-8,8,12,16-tetramethyl-4,17-dioxa-bicyclo[14.1.0]heptadecan-5,9-dion;
(4S,7R,8S,9S,13Z,16S)-4,8-Dihydroxy-7-but-3-inyl-5,5,9,13-tetramethyl-16-(2-methyl-benzothiazol-5-yl)-oxacyclohexadec-13-en-2,6-dion;
(4S,7R,8S,9S,13Z,16S)-4,8-Dihydroxy-16-(2-hydroxymethyl-benzothiazol-5-yl)-7-but-3-inyl-5,5,9,13-tetramethyl-oxacyclohexadec-13-en-2,6-dion;
(4S,7R,8S,9S,13Z,16S)-16-(2-Aminomethyl-benzothiazol-5-yl)-4,8-dihydroxy-7-but-3-inyl-5,5,9,13-tetramethyl-oxacyclohexadec-13-en-2,6-dion;
(1S,3S,7S,10R,11S,12S,16R)-7,11-Dihydroxy-10-but-3-inyl-8,8,12,16-tetramethyl-3-(2-methyl-benzothiazol-5-yl)-4,17-dioxa-bicyclo[14.1.0]heptadecan-5,9-dion;
(1S,3S,7S,10R,11S,12S,16R)-7,11-Dihydroxy-3-(2-hydroxymethyl-benzothiazol-5-yl)-10-but-3-inyl-8,8,12,16-tetramethyl-4,17-dioxa-bicyclo[14.1.0]heptadecan-5,9-dion;
(1S,3S,7S,10R,11S,12S,16R)-3-(2-Aminomethyl-benzothiazol-5-yl)-7,11-dihydroxy-10-but-3-inyl-8,8,12,16-tetramethyl-4,17-dioxa-bicyclo[14.1.0]heptadecan-5,9-dion;
(4S,7R,8S,9S,13Z,16S)-4,8-Dihydroxy-5,5,7,9,13-pentamethyl-16-(2-methyl-benzoxazol-5-yl)-oxacyclohexadec-13-en-2,6-dion;
(4S,7R,8S,9S,13Z,16S)-4,8-Dihydroxy-16-(2-hydroxymethyl-benzoxazol-5-yl)-5, 5,7,9,13-pentamethyl-oxacyclohexadec-13-en-2,6-dion;
(4S,7R,8S,9S,13Z,16S)-16-(2-Aminomethyl-benzoxazol-5-yl)-4,8-dihydroxy-5,5,7,9,13-pentamethyl-oxacyclohexadec-13-en-2,6-dion;
(1S,3S,7S,10R,11S,12S,16R)-7,11-Dihydroxy-8,8,10,12,16-pentamethyl-3-(2-methyl-benzoxazol-5-yl)-4,17-dioxa-bicyclo[14.1.0]heptadecan-5,9-dion;
(1S,3S,7S,10R,11S,12S,16R)-7,11-Dihydroxy-3-(2-hydroxymethyl-benzoxazol-5-yl)-8,8,10,12,16-pentamethyl-4,17-dioxa-bicyclo[14.1.0]heptadecan-5,9-dion;
(1S,3S,7S,10R,11S,125,16R)-3-(2-Aminomethyl-benzoxazol-5-yl)-7,11-dihydroxy-8,8,10,12,16-pentamethyl-4,17-dioxa-bicyclo[14.1.0]heptadecan-5,9-dion;
(4S,7R,8S,9S,13Z,16S)-4,8-Dihydroxy-7-ethyl-5,5,9,13-tetramethyl-16-(2-methyl-benzoxazol-5-yl)-oxacyclohexadec-13-en-2,6-dion;
(4S,7R,8S,9S,13Z,16S)-4,8-Dihydroxy-16-(2-hydroxymethyl-benzoxazol-5-yl)-7-ethyl-5,5,9,13-tetramethyl-oxacyclohexadec-13-en-2,6-dion;
(4S,7R,8S,9S,13Z,16S)-16-(2-Aminomethyl-benzoxazol-5-yl)-4,8-dihydroxy-7-ethyl-5,5,9,13-tetramethyl-oxacyclohexadec-13-en-2,6-dion;
(1S,3S,7S,10R,11S,12S,16R)-7,11-Dihydroxy-10-ethyl-8,8,12,16-tetramethyl-3-(2-methyl-benzoxazol-5-yl)-4,17-dioxa-bicyclo[14.1.0]heptadecan-5,9-dion;
(1S,3S,7S,10R,11S,12S,16 R)-7,11-Dihydroxy-3-(2-hydroxymethyl-benzoxazol-5-yl)-10-ethyl-8,8,12,16-tetramethyl-4,17-dioxa-bicyclo[14.1.0]heptadecan-5,9-dion;
(1S,3S,7S,10R,11S,12S,16R)-3-(2-Aminomethyl-benzoxazol-5-yl)-7,11-dihydroxy-10-ethyl-8,8,12,16-tetramethyl-4,17-dioxa-bicyclo[14.1.0]heptadecan-5,9-dion;
(4S,7R,8S,9S,13Z,16S)-4,8-Dihydroxy-7-propyl-5,5,9,13-tetramethyl-16-(2-methyl-benzoxazol-5-yl)-oxacyclohexadec-13-en-2,6-dion;
(4S,7R,8S,9S,13Z,16S)-4,8-Dihydroxy-16-(2-hydroxymethyl-benzoxazol-5-yl)-7-propyl-5,5,9,13-tetramethyl-oxacyclohexadec-13-en-2,6-dion;
(45,7R,S5,95, 13Z, 16S)-16-(2-Aminomethyl-benzoxazol-5-yl)-4,8-dihydroxy-7-propyl-5,5,9,13-tetramethyl-oxacyclohexadec-13-en-2,6-dion;
(1S,3S,7S,10R,11S,12S,16R)-7,1 1 -Dihydroxy-1 0-propyl-8,8,12,16-tetramethyl-3-(2-methyl-benzoxazol-5-yl)-4,17-dioxa-bicyclo[14.1.0]heptadecan-5,9-dion;
(1S,3S,7S,10R,11S,12S,16R)-7,11-Dihydroxy-3-(2-hydroxymethyl-benzoxazol-5-yl)-10-propyl-8,8,12,16-tetramethyl-4,17-dioxa-bicyclo[14.1.0]heptadecan-5,9-dion;
(1S,3S,7S,10R,11S,12S,16R)-3-(2-Aminomethyl-benzoxazol-5-yl)-7,11-dihydroxy-10-propyl-8,8,12,16-tetramethyl-4,17-dioxa-bicyclo[14.1.0]heptadecan-5,9-dion;
(4S,7R,8S,9S, 13Z,16S)-4,8-Dihydroxy-7-butyl-5,5,9,13-tetramethyl-16-(2-methyl-benzoxazol-5-yl)-oxacyclohexadec-13-en-2,6-dion;
(4S,7R,8S,9S,13Z,16S)-4,8-Dihydroxy-16-(2-hydroxymethyl-benzoxazol-5-yl)-7-butyl-5,5,9,13-tetramethyl-oxacyclohexadec-13-en-2,6-dion;
(4S,7R,8S,9S,13Z,16S)-16-(2-Aminomethyl-benzoxazol-5-yl)-4,8-dihydroxy-7-butyl-5,5,9, 13-tetramethyl-oxacyclohexadec-13-en-2,6-dion;
(1S,3S,7S,10R,11S,12S,16R)-7,11-Dihydroxy-10-butyl-8,8,12,16-tetramethyl-3-(2-methyl-benzoxazol-5-yl)-4,17-dioxa-bicyclo[14.1.0]heptadecan-5,9-dion;
(1S,3S,7S,10R,11S,12S,16R)-7,11-Dihydroxy-3-(2-hydroxymethyl-benzoxazol-5-yl)-10-butyl-8,8,12,16-tetramethyl-4,17-dioxa-bicyclo[14.1.0]heptadecan-5,9-dion;
(1S,3S,7S,10R,11S,12S,16R)-3-(2-Aminomethyl-benzoxazol-5-yl)-7,11-dihydroxy-10-butyl-8,8,12,16-tetramethyl-4,17-dioxa-bicyclo[14.1.0]heptadecan-5,9-dion;
(4S,7R,8S,9S,13Z,16S)-4,8-Dihydroxy-7-allyl-5,5,9,13-tetramethyl-16-(2-methyl-benzoxazol-5-yl)-oxacyclohexadec-13-en-2,6-dion;
(4S,7R,8S,9S,13Z,16S)-4,8-Dihydroxy-16-(2-hydroxymethyl-benzoxazol-5-yl)-7-allyl-5,5,9,13-tetramethyl-oxacyclohexadec-13-en-2,6-dion;
(4S,7R,8S,9S,13Z,16S)-16-(2-Aminomethyl-benzoxazol-5-yl)-4,8-dihydroxy-7-allyl-5,5,9,13-tetramethyl-oxacyclohexadec-13-en-2,6-dion;
(1S,3S,7S,10R,11S,12S,16R)-7,11-Dihydroxy-10-allyl-8,8,12,16-tetramethyl-3-(2-methyl-benzoxazol-5-yl)-4,17-dioxa-bicyclo[14.1.0]heptadecan-5,9-dion;
(1S,3S,7S,10R,11S,12S,16R)-7,11-Dihydroxy-3-(2-hydroxymethyl-benzoxazol-5-yl)-10-allyl-8,8,12,16-tetramethyl-4,17-dioxa-bicyclo[14.1.0]heptadecan-5,9-dion;
(1S,3S,7S,10R,11S,12S,16R)-3-(2-Aminomethyl-benzoxazol-5-yl)-7,11-dihydroxy-10-allyl-8,8,12,16-tetramethyl-4,17-dioxa-bicyclo[14.1.0]heptadecan-5,9-dion;
(4S,7R,8S,9S,13Z,16S)-4,8-Dihydroxy-7-prop-2-inyl-5,5,9,13-tetramethyl-16-(2-methyl-benzoxazol-5-yl)-oxacyclohexadec-13-en-2,6-dion;
(4S,7R,8S,9S,13Z,16S)-4,8-Dihydroxy-16-(2-hydroxymethyl-benzoxazol-5-yl)-7-prop-2-inyl-5,5,9,13-tetramethyl-oxacyclohexadec-13-en-2,6-dion ;
(4S,7R,8S,9S,13Z,16S)-16-(2-Aminomethyl-benzoxazol-5-yl)-4,8-dihydroxy-7-prop-2-inyl-5,5,9,13-tetramethyl-oxacyclohexadec-13-en-2,6-dion;
(1S,3S,7S,10R,11S,12S,16R)-7,11-Dihydroxy-10-prop-2-inyl-8.8.12,16-tetramethyl-3-(2-methyl-benzoxazol-5-yl)-4,17-dioxa-bicyclo[14.1.0]heptadecan-5,9-dion;
(1S,3S,7S,10R,11S,12S,16R)-7,11-Dihydroxy-3-(2-hydroxymethyl-benzoxazol-5-yl)-10-prop-2-inyl-8,8,12,16-tetramethyl-4,17-dioxa-bicyclo[14.1.0]heptadecan-5,9-dion ;
(1S,3S,7S,10R,11S,12S,16R)-3-(2-Aminomethyl-benzoxazol-5-yl)-7,11-dihydroxy-10-prop-2-inyl-8,8,12,16-tetramethyl-4,17-dioxa-bicyclo[14.1.0]heptadecan-5,9-dion;
(4S,7R,8S,9S,13Z,16S)-4,8-Dihydroxy-7-but-3-enyl-5,5,9,13-tetramethyl-16-(2-methyl-benzoxazol-5-yl)-oxacyclohexadec-13-en-2,6-dion;
(4S,7R,8S,9S,13Z,16S)-4,8-Dihydroxy-16-(2-hydroxymethyl-benzoxazol-5-yl)-7-but-3-enyl-5,5,9,13-tetramethyl-oxacyclohexadec-13-en-2,6-dion;
(4S,7R,8S,9S,13Z,16S)-16-(2-Aminomethyl-benzoxazol-5-yl)-4,8-dihydroxy-7-but-3-enyl-5,5,9,13-tetramethyl-oxacyclohexadec-13-en-2,6-dion;
(1S,3S,7S,10R,11S,12S,16R)-7,11-Dihydroxy-10-but-3-enyl-8,8,12,16-tetramethyl-3-(2-methyl-benzoxazol-5-yl)-4,17-dioxa-bicyclo[14.1.0]heptadecan-5,9-dion;
(1S,3S,7S,10R,11S,12S,16R)-7,11-Dihydroxy-3-(2-hydroxymethyl-benzoxazol-5-yl)-10-but-3-enyl-8,8,12,16-tetramethyl-4,17-dioxa-bicyclo[14.1.0]heptadecan-5,9-dion;
(1S,3S,7S,10R,11S,12S,16R)-3-(2-Aminomethyl-benzoxazol-5-yl)-7,11-dihydroxy-10-but-3-enyl-8,8,12,16-tetramethyl-4,17-dioxa-bicyclo[14.1.0]heptadecan-5,9-dion;
(4S,7R,8S,9S,13Z,16S)-4,8-Dihydroxy-7-but-3-inyl-5,5,9,13-tetramethyl-16-(2-methyl-benzoxazol-5-yl)-oxacyclohexadec-13-en-2,6-dion;
(4S,7R,8S,9S,13Z,16S)-4,8-Dihydroxy-16-(2-hydroxymethl-benzoxazol-5-yl)-7-but-3-inyl-5,5,9,13-tetramethyl-oxacyclohexadec-13-en-2,6-dion;
(4S,7R,8S,9S,13Z,16S)-16-(2-Aminomethyl-benzoxazol-5-yl)-4,8-dihydroxy-7-but-3-inyl-5,5,9,13-tetramethyl-oxacyclohexadec-13-en-2,6-dion;
(1S,3S,7S,10R,11S,12S,16R)-7,11-Dihydroxy-10-but-3-inyl-8,8,12,16-tetramethyl-3-(2-methyl-benzoxazol-5-yl)-4,17-dioxa-bicyclo[14.1.0]heptadecan-5,9-dion;
(1S,3S,7S,10R,11S,12S,16R)-7,11-Dihydroxy-3-(2-hydroxymethyl-benzoxazol-5-yl)-10-but-3-inyl-8,8,12,16-tetramethyl-4,17-dioxa-bicyclo[14.1.0]heptadecan-5,9-dion;
(1S,3S,7S,10R,11S,125,16R)-3-(2-Aminomethyl-benzoxazol-5-yl)-7,11-dihydroxy-10-but-3-inyl-8,8,12,16-tetramethyl-4,17-dioxa-bicyclo[14.1.0]heptadecan-5,9-dion;
wobei die Wasserstoffatome in den obengenannten Effektor-Grundkörpern an den in Formel (I) angegebenen Positionen durch Reste L¹- L³ ersetzt sind.

4. Effektor-Konjugat gemäss einem der Ansprüche 1-3, wobei der Linker ausgewählt ist aus der Gruppe bestehend aus Verbindungen der allgemeinen Formel (III), wobei Aa1 eine Bindung darstellt.

5. Effektor-Konjugat gemäss einem der Ansprüche 1-4, wobei
FG¹ C₁-C₁₀ Alkyl-S₃ oder 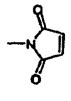 ist.

6. Effektor-Erkennungseinheit-Konjugat der allgemeinen Formel (I), 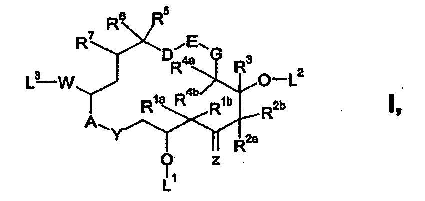 wobei die Substituenten darin die in Anspruch 1 genannten Bedeutungen haben, jedoch mindestens eine Gruppe FG¹ durch eine Gruppe FG^{2a} oder FG^{2b} ersetzt ist, wobei FG^{2a} bzw. FG^{2b} die folgenden Bedeutungen haben können:
FG₂ₐ-S-S- 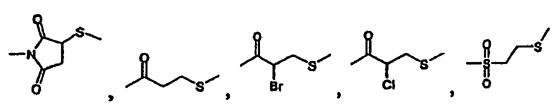 ist
FG_{2b} -CONH- ist
und wobei eine Erkennungseinheit über ein Schwefelatom mit der Gruppe FG^{2a}, wobei das gezeigte Schwefelatom Bestandteil der Erkennungseinheit ist, oder über eine Amidgruppe mit Gruppe FG^{2b}, wobei das gezeigte Stickstoffatom Bestandteil der Erkennungseinheit ist, konjugiert ist;
wobei die Erkennungseinheit ausgewählt ist aus der Gruppe bestehend aus Peptiden, löslichen Rezeptoren, Cytokinen, Lymphokinen, Aptameren, Spiegelmeren, rekombinanten Proteinen, neuen Framework-Strukturen, monoklonalen Antikörpern, und Fragmenten monoklonaler Antikörper;
als einheitliches Isomer oder eine Mischung unterschiedlicher Isomere und/oder als ein pharmazeutisch akzeptables Salz hiervon.

7. Effektor-Erkennungseinheit-Konjugat gemäss Anspruch 6, wobei das Konjugat mehr als eine Erkennungseinheit enthält, und wobei die Erkennungseinheiten identisch sind.

8. Effektor-Erkennungseinheit-Konjugat gemäss Anspruch 6 oder 7, wobei die Erkennungseinheit ein Antikörper ist, oder ein antigen-bindendes Fragment desselben, welcher für ein Antigen spezifisch ist, das ausgewählt ist aus der Gruppe bestehend aus den in Tabelle 1 aufgeführten Antigenen, sowie CD19, CD20, CD40, CD22, CD25, CD5, CD52, CD10, CD2, CD7, CD33, CD38, CD40, CD72, CD4, CD21, CD37, CD30, VCAM, CD31, ELAM, Endoglin, VEGFRI/II, VEGFRvIII, scFv(14E1), αᵥβ₃, Tie1/2, TES23 (CD44ex6), Phosphatidylserin, PSMA, VEGFR/VEGF-Komplex und ED-B-Fibronectin.

9. Linker der allgemeinen Formel (III¹): 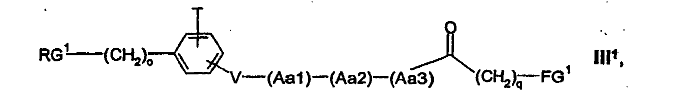 worin
RG¹ eine O=C=N-Gruppe ist, und o, V, q, T, Aa1, Aa2, Aa3 und FG¹ die in Anspruch 1 genannten Bedeutungen haben;
oder Linker der allgemeinen Formel (III₂): 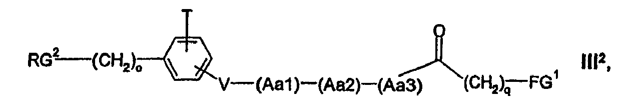 worin
RG₂ eine Hal-C(=O)-CHR²²-Gruppe oder eine Hal-C(=O)-CHR²²-NR²³-C(=O)-Gruppe oder eine R²⁶-C(=O)-O-C(=O)-CHR²²-Gruppe oder eine R²⁶-C(=O)-O-C(=O)-CHR²²-NR²³-C(=O)-Gruppe ist, wobei R²⁶ C₁-C₁₀ Alkyl, Aryl, Aralkyl ist, und o, V, q, T, Aa1, Aa2, Aa3 und FG¹ die in Anspruch 1 genannten Bedeutungen haben;
oder Linker der allgemeinen Formel (III³): 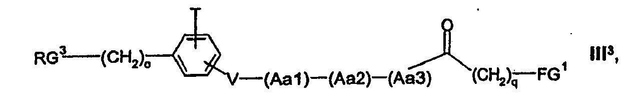 worin
RG³ eine OH-Gruppe oder eine NHR^{24a}-Gruppe oder eine COOH-Gruppe ist, und o, V, q, T, Aa1, Aa2, Aa3 und FG¹ die in Anspruch 1 genannten Bedeutungen haben.

10. Verfahren zur Herstellung von Effektor-Konjugaten gemäss einem der Ansprüche 1-5,
wobei eine Verbindung der allgemeinen Formel (I), wobei die Substituenten die in Anspruch 1 genannten Bedeutungen haben, jedoch
die Bedingung, das mindestens eine Gruppe L¹, L² oder L⁴ einen Linker der allgemeinen Formel (III) darstellt, nicht erfüllt sein muss, und
mindestens ein Substituent L¹, L² oder L⁴ Wasserstoff oder eine Gruppe C(=O)Cl darstellt;
mit einem Linker, der ausgewählt ist aus der Gruppe bestehend aus einem Linker der allgemeinen Formel (III¹) oder (III₂) oder (III³), wie in Anspruch 9 beschrieben, umgesetzt wird.

11. Verfahren zur Herstellung von Effektor-Erkennungseinheit-Konjugaten gemäss einem der Ansprüche 6 bis 8, wobei ein Effektor-Konjugat gemäss einem der Ansprüche 1-5 mit mindestens einer Erkennungseinheit, wie in Anspruch 6 und 8 definiert, umgesetzt wird.

12. Verwendung einer Verbindung der allgemeinen Formel (I), wobei die Substituenten die in Anspruch 1 genannten Bedeutungen haben, jedoch
die Bedingung, dass mindestens eine Gruppe L¹, L² oder L⁴ einen Linker der allgemeinen Formel (III) darstellt, nicht erfüllt sein muss, und
mindestens ein Substituent L¹, L² oder L⁴ Wasserstoff oder eine Gruppe C(=O)Cl darstellt;
in einem Verfahren gemäss Anspruch 10.

13. Verwendung einer Verbindung der allgemeinen Formel (I) zur Herstellung eines Effektor-Erkennungseinheit-Konjugats gemäss den Ansprüchen 6 bis 8.

14. Verwendung eines Linkers der allgemeinen Formel (III¹), (III₂) oder (III³) in einem Verfahren gemäss Anspruch 10.

15. Verwendung eines Linkers der allgemeinen Formel (III¹), (III₂) oder (III³) zur Herstellung eines Effektor-Erkennungseinheit-Konjugats gemäss einem der Ansprüche 6 bis 8.

16. Verwendung einer Erkennungseinheit, wie in Anspruch 6 oder 8 definiert, in einem Verfahren gemäss Anspruch 11.

17. Effektor-Erkennungseinheit-Konjugat gemäss einem der Ansprüche 6 bis 8 zur Verwendung als Medikament.

18. Effektor-Erkennungseinheit-Konjugat gemäss einem der Ansprüche 6 bis 8 zur Verwendung als Medikament zur Behandlung von Erkrankungen, die mit proliferativen Prozessen assoziiert sind.

19. Effektor-Erkennungseinheit-Konjugat gemäss einem der Ansprüche 6 bis 8 zur Verwendung als Medikament zur Behandlung einer Krankheit, die ausgewählt ist aus der Gruppe bestehend aus Tumoren, entzündlichen Erkrankungen, neurodegenerativen Erkrankungen, Angiogenese-assoziierten Erkrankungen, Multipler Sklerose, Morbus Alzheimer, und rheumatoider Arthritis.

## Revendications

1. Conjugué d'effecteurs de formule générale (I) : 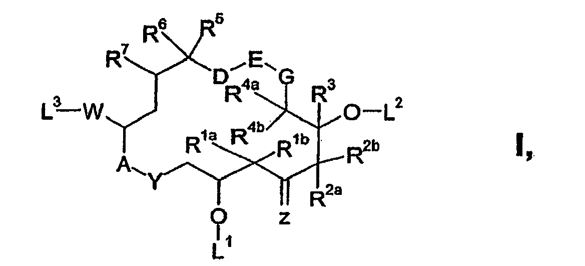 dans laquelle :
R^{1a}, R^{1b} sont, indépendamment l'un de l'autre, hydrogène, C₁-C₁₀-alkyle, aryle, aralkyle ou, ensemble, un groupement -(CH₂)ₘ dans lequel est m est de 2 à 5 ;
R^{2a}, R^{2b} sont, indépendamment l'un de l'autre, hydrogène, C₁-C₁₀-alkyle, aryle, aralkyle ou, ensemble, un groupement -(CH₂)ₙ dans lequel n est de 2 à 5, ou C₂-C₁₀-alcényle ou C₂-C₁₀-alcinyle ;
R³ est hydrogène, C₁-C₁₀-alkyle, aryle ou aralkyle, et
R^{4a}, R^{4b} sont, indépendamment l'un de l'autre, hydrogène, C₁-C₁₀-alkyle, aryle, aralkyle ou, ensemble, un groupement -(CH₂)p dans lequel p est de 2 à 5 ;
R⁵ est hydrogène, C₁-C₁₀-alkyle, aryle, aralkyle, CO₂H, CO₂-alkyle, CH₂OH, CH₂O-alkyle, CH₂O-acyle, CN, CH₂NH₂, CH₂N(alkyl, acyl)_{1,2} ou CH₂Hal ;
Hal est un atome d'halogène ;
R⁶, R⁷ sont, dans chaque cas, hydrogène ou, ensemble, une liaison supplémentaire ou, ensemble, un atome d'oxygène ou, ensemble, un groupement NH ou, ensemble, un groupement N-alkyle ou, ensemble, un groupement CH₂, et
G est un atome d'oxygène ou CH₂ ;
D-E est un groupement H₂C-CH₂, HC=CH, C≡C, CH (OH)-CH(OH), CH(OH)-CH₂, CH₂-CH(OH), 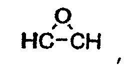 O-CH₂ ou, si G représente un groupement CH₂, D-E est également CH₂-O ;
W est un groupement C(=X)R⁸ ou un radical aromatique ou hétéroaromatique bi- ou tri- cyclique ;
L³ est hydrogène ou, si un radical dans W contient un groupement hydroxy, forme un groupement O-L⁴ avec celui-ci ou, si un radical dans W contient un groupement amino, forme un groupement NR²⁵-L⁴ avec celui-ci ;
R²⁵ est hydrogène ou C₁-C₁₀-alkyle ;
X est un atome d'oxygène ou deux groupements OR²⁰, ou un groupement C₂-C₁₀-alkylènedioxy, qui doit être à chaîne droite ou ramifiée, ou H/OR⁹ ou un groupement CR¹⁰R¹¹;
R⁸ est hydrogène, C₁-C₁₀-alkyle, aryle, aralkyle, halogène ou CN, et
R⁹ est hydrogène ou un groupement protecteur PG^{X} ;
R¹⁰ R¹¹ sont, dans chaque cas, indépendamment l'un de l'autre, hydrogène, C₁-C₂₀-alkyle, aryle ou aralkyle ou, ensemble avec un atome de carbone de méthylène, forment un cycle carbocyclique de 5 à 7 chaînons ;
Z peut représenter oxygène ou H/OR¹² ;
R¹² peut représenter hydrogène ou un groupement protecteur PG^{z} ;
A-Y peut représenter un groupement O-C(=O), O-CH₂, CH₂-C(=O), NR²¹-C(=O) ou NR²¹-SO₂ ;
R²⁰ peut représenter C₁-C₂₀-alkyle ;
R²¹ peut représenter un atome d'hydrogène ou C₁-C₁₀-alkyle ;
PG^{x}, PG^{Y} et PG^{z} peuvent représenter un groupement protecteur PG, et
L¹, L², L⁴ peuvent représenter, indépendamment les uns des autres, hydrogène, un groupement C(=O)Cl, un groupement C(=S)Cl, un groupement PG^{Y} ou un lieur de formule générale (III) ;
à condition qu'au moins un substituant L¹, L² ou L⁴ représente un lieur de formule générale (III) ;
le lieur de formule générale (III) a la structure suivante : 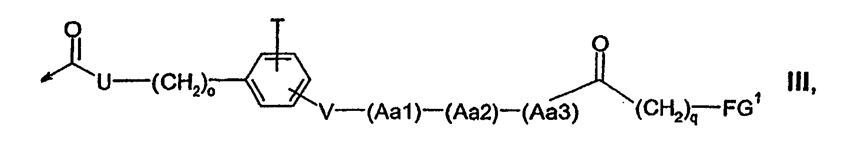 dans laquelle :
T représente halogène ou des groupements OH, O-alkyle, CO₂H CO₂-alkyle, -NHR²³, -NO₂, -N₃, -CN, C₁-C₂₀-alkyle, C₁-C₂₀-acyle ou C₁-C₂₀-acyloxy ;
U représente une liaison, oxygène, ou NR^{24a} ;
o représente de 0 à 5 ;
V représente oxygène ou NR^{24b};
Aa1 représente une liaison ou un groupement de formule générale IV, et
Aa2, Aa3 représentent, indépendamment l'un de l'autre, un groupement de formule générale IV : 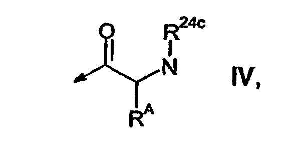 qui est issu d'un acide aminé naturel ou non naturel HO-Aa1-H, HO-Aa2-H ou HO-Aa3-H de formule générale IV' : 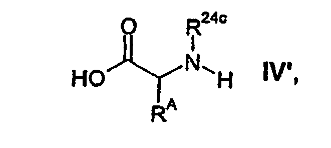 dans laquelle R^{A} peut être identique ou différent dans HO-Aa1-H, HO-Aa2-H ou HO-Aa3-H, et le substituant Aa représente un acide aminé naturel ou non naturel ;
R²² peut représenter hydrogène, C₁-C₁₀-alkyle, aryle ou aralkyle ;
R²³ peut représenter hydrogène, C₁-C₁₀-alkyle ou C₁-C₁₀-acyle ;
R^{24a}, R^{24b}, R^{24c} peuvent représenter, indépendamment les uns des autres, hydrogène ou C₁-C₁₀-alkyle ;
q peut représenter de 1 à 20 ;
FG¹ peut représenter C₁-C₁₀-alkyl-S₃, 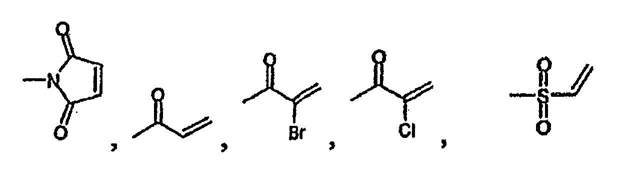 ou CO₂H ;
sous forme d'un isomère uniforme ou d'un mélange de différents isomères et/ou sous forme d'un sel pharmaceutiquement acceptable de celui-ci.

2. Conjugué d'effecteurs selon la revendication 1, **caractérisé en ce que** :
A-Y représente O-C(=O) ou NR²¹-C(=O) ;
D-E représente un groupement H₂C-CH₂ ou un groupement HC=CH ;
G représente un groupement CH₂ ;
Z représente un atome d'oxygène ;
R^{1a}, R^{1b} représentent, dans chaque cas, C₁-C₁₀-alkyle ou, ensemble, un groupement -(CH2)ₚ, p étant égal à 2, 3 ou 4 ;
R^{2a}, R^{2b} représentent, indépendamment l'un de l'autre, hydrogène, C₁-C₁₀-alkyle, C₂-C₁₀-alcényle ou C₂-C₁₀-alcinyle ;
R³ représente hydrogène ;
R^{4a}, R^{4b} représentent, indépendamment l'un de l'autre, hydrogène ou C₁-C₁₀-alkyle ;
R⁵ représente hydrogène ou C₁-C₄-alkyle, ou CH₂OH ou CH₂NH₂, ou CH₂N (alkyl, acyl)_{1,2} ou CH₂Hal ;
R⁶ et R⁷ représentent, ensemble, une liaison supplémentaire ou, ensemble, un groupement NH ou, ensemble, un groupement N-alkyle ou, ensemble, un groupement CH₂ ou, ensemble, un groupement d'oxygène,
W représente un groupement C(=X)R⁸ ou un radical 2-méthylbenzothiazol-5-yle ou un radical 2-méthylbenzoxazol-5-yle ou un radical quinoléin-7-yle ou un radical 2-aminométhylbenzothiazol-5-yle ou un radical 2-hydroxyméthylbenzothiazol-5-yle ou un radical 2-aminométhylbenzoxazol-5-yle ou un radical 2-hydroxyméthylbenzoxazol-5-yle ;
X représente un groupement CR¹⁰R¹¹;
R⁸ représente hydrogène ou C₁-C₄-alkyle ou un atome de fluor ou un atome de chlore ou un atome de brome ;
R¹⁰/R¹¹ représente hydrogène/2-méthylthiazol-4-yle ou hydrogène/2-pyridyle ou hydrogène/2-méthyloxazol-4-yle ou hydrogène/2-amino-méthylthiazol-4-yle ou hydrogène/2-amino-méthyloxazol-4-yle ou hydrogène/2-hydroxy-méthylthiazol-4-yle ou hydrogène/2-hydroxy-méthyloxazol-4-yle.

3. Conjugué d'effecteurs selon la revendication 1 ou 2, **caractérisé en ce que** l'élément effecteur est choisi dans le groupe constitué de :
(4S,7R,8S,9S,13Z,16S(E))-4,8-dihydroxy-5,5,7,9,13-pentaméthyl-16-[1-méthyl-2-(2-méthylthiazol-4-yl)-vinyl]-oxacyclohexadéc-13-ène-2,6-dione ;
(4S,7R,8S,9S,13Z,16S(E))-4,8-dihydroxy-16-[2-(2-hydroxyméthylthiazol-4-yl)-1-méthylvinyl]-5,5,7,9,13-pentaméthyl-oxacyclohexadéc-13-ène-2,6-dione ;
(4S,7R,8S,9S,13Z,16S(E))-16-[2-(2-aminométhylthiazol-4-yl)-1-méthylvinyl]-4,8-dihydroxy-5,5,7,9,13-pentaméthyl-oxacyclohexadéc-13-ène-2,6-dione ;
(1S,3S(E),7S,10R,11S,12S,16R)-7,11-dihydroxy-8,8,10,12,16-pentaméthyl-3-[1-méthyl-2-(2-méthylthiazol-4-yl)-vinyl]-4,17-dioxabicyclo[14,1,0]-heptadécane-5,9-dione ;
(1S,3S(E),7S,10R,11S,12S,16R)-7,11-dihydroxy-3-[2-(2-hydroxyméthylthiazol-4-yl)-1-méthylvinyl]-8,8,10,12,16-pentaméthyl-4,17-dioxabicyclo-[14,1,0]heptadécane-5,9-dione ;
(1S, 3S(E), 7S, 10R, 11S, 12S, 16R)-3-[2-(2-aminométhylthiazol-4-yl)-1-méthylvinyl]-7,11-dihydroxy-8,8,10,12,16-pentaméthyl-4,17-dioxabicyclo-[14,1,0]heptadécane-5,9-dione ;
(4S,7R,8S,9S,13Z,16S(E))-4,8-dihydroxy-7-éthyl-5,5,9,13-tétraméthyl-16-[1-méthyl-2-(2-méthylthiazol-4-yl)-vinyl]-oxacyclohexadéc-13-ène-2,6-dione ;
(4S, 7R, 8S, 9S, 13Z, 16S(E))-4,8-dihydroxy-16-[2-(2-hydroxyméthylthiazol-4-yl)-1-méthylvinyl]-7-éthyl-5,5,9,13-tétraméthyl-oxacyclohexadéc-13-ène-2,6-dione ;
(4S, 7R, 8S, 9S, 13Z, 16S(E))-16-[2-(2-aminométhylthiazol-4-yl)-1-méthylvinyl]-4,8-dihydroxy-7-éthyl-5,5,9,13-tétraméthyl-oxacyclohexadéc-13-ène-2,6-dione ;
(1S,3S(E),7S,10R,11S,12S,16R)-7,11-dihydroxy-10-éthyl-8,8,12,16-tétraméthyl-3-[1-méthyl-2-(2-méthylthiazol-4-yl)-vinyl]-4,17-dioxabicyclo[14,1,0]-heptadécane-5,9-dione ;
(1S,3S(E),7S,10R,11S,12S,16R)-7,11-dihydroxy-3-[2-(2-hydroxyméthylthiazol-4-yl)-1-méthylvinyl]-10-éthyl-8,8,12,16-tétraméthyl-4,17-dioxabicyclo[14,1,0]-heptadécane-5,9-dione ;
(1S,3S(E),7S,10R,11S,12S,16R)-3-[2-(2-aminométhylthiazol-4-yl)-1-méthylvinyl]-7,11-dihydroxy-10-éthyl-8,8,12,16-tétraméthyl-4,17-dioxabicyclo[14,1,0]-heptadécane-5,9-dione ;
(4S,7R,8S,9S,13Z,16S(Z))-4,8-dihydroxy-5,5,7,9,13-pentaméthyl-16-[1-fluoro-2-(2-méthylthiazol-4-yl)-vinyl]-oxacyclohexadéc-13-ène-2,6-dione ;
(4S,7R,8S,9S,13Z,16S(Z))-4,8-dihydroxy-16-[2-(2-hydroxyméthylthiazol-4-yl)-1-fluorovinyl]-5,5,7,9,13-pentaméthyl-oxacyclohexadéc-13-ène-2,6-dione ;
(4S, 7R, 8S, 9S, 13Z, 16S (Z))-16-[2- (2-aminométhylthiazol-4-yl)-1-fluorovinyl]-4,8-dihydroxy-5,5,7,9,13-pentaméthyl-oxacyclohexadéc-13-ène-2,6-dione ;
(1S,3S(Z),7S,10R,11S,12S,16R)-7,11-dihydroxy-8,8,10,12,16-pentaméthyl-3-[1-fluoro-2-(2-méthylthiazol-4-yl)-vinyl]-4,17-dioxabicyclo[14,1,O]-heptadécane-5,9-dione ;
(1S,3S(Z),7S,10R,11S,12S,16R)-7,11-dihydroxy-3-[2-(2-hydroxyméthylthiazol-4-yl)-1-fluorovinyl]-8,8,10,12,16-pentaméthyl-4,17-dioxabicyclo[14,1,0]-heptadécane-5,9-dione ;
(1S,3S(Z),7S,10R,11S,12S,16R)-3-[2-(2-aminométhylthiazol-4-yl)-1-fluorovinyl]-7,11-dihydroxy-8,8,10,12,16-pentaméthyl-4,17-dioxabicyclo[14,1,0]-heptadécane-5,9-dione ;
(4S,7R,8S,9S,13Z,16S(Z))-4,8-dihydroxy-5,5,7,9,13-pentaméthyl-16-[1-chloro-2-(2-méthylthiazol-4-yl)-vinyl]-oxacyclohexadéc-13-ène-2,6-dione ;
(4S,7R,8S,9S,13Z,16S(Z))-4,8-dihydroxy-16-[2-(2-hydroxyméthylthiazol-4-yl)-1-chlorovinyl]-5,5,7,9,13-pentaméthyl-oxacyclohexadéc-13-ène-2,6-dione ;
(4S,7R,8S,9S,13Z,16S(Z))-16-[2-(2-aminométhylthiazol-4-yl)-1-chlorovinyl]-4,8-dihydroxy-5,5,7,9,13-pentaméthyl-oxacyclohexadéc-13-ène-2,6-dione ;
(1S, 3S (Z), 7S, 10R, 11S, 12S, 16R)-7,11-dihydroxy-8, 8, 10, 12, 16-pentaméthyl-3-[1-chloro-2-(2-méthylthiazol-4-yl)-vinyl]-4,17-dioxabicyclo[14,1,0]heptadécane-5,9-dione ;
(1S, 3S(Z), 7S, 10R, 11S, 12S, 16R)-7,11-dihydroxy-3-[2-(2-hydroxyméthylthiazol-4-yl)-1-chlorovinyl]-8,8,10,12,16-pentaméthyl-4,17-dioxabicyclo[14,1,0]-heptadécane-5,9-dione ;
(1S,3S(Z),7S,10R,11S,12S,16R)-3-[2-(2-aminométhylthiazol-4-yl)-1-chlorovinyl]-7,11-dihydroxy-8,8,10,12,16-pentaméthyl-4,17-dioxabicyclo[14,1,0]-heptadécane-5,9-dione ;
(4S,7R,8S,9S,13Z,16S(Z))-4,8-dihydroxy-7-éthyl-5,5,9,13-tétraméthyl-16-[1-fluoro-2-(2-méthylthiazol-4-yl)-vinyl]-oxacyclohexadéc-13-ène-2,6-dione ;
(4S,7R,8S,9S,132,165(Z))-4,8-dihydroxy-16-[2-(2-hydroxyméthylthiazol-4-yl)-1-fluorovinyl]-7-éthyl-5,5,9,13-tétraméthyl-oxacyclohexadéc-13-ène-2,6-dione ;
(4S,7R,8S,9S,13Z,16S(Z))-16-[2-(2-aminométhylthiazol-4-yl)-1-fluorovinyl]-4,8-dihydroxy-7-éthyl-5,5,9,13-tétraméthyl-oxacyclohexadéc-13-ène-2,6-dione ;
(1S,3S(Z),7S,10R,11S,12S,16R)-7,11-dihydroxy-10-éthyl-8,8,12,16-tétraméthyl-3-[1-fluoro-2-(2-méthylthiazol-4-yl)-vinyl]-4,17-dioxabicyclo[14,1,0]-heptadécane-5,9-dione ;
(1S,3S(Z),7S,10R,11S,12S,16R)-7,11-dihydroxy-3-[2-(2-hydroxyméthylthiazol-4-yl)-1-fluorovinyl]-10-éthyl-8,8,12,16-tétraméthyl-4,17-dioxabicyclo[14,1,0]-heptadécane-5,9-dione ;
(1S,3S(Z),7S,10R,11S,12S,16R)-3-[2-(2-aminométhylthiazol-4-yl)-1-fluorovinyl]-7,11-dihydroxy-10-éthyl-8,8,12,16-tétraméthyl-4,17-dioxabicyclo[14,1,0]-heptadécane-5,9-dione;
(4S,7R,8S,9S,13Z,16S(Z))-4,8-dihydroxy-7-éthyl-5,5,9,13-tétraméthyl-16-[1-chloro-2-(2-méthylthiazol-4-yl)-vinyl]-oxacyclohexadéc-13-ène-2,6-dione ;
(4S,7R,8S,9S,13Z,16S(Z))-4,8-dihydroxy-16-[2-(2-hydroxyméthylthiazol-4-yl)-1-chlorovinyl]-7-éthyl-5,5,9,13-tétraméthyl-oxacyclohexadéc-13-ène-2,6-dione ;
(4S,7R,8S,9S,13Z,16S(Z))-16-[2-(2-aminométhylthiazol-4-yl)-1-chlorovinyl]-4,8-dihydroxy-7-éthyl-5,5,9,13-tétraméthyl-oxacyclohexadéc-13-ène-2,6-dione ;
(1S,3S(Z),7S,10R,11S,12S,16R)-7,11-dihydroxy-10-éthyl-8,8,12,16-tétraméthyl-3-[1-chloro-2-(2-méthylthiazol-4-yl)-vinyl]-4,17-dioxabicyclo[14,1,0]-heptadécane-5,9-dione ;
(1S,3S(Z),7S,10R,11S,12S,16R)-7,11-dihydroxy-3-[2-(2-hydroxyméthylthiazol-4-yl)-1-chlorovinyl]-10-éthyl-8,8,12,16-tétraméthyl-4,17-dioxabicyclo(14.1.0]-heptadécane-5,9-dione ;
(1S,3S(Z),7S,10R,11S,12S,16R)-3-[2-(2-aminométhylthiazol-4-yl)-1-chlorovinyl]-7,11-dihydroxy-10-éthyl-8,8,12,16-tétraméthyl-4,17-dioxabicyclo[14,1,0]-heptadécane-5,9-dione ;
(4S,7R,8S,9S,13Z,16S(E))-4,8-dihydroxy-5,5,7,9,13-pentaméthyl-16-[1-méthyl-2-(2-pyridyl)-vinyl]-oxacyclohexadéc-13-ène-2,6-dione ;
(1S,3S(E),7S,10R,11S,12S,16R)-7,11-dihydroxy-8,8,10,12,16-pentaméthyl-3-[1-méthyl-2-(2-pyridyl)-vinyl]-4,17-dioxabicyclo[14,1,0]-heptadécane-5,9-dione ;
(4S,7R,8S,9S,13Z,16S(E))-4,8-dihydroxy-7-éthyl-5,5,9,13-tétraméthyl-16-[1-méthyl-2-(2-pyridyl)-vinyl]-oxacyclohexadéc-13-ène-2,6-dione ;
(1S,3S(E),7S,10R,11S,12S,16R)-7,11-dihydroxy-10-éthyl-8,8,12,16-tétraméthyl-3-[1-méthyl-2-(2-pyridyl)-vinyl]-4,17-dioxabicyclo[14,1,0]-heptadécane-5,9-dione ;
(4S,7R,8S,9S,13Z,16S(Z))-4,8-dihydroxy-5,5,7,9,13-pentaméthyl-16-[1-fluoro-2-(2-pyridyl)-vinyl]-oxacyclohexadéc-13-ène-2,6-dione ;
(1S,3S(Z),7S,10R,11S,12S,16R)-7,11-dihydroxy-8,8,10,12,16-pentaméthyl-3-[1-fluoro-2-(2-pyridyl)-vinyl]-4,17-dioxabicyclo[14.1.0]-heptadécane-5,9-dione ;
(4S,7R,8S,9S,13Z,16S (Z))-4,8-dihydroxy-5,5,7,9,13-pentaméthyl-16-[1-chloro-2-(2-pyridyl)-vinyl]-oxacyclohexadéc-13-ène-2,6-dione ;
(1*S*,3*S* (Z), 7*S*,10R,11S,12*S*, 16R)-7,11-dihydroxy-8,8,10,12,16-pentaméthyl-3-[1-chloro-2-(2-pyridyl)-vinyl]-4,17-dioxabicyclo[14,1,0]-heptadécane-5,9-dione ;
(4S,7R,8S,9S,13Z,16S(Z))-4,8-dihydroxy-7-éthyl-5,5,9,13-tétraméthyl-16-[1-fluoro-2-(2-pyridyl)-vinyl]-oxacyclohexadéc-13-ène-2,6-dione ;
(1S,3S(Z),7S,10R,11S,12S,16R)-7,11-dihydroxy-10-éthyl-8,8,12,16-tétraméthyl-3-[1-fluoro-2-(2-pyridyl)-vinyl]-4,17-dioxabicyclo[14,1,0]-heptadécane-5,9-dione ;
(4S, 7R, 8S, 9S, 13Z, 16S(Z))-4,8-dihydroxy-7-éthyl-5,5,9,13-tétraméthyl-16-[1-chloro-2-(2-pyridyl)-vinyl]-oxacyclohexadéc-13-ène-2,6-dione ;
(1S,3S(Z),7S,10R,11S,12S,16R)-7,11-dihydroxy-10-éthyl-8,8,12,16-tétraméthyl-3-[1-chloro-2-(2-pyridyl)-vinyl]-4,17-dioxabicyclo[14,1,0]-heptadécane-5,9-dione ;
(4S,7R,8S,9S,13Z,16S(E))-4,8-dihydroxy-5,5,7;9,13-pentaméthyl-16-[1-méthyl-2-(2-méthyloxazol-4-yl)-vinyl]-oxacyclohexadéc-13-ène-2,6-dione ;
(4S,7R,8S,9S,13Z,16S(E))-4,8-dihydroxy-16-[2-(2-hydroxyméthyloxazol-4-yl)-1-méthylvinyl]-5,5,7,9,13-pentaméthyl-oxacyclohexadéc-13-ène-2,6-dione ;
(4S,7R,8S,9S,13Z,16S(E))-16-[2-(2-aminométhyloxazol-4-yl)-1-méthylvinyl]-4,8-dihydroxy-5, 5, 7, 9,13-pentaméthyl-oxacyclohexadéc-13-ène-2,6-dione ;
(1S,3S(E),7S,10R,11S,12S,16R)-7,11-dihydroxy-8,8,10,12,16-pentaméthyl-3-[1-méthyl-2-(2-méthyloxazol-4-yl)-vinyl]-4,17-dioxabicyclo [14,1,0]-heptadécane-5,9-dione ;
(1S, 3S (E), 7S, 10R, 11S, 12S, 16R)-7,11-dihydroxy-3-[2-(2-hydroxyméthyloxazol-4-yl)-1-méthylvinyl]-8,8,10,12,16-pentaméthyl-4,17-dioxabicyclo [14,1,0]-heptadécane-5,9-dione ;
(1S,3S(E),7S,10R,11S,12S,16R)-3-[2-(2-aminométhyloxazol-4-yl)-1-méthylvinyl]-7,11-dihydroxy-8,8,10,12,16-pentaméthyl-4,17-dioxabicyclo[14,1,0]-heptadécane-5,9-dione ;
(4S,7R,8S,9S,13Z,16S(E))-4,8-dihydroxy-7-éthyl-5,5,9,13-tétraméthyl-16-[1-méthyl-2-(2-méthyloxazol-4-yl)-vinyl]-oxacyclohexadéc-13-ène-2,6-dione ;
(4S,7R,8S,9S,13Z,16S(E))-4,8-dihydroxy-16-[2-(2-hydroxyméthyloxazol-4-yl)-1-méthylvinyl]-7-éthyl-5,5,9,13-tétraméthyl-oxacyclohexadéc-13-ène-2,6-dione ;
(4S,7R,8S,9S,13Z,16S(E))-16- [2- (2-aminométhyloxazol-4-yl)-1-méthylvinyl]-4,8-dihydroxy-7-éthyl-5,5,9,13-tétraméthyl-oxacyclohexadéc-13-ène-2,6-dione ;
(1S,3S(E),7S,10R,11S,12S,16R)-7,11-dihydroxy-10-éthyl-8,8,12,16-tétraméthyl-3-[1-méthyl-2-(2-méthyloxazol-4-yl)-vinyl]-4,17-dioxabicyclo[14,1,0]-heptadécane-5,9-dione ;
(1S,3S(E),7S,10R,11S,12S,16R)-7,11-dihydroxy-3-[2-(2-hydroxyméthyloxazol-4-yl)-1-méthylvinyl]-10-éthyl-8,8,12,16-tétraméthyl-4,17-dioxabicyclo[14,1,0]-heptadécane-5,9-dione ;
(1S,3S(E),7S,10R,11S,12S,16R)-3-[2-(2-aminométhyloxazol-4-yl)-1-méthylvinyl]-7,11-dihydroxy-10-éthyl-8,8,12,16-tétraméthyl-4,17-dioxabicyclo[14,1,0]-heptadécane-5,9-dione ;
(4S,7R,8S,9S,13Z,16S(Z))-4,8-dihydroxy-5,5,7,9,13-pentaméthyl-16-[1-fluoro-2-(2-méthyloxazol-4-yl)-vinyl]-oxacyclohexadéc-13-ène-2,6-dione ;
(4S,7R,8S,9S,13Z,16S(Z))-4,8-dihydroxy-16-[2-(2-hydroxyméthyloxazol-4-yl)-1-fluorovinyl]-5,5,7,9,13-pentaméthyl-oxacyclohexadéc-13-ène-2,6-dione ;
(4S,7R,8S,9S,13Z,16S(Z))-16-[2-(2-aminométhyloxazol-4-yl)-1-fluorovinyl]-4,8-dihydroxy-5,5,7,9,13-pentaméthyl-oxacyclohexadéc-13-ène-2,6-dione ;
(1S,3S(Z),7S,10R,11S,12S,16R)-7,11-dihydroxy-8,8,10,12,16-pentaméthyl-3-[1-fluoro-2-(2-méthyloxazol-4-yl)-vinyl]-4,17-dioxabicyclo[14,1,0]-heptadécane-5,9-dione ;
(1S,3S(Z),7S,10R,11S,12S,16R)-7,11-dihydroxy-3-[2-(2-hydroxyméthyloxazol-4-yl)-1-fluorovinyl]-8,8,10,12,16-pentaméthyl-4,17-dioxabicyclo[14.1.0]-heptadécane-5,9-dione ;
(1S,3S(Z),7S,10R,11S,12S,16R)-3-[2-(2-aminométhyloxazol-4-yl)-1-fluorovinyl]-7,11-dihydroxy-8,8,10,12,16-pentaméthyl-4,17-dioxabicyclo[14,1,0]-heptadécane-5,9-dione ;
(4S,7R,8S,9S,13Z,16S(Z))-4,8-dihydroxy-5,5,7,9,13-pentaméthyl-16-[1-chloro-2-(2-méthyloxazol-4-yl)-vinyl]-oxacyclohexadéc-13-ène-2,6-dione ;
(4S,7R,8S,9S,13Z,16S(Z))-4,8-dihydroxy-16-[2-(2-hydroxyméthyloxazol-4-yl)-1-chlorovinyl]-5,5,7,9,13-pentaméthyl-oxacyclohexadéc-13-ène-2,6-dione ;
(4S,7R,8S,9S,13Z,16S(Z))-16-[2-(2-aminométhyloxazol-4-yl)-1-chlorovinyl]-4,8-dihydroxy-5,5,7,9,13-pentaméthyl-oxacyclohexadéc-13-ène-2,6-dione ;
(1S,3S(Z),7S,10R,11S,12S,16R)-7,11-dihydroxy-8,8,10,12,16-pentaméthyl-3-[1-chloro-2-(2-méthyloxazol-4-yl)-vinyl]-4,17-dioxabicyclo[14,1,0]-heptadécane-5,9-dione ;
(1S,3S(Z),7S,10R,11S,12S,16R)-7,11-dihydroxy-3-[2-(2-hydroxyméthyloxazol-4-yl)-1-chlorovinyl]-8,8,10,12,16-pentaméthyl-4,17-dioxabicyclo[14, 1, 0]-heptadécane-5,9-dione ;
(1S,3S(Z),7S,10R,11S,12S,16R)-3-[2-(2-aminométhyloxazol-4-yl)-1-chlorovinyl]-7,11-dihydroxy-8,8,10,12,16-pentaméthyl-4,17-dioxabicyclo[14,1,0]-heptadécane-5,9-dione ;
(4S,7R,8S,9S,13Z,16S(Z))-4,8-dihydroxy-7-éthyl-5,5,9,13-tétraméthyl-16-[1-fluoro-2-(2-méthyloxazol-4-yl)-vinyl]-oxacyclohexadéc-13-ène-2,6-dione ;
(4S,7R,8S,9S,13Z,16S(Z))-4,8-dihydroxy-16-[2-(2-hydroxyméthyloxazol-4-yl)-1-fluorovinyl]-7-éthyl-5,5,9,13-tétraméthyl-oxacyclohexadéc-13-ène-2,6-dione ;
(4S,7R,8S,9S,13Z,16S(Z))-16-[2-(2-aminométhyloxazol-4-yl)-1-fluorovinyl]-4,8-dihydroxy-7-éthyl-5,5,9,13-tétraméthyl-oxacyclohexadéc-13-ène-2,6-dione ;
(1S, 3S(Z), 7S, 10R, 11S, 12S, 16R)-7,11-dihydroxy-10-éthyl-8,8,12,16-tétraméthyl-3-[1-fluoro-2-(2-méthyloxazol-4-yl)-vinyl]-4,17-dioxabicyclo[14.1.0]-heptadécane-5,9-dione ;
(1S,3S(Z),7S,10R,11S,12S,16R)-7,11-dihydroxy-3-[2-(2-hydroxyméthyloxazol-4-yl)-1-fluorovinyl]-10-éthyl-8,8,12,16-tétraméthyl-4,17-dioxabicyclo[14,1,0]-heptadécane-5,9-dione ;
(1S,3S(Z),7S,10R,11S,12S,16R)-3-[2-(2-aminométhyloxazol-4-yl)-1-fluorovinyl]-7,11-dihydroxy-10-éthyl-8,8,12,16-tétraméthyl-4,17-dioxabicyclo[14,1,0]-heptadécane-5,9-dione ;
(4S,7R,8S,9S,13Z,16S(Z))-4,8-dihydroxy-7-éthyl-5,5,9,13-tétraméthyl-16-[1-chloro-2-(2-méthyloxazol-4-yl)-vinyl]-oxacyclohexadéc-13-ène-2,6-dione ;
(4S,7R,8S,9S,13Z,16S(Z))-4,8-dihydroxy-16-[2-(2-hydroxyméthyloxazol-4-yl)-1-chlorovinyl]-7-éthyl-5,5,9,13-tétraméthyl-oxacyclohexadéc-13-ène-2,6-dione ;
(4S,7R,8S,9S,13Z,16S(Z))-16-[2-(2-aminométhyloxazol-4-yl)-1-chlorovinyl]-4,8-dihydroxy-7-éthyl-5,5,9,13-tétraméthyl-oxacyclohexadéc-13-ène-2,6-dione ;
(1S,3S(Z),7S,10R,11S,12S,16R)-7,11-dihydroxy-10-éthyl-8,8,12,16-tétraméthyl-3-[1-chloro-2-(2-méthyloxazol-4-yl)-vinyl]-4,17-dioxabicyclo[14,1,0]-heptadécane-5,9-dione ;
(1S,3S(Z),7S,10R,11S,12S,16R)-7,11-dihydroxy-3-[2-(2-hydroxyméthyloxazol-4-yl)-1-chlorovinyl]-10-éthyl-8,8,12,16-tétraméthyl-4,17-dioxabicyclo[14,1,0]-heptadécane-5,9-dione ;
(1S,3S(Z),7S,10R,11S,12S,16R)-3-[2-(2-aminométhyloxazol-4-yl)-1-chlorovinyl]-7,11-dihydroxy-10-éthyl-8,8,12,16-tétraméthyl-4,17-dioxabicyclo[14,1,0]-heptadécane-5,9-dione ;
(4S,7R,8S,9S,13Z,16S(E))-4,8-dihydroxy-5,5,7,9,13-pentaméthyl-16-[2-(2-méthylthiazol-4-yl)-vinyl]-oxacyclohexadéc-13-ène-2,6-dione ;
(4S,7R,8S,9S,13Z,16S(E))-4,8-dihydroxy-16-[2-(2-hydroxyméthylthiazol-4-yl)-vinyl]-5,5,7,9,13-pentaméthyl-oxacyclohexadéc-13-ène-2,6-dione ;
(4S,7R,8S,9S,13Z,16S(E))-16-[2-(2-aminométhylthiazol-4-yl)-vinyl]-4,8-dihydroxy-5,5,7,9,13-pentaméthyl-oxacyclohexadéc-13-ène-2,6-dione ;
(1S,3S(E),7S,10R,11S,12S,16R)-7,11-dihydroxy-8,8,10,12,16-pentaméthyl-3-[2-(2-méthylthiazol-4-yl)-vinyl]-4,17-dioxabicyclo[14,1,0]heptadécane-5,9-dione ;
(1S,3S(E),7S,10R,11S,12S,16R)-7,11-dihydroxy-3-[2-(2-hydroxyméthylthiazol-4-yl)-vinyl]-8,8,10,12,16-pentaméthyl-4,17-dioxabicyclo[14,1,0]-heptadécane-5,9-dione ;
(1S,3S(E),7S,10R,11S,12S,16R)-3-[2-(2-aminométhylthiazol-4-yl)-vinyl]-7,11-dihydroxy-8,8,10,12,16-pentaméthyl-4,17-dioxabicyclo[14,1,0]heptadécane-5,9-dione ;
(4S,7R,8S,9S,13Z,16S(E))-4,8-dihydroxy-7-éthyl-5,5,9,13-tétraméthyl-16-[2-(2-méthylthiazol-4-yl)-vinyl]-oxacyclohexadéc-13-ène-2,6-dione ;
(4S,7R,8S,9S,13Z,16S(E))-4,8-dihydroxy-16-[2-(2-hydroxyméthylthiazol-4-yl)-vinyl]-7-éthyl-5,5,9,13-tétraméthyl-oxacyclohexadéc-13-ène-2,6-dione ;
(4S, 7R, 8S, 9S, 13Z, 16S(E))- 16-[2-(2-aminométhylthiazol-4-yl)-vinyl]-4,8-dihydroxy-7-éthyl-5,5,9,13-tétraméthyl-oxacyclohexadéc-13-ène-2,6-dione ;
(1S, 3S(E), 7S, 10R, 11S, 12S, 16R)-7,11-dihydroxy-10-éthyl-8,8,12,16-tétraméthyl-3-[2-(2-méthylthiazol-4-yl)-vinyl]-4,17-dioxabicyclo[14,1,0]-heptadécane-5,9-dione ;
(1S,3S(E),7S,10R,11S,12S,16R)-7,11-dihydroxy-3-[2-(2-hydroxyméthylthiazol-4-yl)-vinyl]-10-éthyl-8,8,12,16-tétraméthyl-4,17-dioxabicyclo[14,1,0]-heptadécane-5,9-dione ;
(1S,3S(E),7S,10R,11S,12S,16R)-3-[2-(2-aminométhylthiazol-4-yl)-vinyl]-7,11-dihydroxy-10-éthyl-8,8,12,16-tétraméthyl-4,17-dioxabicyclo[14,1,0]-heptadécane-5,9-dione ;
(4S,7R,8S,9S,13Z,16S(E))-4,8-dihydroxy-5,5,7,9,13-pentaméthyl-16-[2-(2-pyridyl)-vinyl]-oxacyclohexadéc-13-ène-2,6-dione ;
(1S,3S(E),7S,10R,11S,12S,16R)-7,11-dihydroxy-8,8,10,12,16-pentaméthyl-3-[2-(2-pyridyl)-vinyl]-4,17-dioxabicyclo[14,1,0]heptadécane-5,9-dione ;
(4S,7R,8S,9S,13Z,16S(E))-4,8-dihydroxy-7-éthyl-5,5,9,13-tétraméthyl-16-[2-(2-pyridyl)-vinyl]-oxacyclohexadéc-13-ène-2,6-dione ;
(1S,3S(E),7S,10R,11S,12S,16R)-7,11-dihydroxy-10-éthyl-8,8,12,16-tétraméthyl-3-[2-(2-pyridyl)-vinyl]-4,17-dioxabicyclo[14,1,0]heptadécane-5,9-dione ;
(4S,7R,8S,9S,13Z,16S)-4,8-dihydroxy-5,5,7,9,13-pentaméthyl-16-(2-méthylbenzothiazol-5-yl)-oxacyclohexadéc-13-ène-2,6-dione ;
(4S,7R,8S,9S,13Z,16S)-4,8-dihydroxy-16-(2-hydroxyméthylbenzothiazol-5-yl)-5,5,7,9,13-pentaméthyl-oxacyclohexadéc-13-ène-2,6-dione ;
(4S, 7R, 8S, 9S, 13Z, 16S)-16-(2-aminométhylbenzothiazol-5-yl)-4,8-dihydroxy-5,5,7,9,13-pentaméthyl-oxacyclohexadéc-13-ène-2,6-dione ;
(1S, 3S, 7S, 10R, 11S, 12S, 16R)-7,11-dihydroxy-8,8,10,12,16-pentaméthyl-3-(2-méthylbenzothiazol-5-yl)-4,17-dioxabicyclo[14,1,0]heptadécane-5,9-dione ;
(1S,3S,7S,10R,11S,12S,16R)-7,11-dihydroxy-3-(2-hydroxyméthylbenzothiazol-5-yl)-8,8,10,12,16-pentaméthyl-4,17-dioxabicyclo[14,1,0]heptadécane-5,9-dione ;
(1S,3S,7S,10R,11S,12S,16R)-3-(2-aminométhylbenzothiazol-5-yl)-7,11-dihydroxy-8,8,10,12,16-pentaméthyl-4,17-dioxabicyclo[14,1,0]heptadécane-5,9-dione ;
(4S,7R,8S,9S,13Z,16S)-4,8-dihydroxy-7-éthyl-5,5,9,13-tétraméthyl-16-(2-méthylbenzothiazol-5-yl)-oxacyclohexadéc-13-ène-2,6-dione ;
(4S,7R,8S,9S,13Z,16S)-4,8-dihydroxy-16-(2-hydroxyméthylbenzothiazol-5-yl)-7-éthyl-5,5,9,13-tétraméthyl-oxacyclohexadéc-13-ène-2,6-dione ;
(4S,7R,8S,9S,13Z,16S)-16-(2-aminométhylbenzothiazol-5-yl)-4,8-dihydroxy-7-éthyl-5,5,9,13-tétraméthyl-oxacyclohexadéc-13-ène-2,6-dione ;
(1S,3S,7S,10R,11S,12S,16R)-7,11-dihydroxy-10-éthyl-8,8,12,16-tétraméthyl-3-(2-méthylbenzothiazol-5-yl)-4,17-dioxabicyclo[14,1,0]-heptadécane-5,9-dione ;
(1S,3S,7S,10R,11S,12S,16R)-7,11-dihydroxy-3-(2-hydroxyméthylbenzothiazol-5-yl)-10-éthyl-8,8,12,16-tétraméthyl-4,17-dioxabicyclo[14,1,0]-heptadécane-5,9-dione ;
(1S,3S,7S,10R,11S,12S,16R)-3-(2-aminométhylbenzothiazol-5-yl)-7,11-dihydroxy-10-éthyl-8,8,12,16-tétraméthyl-4,17-dioxabicyclo[14,1,0]-heptadécane-5,9-dione ;
(4S,7R,8S,9S,13Z,16S)-4,8-dihydroxy-7-propyl-5,5,9,13-tétraméthyl-16-(2-méthylbenzothiazol-5-yl)-oxacyclohexadéc-13-ène-2,6-dione ;
(4S,7R,8S,9S,13Z,16S)-4,8-dihydroxy-16-(2-hydroxyméthylbenzothiazol-5-yl)-7-propyl-5,5,9,13-tétraméthyl-oxacyclohexadéc-13-ène-2,6-dione ;
(4S,7R,8S,9S,13Z,16S)-16-(2-aminométhylbenzothiazol-5-yl)-4,8-dihydroxy-7-propyl-5,5,9,13-tétraméthyl-oxacyclohexadéc-13-ène-2,6-dione ;
(1S,3S,7S,10R,11S,12S,16R)-7,11-dihydroxy-10-propyl-8,8,12,16-tétraméthyl-3-(2-méthylbenzothiazol-5-yl)-4,17-dioxabicyclo[14,1,0]-heptadécane-5,9-dione ;
(1S,3S,7S,10R,11S,12S,16R)-7,11-dihydroxy-3-(2-hydroxyméthylbenzothiazol-5-yl)-10-propyl-8,8,12,16-tétraméthyl-4,17-dioxabicyclo[14,1,0]-heptadécane-5,9-dione ;
(1S,3S,7S,10R,11S,12S,16R)-3-(2-aminométhylbenzothiazol-5-yl)-7,11-dihydroxy-10-propyl-8,8,12,16-tétraméthyl-4,17-dioxabicyclo[14,1,0]-heptadécane-5,9-dione ;
(4S,7R,8S,9S,13Z,16S)-4,8-dihydroxy-7-butyl-5,5,9,13-tétraméthyl-16-(2-méthylbenzothiazol-5-yl)-oxacyclohexadéc-13-ène-2,6-dione ;
(4S,7R,8S,9S,13Z,16S)-4,8-dihydroxy-16-(2-hydroxyméthylbenzothiazol-5-yl)-7-butyl-5,5,9,13-tétraméthyl-oxacyclohexadéc-13-ène-2,6-dione ;
(4S,7R,8S,9S,13Z,16S)-16-(2-aminométhylbenzothiazol-5-yl)-4,8-dihydroxy-7-butyl-5,5,9,13-tétraméthyl-oxacyclohexadéc-13-ène-2,6-dione ;
(1S,3S,7S,10R,11S,12S,16R)-7,11-dihydroxy-10-butyl-8,8,12,16-tétraméthyl-3-(2-méthylbenzothiazol-5-yl)-4,17-dioxabicyclo[14,1,0]-heptadécane-5,9-dione ;
(15,3S,7S,10R,11S,12S,16R)-7,11-dihydroxy-3-(2-hydroxyméthylbenzothiazol-5-yl)-10-butyl-8,8,12,16-tétraméthyl-4,17-dioxabicyclo[14,1,0]-heptadécane-5,9-dione ;
(1S,3S,7S,10R,11S,12S,16R)-3-(2-aminométhylbenzothiazol-5-yl)-7,11-dihydroxy-10-butyl-8,8,12,16-tétraméthyl-4,17-dioxabicyclo[14,1,0]-heptadécane-5,9-dione ;
(4S,7R,8S,9S,13Z,16S)-4,8-dihydroxy-7-allyl-5,5,9,13-tétraméthyl-16-(2-méthylbenzothiazol-5-yl)-oxacyclohexadéc-13-ène-2,6-dione ;
(4S,7R,8S,9S,13Z,16S)-4,8-dihydroxy-16-(2-hydroxyméthylbenzothiazol-5-yl)-7-allyl-5,5,9,13-tétraméthyl-oxacyclohexadéc-13-ène-2,6-dione ;
(4S,7R,8S,9S,13Z,16S)-16-(2-aminométhylbenzothiazol-5-yl)-4,8-dihydroxy-7-allyl-5,5,9,13-tétraméthyl-oxacyclohexadéc-13-ène-2,6-dione ;
(1S,3S,7S,10R,11S,12S,16R)-7,11-dihydroxy-10-allyl-8,8,12,16-tétraméthyl-3-(2-méthylbenzothiazol-5-yl)-4,17-dioxabicyclo[14,1,0]-heptadécane-5,9-dione ;
(1S,3S,7S,10R,11S,12S,16R)-7,11-dihydroxy-3-(2-hydroxyméthylbenzothiazol-5-yl)-10-allyl-8,8,12,16-tétraméthyl-4,17-dioxabicyclo[14,1,0]-heptadécane-5,9-dione ;
(1S,3S,7S,10R,11S,12S,16R)-3-(2-aminométhylbenzothiazol-5-yl)-7,11-dihydroxy-10-allyl-8,8,12,16-tétraméthyl-4,17-dioxabicyclo[14,1,0]-heptadécane-5,9-dione ;
(4S,7R,8S,9S,13Z,16S)-4,8-dihydroxy-7-prop-2-inyl-5,5,9,13-tétraméthyl-16-(2-méthylbenzothiazol-5-yl)-oxacyclohexadéc-13-ène-2,6-dione ;
(4S,7R,8S,9S,13Z,16S)-4,8-dihydroxy-16-(2-hydroxyméthylbenzothiazol-5-yl)-7-prop-2-inyl-5,5,9,13-tétraméthyl-oxacyclohexadéc-13-ène-2,6-dione ;
(4S,7R,8S,9S,13Z,16S)-16-(2-aminométhylbenzothiazol-5-yl)-4,8-dihydroxy-7-prop-2-inyl-5,5,9,13-tétraméthyl-oxacyclohexadéc-13-ène-2,6-dione ;
(1S,3S,7S,10R,11S,12S,16R)-7,11-dihydroxy-10-prop-2-inyl-8,8,12,16-tétraméthyl-3-(2-méthylbenzothiazol-5-yl)-4,17-dioxabicyclo[14,1,0]-heptadécane-5,9-dione ;
(1S,3S,7S,10R,11S,12S,16R)-7,11-dihydroxy-3-(2-hydroxyméthylbenzothiazol-5-yl)-10-prop-2-inyl-8,8,12,16-tétraméthyl-4,17-dioxabicyclo[14,1,0]-heptadécane-5,9-dione ;
(1S,3S,7S,10R,11S,12S,16R)-3-(2-aminométhylbenzothiazol-5-yl)-7,11-dihydroxy-10-prop-2-inyl-8,8,12,16-tétraméthyl-4,17-dioxabicyclo [14,1,0]-heptadécane-5,9-dione ;
(4S,7R,8S,9S,13Z,16S)-4,8-dihydroxy-7-but-3-ényl-5,5,9,13-tétraméthyl-16-(2-méthylbenzothiazol-5-yl)-oxacyclohexadéc-13-ène-2,6-dione ;
(4S,7R,8S,9S,13Z,16S)-4,8-dihydroxy-16-(2-hydroxyméthylbenzothiazol-5-yl)-7-but-3-ényl-5,5,9,13-tétraméthyl-oxacyclohexadéc-13-ène-2,6-dione ;
(4S,7R,8S,9S,13Z,16S)-16-(2-aminométhylbenzothiazol-5-yl)-4,8-dihydroxy-7-but-3-ényl-5,5,9,13-tétraméthyl-oxacyclohexadéc-13-ène-2,6-dione ;
(1S, 3S, 7S, 10R, 11S, 12S, 16R)-7, 11-dihydroxy-10-but-3-ényl-8,8,12,16-tétraméthyl-3-(2-méthylbenzothiazol-5-yl)-4,17-dioxabicyclo[14,1,0]-heptadécane-5,9-dione ;
(1S,3S,7S,10R,11S,12S,16R)-7,11-dihydroxy-3-(2-hydroxyméthylbenzothiazol-5-yl)-10-but-3-ényl-8,8,12,16-tétraméthyl-4,17-dioxabicyclo[14,1,0]-heptadécane-5,9-dione ;
(1S,3S,7S,10R,11S,12S,16R)-3-(2-aminométhylbenzothiazol-5-yl)-7,11-dihydroxy-10-but-3-ényl-8,8,12,16-tétraméthyl-4,17-dioxabicyclo[14,1,0]-heptadécane-5,9-dione ;
(4S,7R,8S,9S,13Z,16S)-4,8-dihydroxy-7-but-3-inyl-5,5,9,13-tétraméthyl-16-(2-méthylbenzothiazol-5-yl)-oxacyclohexadéc-13-ène-2,6-dione ;
(4S,7R,8S,9S,13Z,16S)-4, 8-dihydroxy-16-(2-hydroxyméthylbenzothiazol-5-yl)-7-but-3-inyl-5,5,9,13-tétraméthyl-oxacyclohexadéc-13-ène-2,6-dione ;
(4S, 7R, 8S, 9S, 13Z, 16S)-16- (2-aminométhylthiazol-5-yl)-4,8-dihydroxy-7-but-3-inyl-5,5,9,13-tétraméthyl-oxacyclohexadéc-13-ène-2,6-dione ;
(1S,3S,7S,10R,11S,12S,16R)-7,11-dihydroxy-10-but-3-inyl-8,8,12,16-tétraméthyl-3-(2-méthylbenzothiazol-5-yl)-4,17-dioxabicyclo[14,1,0]-heptadécane-5,9-dione ;
(1S,3S,7S,10R,11S,12S,16R)-7,11-dihydroxy-3-(2-hydroxyméthylbenzothiazol-5-yl)-10-but-3-inyl-8,8,12,16-tétraméthyl-4,17-dioxabicyclo [14,1,0]-heptadécane-5,9-dione ;
(1S,3S,7S,10R,11S,12S,16R)-3-(2-aminométhylbenzothiazol-5-yl)-7,11-dihydroxy-10-but-3-inyl-8,8,12,16-tétraméthyl-4,17-dioxabicyclo[14,1,0]-heptadécane-5,9-dione ;
(4S,7R,8S,9S,13Z,16S)-4,8-dihydroxy-5,5,7,9,13-pentaméthyl-16-(2-méthylbenzoxazol-5-yl)-oxacyclohexadéc-13-ène-2,6-dione ;
(4S,7R,8S,9S,13Z,16S)-4,8-dihydroxy-16-(2-hydroxyméthylbenzoxazol-5-yl)-5,5,7,9,13-pentaméthyl-oxacyclohexadéc-13-ène-2,6-dione ;
(4S,7R,8S,9S,13Z,16S)-16-(2-aminométhylbenzoxazol-5-yl)-4,8-dihydroxy-5,5,7,9,13-pentaméthyl-oxacyclohexadéc-13-ène-2,6-dione ;
(1S,3S,7S,10R,11S,12S,16R)-7,11-dihydroxy-8,8,10,12,16-pentaméthyl-3-(2-méthylbenzoxazol-5-yl)-4,17-dioxabicyclo[14,1,0]heptadécane-5,9-dione ;
(1S,3S,7S,10R,11S,12S,16R)-7,11-dihydroxy-3-(2-hydroxyméthybenzoxazol-5-yl)-8,8,10,12,16-pentaméthyl-4,17-dioxabicyclo[14,1,0]heptadécane-5,9-dione ;
(1S,3S,7S,10R,11S,12S,16R)-3-(2-aminométhylbenzoxazol-5-yl)-7,11-dihydroxy-8,8,10,12,16-pentaméthyl-4,17-dioxabicyclo[14,1,0]heptadécane-5,9-dione ;
(4S,7R,8S,9S,13Z,16S)-4,8-dihydroxy-7-éthyl-5,5,9,13-tétraméthyl-16-(2-méthylbenzoxazol-5-yl)-oxacyclohexadéc-13-ène-2,6-dione ;
(4S,7R,8S,9S,13Z,16S)-4,8-dihydroxy-16-(2-hydroxyméthylbenzoxazol-5-yl)-7-éthyl-5,5,9,13-tétraméthyl-oxacyclohexadéc-13-ène-2,6-dione ;
(4S,7R,8S,9S,13Z,16S)-16-(2-aminométhylbenzoxazol-5-yl)-4,8-dihydroxy-7-éthyl-5,5,9,13-tétraméthyl-oxacyclohexadéc-13-ène-2,6-dione ;
(1S,3S,7S,10R,11S,12S,16R)-7,11-dihydroxy-10-éthyl-8,8,12,16-tétraméthyl-3-(2-méthylbenzoxazol-5-yl)-4,17-dioxabicyclo[14,1,0]-heptadécane-5,9-dione ;
(1S,3S,7S,10R,11S,12S,16R)-7,11-dihydroxy-3-(2-hydroxyméthylbenzoxazol-5-yl)-10-éthyl-8,8,12,16-tétraméthyl-4,17-dioxabicyclo[14,1,0]-heptadécane-5,9-dione ;
(1S,3S,7S,10R,11S,12S,16R)-3-(2-aminométhylbenzoxazol-5-yl)-7,11-dihydroxy-10-éthyl-8,8,12,16-tétraméthyl-4,17-dioxabicyclo[14,1,0]-heptadécane-5,9-dione ;
(4S,7R,8S,9S,13Z,16S)-4,8-dihydroxy-7-propyl-5,5,9,13-tétraméthyl-16-(2-méthylbenzoxazol-5-yl)-oxacyclohexadéc-13-ène-2,6-dione ;
(4S, 7R, 8S, 9S, 13Z, 16S)-4,8-dihydroxy-16-(2-hydroxyméthylbenzoxazol-5-yl)-7-propyl-5,5,9,13-tétraméthyl-oxacyclohexadéc-13-ène-2,6-dione ;
(4S,7R,8S,9S,13Z,16S)-16-(2-aminométhylbenzoxazol-5-yl)-4,8-dihydroxy-7-propyl-5,5,9,13-tétraméthyl-oxacyclohexadéc-13-ène-2,6-dione ;
(1S, 3S, 7S, 10R, 11S, 12S, 16R)-7,11-dihydroxy-10-propyl-8,8,12,16-tétraméthyl-3-(2-méthylbenzoxazol-5-yl)-4,17-dioxabicyclo[14,1,0]-heptadécane-5,9-dione ;
(1S, 3S, 7S, 10R, 11S, 12S, 16R)-7,11-dihydroxy-3-(2-hydroxyméthylbenzoxazol-5-yl)-10-propyl-8,8,12,16-tétraméthyl-4,17-dioxabicyclo[14,1,0]-heptadécane-5,9-dione ;
(1S,3S,7S,10R,11S,12S,16R)-3-(2-aminométhylbenzoxazol-5-yl)-7,11-dihydroxy-10-propyl-8,8,12,16-tétraméthyl-4,17-dioxabicyclo[14,1,0]-heptadécane-5,9-dione ;
(4S,7R,8S,9S,13Z,16S)-4,8-dihydroxy-7-butyl-5,5,9,13-tétraméthyl-16-(2-méthylbenzoxazol-5-yl)-oxacyclohexadéc-13-ène-2,6-dione ;
(4S,7R,8S,9S,13Z,16S)-4,8-dihydroxy-16-(2-hydroxyméthylbenzoxazol-5-yl)-7-butyl-5,5,9,13-tétraméthyl-oxacyclohexadéc-13-ène-2,6-dione ;
(4S,7R,8S,9S,13Z,16S)-16-(2-aminométhylbenzoxazol-5-yl)-4,8-dihydroxy-7-butyl-5,5,9,13-tétraméthyl-oxacyclohexadéc-13-ène-2,6-dione ;
(1S,3S,7S,10R,11S,12S,16R)-7,11-dihydroxy-10-butyl-8,8,12,16-tétraméthyl-3-(2-méthylbenzoxazol-5-yl)-4,17-dioxabicyclo[14.1.0]-heptadécane-5,9-dione ;
(1S,3S,7S,10R,11S,12S,16R)-7,11-dihydroxy-3-(2-hydroxyméthylbenzoxazol-5-yl)-10-butyl-8,8,12,16-tétraméthyl-4,17-dioxabicyclo[14.1.0]heptadécane-5,9-dione ;
(1S,3S,7S,10R,11S,12S,16R)-3-(2-aminométhylbenzoxazol-5-yl)-7,11-dihydroxy-10-butyl-8,8,12,16-tétraméthyl-4,17-dioxabicyclo[14,1,0]-heptadécane-5,9-dione ;
(4S,7R,8S,9S,13Z,16S)-4,8-dihydroxy-7-allyl-5,5,9,13-tétraméthyl-16-(2-méthylbenzoxazol-5-yl)-oxacyclohexadéc-13-ène-2,6-dione ;
(4S,7R,8S,9S,13Z,16S)-4,8-dihydroxy-16-(2-hydroxyméthylbenzoxazol-5-yl)-7-allyl-5,5,9,13-tétraméthyl-oxacyclohexadéc-13-ène-2,6-dione ;
(4S,7R,8S,9S,13Z,16S)-16-(2-aminométhylbenzoxazol-5-y1)-4,8-dihydroxy-7-allyl-5,5,9,13-tétraméthyl-oxacyclohexadéc-13-ène-2,6-dione ;
(1S,3S,7S,10R,11S,12S,16R)-7,11-dihydroxy-10-allyl-8,8,12,16-tétraméthyl-3-(2-méthylbenzoxazol-5-yl)-4,17-dioxabicyclo[14,1,0]-heptadécane-5,9-dione ;
(1S, 3S, 7S, 10R, 11S, 12S, 16R)-7,11-dihydroxy-3-(2-hydroxyméthylbenzoxazol-5-yl)-10-allyl-8,8,12,16-tétraméthyl-4,17-dioxabicyclo[14,1,0]-heptadécane-5,9-dione ;
(1S,3S,7S,10R,11S,12S,16R)-3-(2-aminométhylbenzoxazol-5-yl)-7,11-dihydroxy-10-allyl-8,8,12,16-tétraméthyl-4,17-dioxabicyclo[14,1,0]-heptadécane-5,9-dione ;
(4S,7R,8S,9S,13Z,16S)-4,8-dihydroxy-7-prop-2-inyl-5,5,9,13-tétraméthyl-16-(2-méthylbenzoxazol-5-yl)-oxacyclohexadéc-13-ène-2,6-dione ;
(4S,7R,8S,9S,13Z,16S)-4,8-dihydroxy-16-(2-hydroxyméthylbenzoxazol-5-yl)-7-prop-2-inyl-5,5,9,13-tétraméthyl-oxacyclohexadéc-13-ène-2,6-dione ;
(4S,7R,8S,9S,13Z,16S)-16-(2-aminométhylbenzoxazol-5-yl)-4,8-dihydroxy-7-prop-2-inyl-5,5,9,13-tétraméthyl-oxacyclohexadéc-13-ène-2,6-dione ;
(1S,3S,7S,10R,11S,12S,16R)-7,11-dihydroxy-10-prop-2-inyl-8,8,12,16-tétraméthyl-3-(2-méthylbenzoxazol-5-yl)-4,17-dioxabicyclo[14,1,0]-heptadécane-5,9-dione ;
(1S,3S,7S,10R,11S,12S,16R)-7,11-dihydroxy-3-(2-hydroxyméthylbenzoxazol-5-yl)-10-prop-2-inyl-8,8,12,16-tétraméthyl-4,17-dioxabicyclo[14,1,0]-heptadécane-5,9-dione ;
(1S,3S,7S,10R,11S,12S,16R)-3-(2-aminométhylbenzoxazol-5-yl)-7,11-dihydroxy-10-prop-2-inyl-8,8,12,16-tétraméthyl-4,17-dioxabicyclo[14,1,0]-heptadécane-5,9-dione ;
(4S,7R,8S,9S,13Z,16S)-4,8-dihydroxy-7-but-3-ényl-5,5,9,13-tétraméthyl-16-(2-méthylbenzoxazol-5-yl)-oxacyclohexadéc-13-ène-2,6-dione ;
(4S,7R,8S,9S,13Z,16S)-4,8-dihydroxy-16-(2-hydroxyméthylbenzoxazol-5-yl)-7-but-3-ényl-5,5,9,13-tétraméthyl-oxacyclohexadéc-13-ène-2,6-dione ;
(4S,7R,8S,9S,13Z,16S)-16-(2-aminométhylbenzoxazol-5-yl)-4,8-dihydroxy-7-but-3-ényl-5,5,9,13-tétraméthyl-oxacyclohexadéc-13-ène-2,6-dione ;
(1S,3S,7S,10R,11S,12S,16R)-7,11-dihydroxy-10-but-3-ényl-8,8,12,16-tétraméthyl-3-(2-méthylbenzoxazol-5-yl)-4,17-dioxabicyclo[14,1,0]-heptadécane-5,9-dione ;
(1S,3S,7S,10R,11S,12S,16R)-7,11-dihydroxy-3-(2-hydroxyméthylbenzoxazol-5-yl)-10-but-3-ényl-8,8,12,16-tétraméthyl-4,17-dioxabicyclo[14,1,0]-heptadécane-5,9-dione ;
(1S,3S,7S,10R,11S,12S,16R)-3-(2-aminométhylbenzoxazol-5-yl)-7,11-dihydroxy-10-but-3-ényl-8,8,12,16-tétraméthyl-4,17-dioxabicyclo[14,1,0]-heptadécane-5,9-dione ;
(4S,7R,8S,9S,13Z,16S)-4,8-dihydroxy-7-but-3-inyl-5,5,9,13-tétraméthyl-16-(2-méthylbenzoxazol-5-yl)-oxacyclohexadéc-13-ène-2,6-dione ;
(4S,7R,8S,9S,13Z,16S)-4,8-dihydroxy-16-(2-hydroxyméthylbenzoxazol-5-yl)-7-but-3-inyl-5,5,9,13-tétraméthyl-oxacyclohexadéc-13-ène-2,6-dione ;
(4S,7R,8S,9S,13Z,16S)-16-(2-aminométhylbenzoxazol-5-yl)-4,8-dihydroxy-7-but-3-inyl-5,5,9,13-tétraméthyl-oxacyclohexadéc-13-ène-2,6-dione ;
(1S,3S,7S,10R,11S,12S,16R)-7,11-dihydroxy-10-but-3-inyl-8,8,12,16-tétraméthyl-3-(2-méthylbenzoxazol-5-yl)-4,17-dioxabicyclo[14,1,0]-heptadécane-5,9-dione ;
(1S,3S,7S,10R,11S,12S,16R)-7,11-dihydroxy-3-(2-hydroxyméthylbenzoxazol-5-yl)-10-but-3-inyl-8,8,12,16-tétraméthyl-4,17-dioxabicyclo[14,1,0]-heptadécane-5,9-dione ;
(1S,3S,7S,10R,11S,12S,16R)-3-(2-aminométhylbenzoxazol-5-yl)-7,11-dihydroxy-10-but-3-inyl-8,8,12,16-tétraméthyl-4,17-dioxabicyclo[14,1,0]-heptadécane-5,9-dione ;
les atomes d'hydrogène dans les éléments effecteurs mentionnés ci-dessus étant remplacés par des radicaux L¹-L³ dans les positions indiquées dans la formule (I).

4. Conjugué d'effecteurs selon l'une des revendications 1 à 3, **caractérisé en ce que** le lieur est choisi dans le groupe constitué de composés de formule générale (III), dans laquelle Aa1 représente une liaison.

5. Conjugué d'effecteurs selon l'une des revendications 1 à 4, **caractérisé en ce que** :
FG¹ est C₁-C₁₀-alkyl-S₃ ou

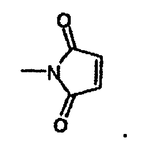

6. Conjugué d'effecteur-unité de reconnaissance de formule générale (I) : 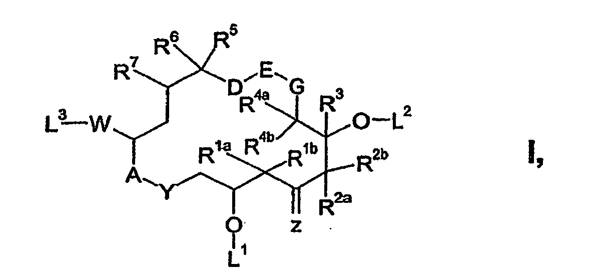 dans laquelle les substituants ont ici les significations qui sont mentionnées dans la revendication 1, mais au moins un groupement FG¹ est remplacé par un groupement FG^{2a} ou FG^{2b}, où FG^{2a} ou FG^{2b} peuvent avoir les significations suivantes :
FG^{2a} est -S-S-, 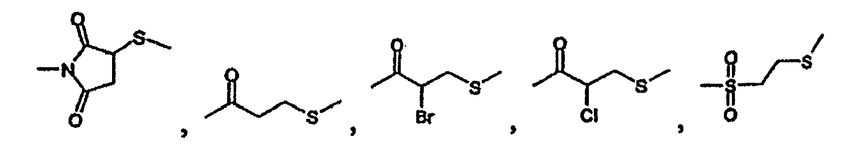
FG^{2b} est -CONH- ,
et où une unité de reconnaissance est conjuguée par l'intermédiaire d'un atome de soufre avec le groupement FG^{2a}, où l'atome de soufre qui est montré est un composant de l'unité de reconnaissance, ou par l'intermédiaire d'un groupement amide avec le groupement FG^{2b}, où l'atome d'azote qui est montré est un composant de l'unité de reconnaissance ;
où l'unité de reconnaissance est choisie dans le groupe constitué de peptides, de récepteurs solubles, de cytokines, de lymphokines, d'aptamères, de spiegelmères, de protéines recombinantes, de nouvelles structures de trames, d'anticorps monoclonaux et de fragments d'anticorps monoclonaux ;
sous forme d'un isomère uniforme ou d'un mélange de différents isomères et/ou sous forme d'un sel pharmaceutiquement acceptable de celui-ci.

7. Conjugué d'effecteur-unité de reconnaissance selon la revendication 6, **caractérisé en ce** le conjugué contient plus d'une unité de reconnaissance et en ce que les unités de reconnaissance sont identiques.

8. Conjugué d'effecteur-unité de reconnaissance selon la revendication 6 ou 7, **caractérisé en ce que** l'unité de reconnaissance est un anticorps ou un fragment de liaison d'antigène de celui-ci, qui est spécifique à un antigène choisi dans le groupe constitué des antigènes indiqués dans le tableau 1, ainsi que CD19, CD20, CD40, CD22, CD25, CD5, CD52, CD10, CD2, CD7, CD33, CD38, CD40, CD72, CD4, CD21, CD37, CD30, VCAM, CD31, ELAM, endogline, VEGFRI/II, VEGFRvIII, scFv(14E1), αᵥβ₃, Tie1/2, TES23 (CD44ex6), phosphatidylsérine, PSMA, le complexe VEGFR/VEGF et ED-B-fibronectine.

9. Lieurs de formule générale (III¹) : 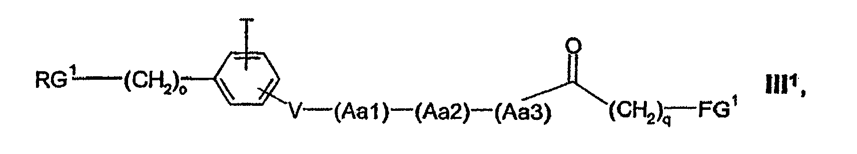 dans laquelle :
RG¹ est un groupement O=C=N, et o, V, q, T, Aa1, Aa2, Aa3 et FG¹ ont les significations qui sont mentionnées dans la revendication 1 ;
ou lieurs de formule générale (III²) : 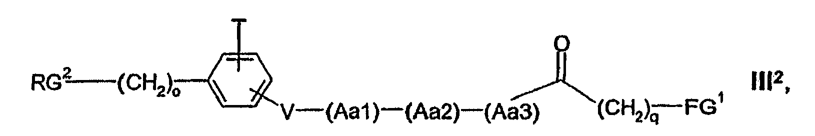 dans laquelle :
RG² est un groupement Hal-C(=O)-CHR²² ou un groupement Hal-C(=O)-CHR²²-NR²³-C (=O), ou un groupement R²⁶-C(=O)-O-C(=O)-CHR²² ou un groupement R²⁶ -C (=O)-O-C(=O)-CHR²²-NR²³-C (=O), où R²⁶ est C₁-C₁₀-alkyle, aryle ou aralkyle, et o, V, q, T, Aa1, Aa2, Aa3 et FG¹ ont les significations qui sont mentionnées dans la revendication 1 ;
ou lieurs de formule générale (III³) : 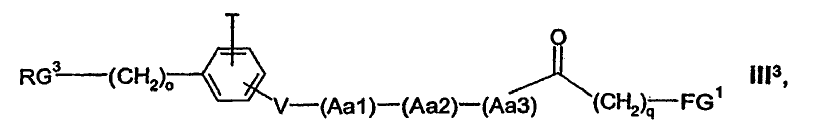 dans laquelle :
RG³ est un groupement OH ou un groupement NHR^{24a} ou un groupement COOH, et o, V, q, T, Aa1,
Aa2, Aa3 et FG¹ ont les significations qui sont mentionnées dans la revendication 1.

10. Procédé de production de conjugués d'effecteurs selon l'une des revendications 1 à 5, **caractérisé en ce qu'**un composé de formule générale (I), dans laquelle les substituants ont les significations qui sont mentionnées dans la revendication 1, sauf que :
la condition selon laquelle au moins un groupement L¹, L² ou L⁴ représente un lieur de formule générale (III) n'a pas à être remplie, et au moins un substituant L¹, L² ou L⁴ représente hydrogène ou un groupement C(=O)Cl ;
est mis en réaction avec un lieur qui est choisi dans le groupe qui est constitué d'un lieur de formule générale (III¹) ou (III²) ou (III³), tel que décrit dans la revendication 9.

11. Procédé de production de conjugués d'effecteur-unité de reconnaissance selon l'une des revendications 6 à 8, **caractérisé en ce qu'**un conjugué d'effecteurs selon l'une des revendications 1 à 5 est mis en réaction avec au moins une unité de reconnaissance, telle que définie dans les revendications 6 et 8.

12. Utilisation d'un composé de formule générale (I), dans laquelle les substituants ont les significations qui sont mentionnées dans la revendication 1, sauf que :
la condition selon laquelle au moins un groupement L¹, L² ou L⁴ représente un lieur de formule générale (III) n'a pas à être remplie, et au moins un substituant L¹, L² ou L⁴ représente hydrogène ou un groupement C(=O)Cl ;
dans un procédé selon la revendication 10.

13. Utilisation d'un composé de formule générale (I) pour la production d'un conjugué d'effecteur-unité de reconnaissance selon les revendications 6 à 8.

14. Utilisation d'un lieur de formule générale (III¹), (III²) ou (III³) dans un procédé selon la revendication 10.

15. Utilisation d'un lieur de formule générale (III¹), (III²) ou (III³) pour la production d'un conjugué d'effecteur-unité de reconnaissance selon l'une des revendications 6 à 8.

16. Utilisation d'une unité de reconnaissance, telle que définie dans la revendication 6 ou 8, dans un procédé selon la revendication 11.

17. Conjugué d'effecteur-unité de reconnaissance selon l'une des revendications 6 à 8, destiné à être utilisé comme médicament.

18. Conjugué d'effecteur-unité de reconnaissance selon l'une des revendications 6 à 8, destiné à être utilisé comme médicament pour le traitement de maladies qui sont associées à des processus prolifératifs.

19. Conjugué d'effecteur-unité de reconnaissance selon l'une des revendications 6 à 8, destiné à être utilisé comme médicament pour le traitement d'une maladie qui figure dans le groupe constitué de tumeurs, de maladies inflammatoires, de maladies neuro-dégénératives, de maladies associées à l'angiogénèse, de la sclérose en plaques, de la maladie d'Alzheimer et de la polyarthrite rhumatoïde.
